# EUROPEAN PATENT SPECIFICATION

(11) **EP 4 408 854 B1**
(45) Date of publication and mention of the grant of the patent: **22.04.2026**
(21) Application number: 22793436.1
(22) Date of filing: 28.09.2022
(51) Int. Cl.: C07H 19/14, C07H 19/23, A61P 33/00, A61K 31/519

(54) **NUCLEOSIDE ANALOGUES FOR THE TREATMENT OF PARASITIC INFECTIONS**
NUKLEOSIDANALOGA ZUR BEHANDLUNG PARASITÄRER INFEKTIONEN
ANALOGUES DE NUCLÉOSIDE POUR LE TRAITEMENT D'INFECTIONS PARASITAIRES

(30) Priority: 28.09.2021 EP 21199547
(43) Date of publication of application: 07.08.2024
(73) Proprietor: Universiteit Antwerpen, 2000 Antwerpen (BE); Universiteit Gent, 9000 Gent (BE)
(72) Inventor: VAN CALENBERGH, Serge, 9000 Gent (BE); CALJON, Guy, 3191 Hever (BE); HULPIA, Fabian, 9100 Sint-Niklaas (BE); BOUTON, Jakob, 8000 Brugge (BE)
(74) Representative: Arnold & Siedsma
(86) International application number: PCT/EP2022/077048
(87) International publication number: WO 2023/052468

(56) References cited:
- WO-A1-2019/076633
- AL SAFARJALANI O N ET AL: "7-Deaza-6-benzylthioinosine analogues as subversive substrate of Toxoplasma gondii adenosine kinase: Activities and selective toxicities", BIOCHEMICAL PHARMACOLOGY, ELSEVIER, US, vol. 76, no. 8, 15 October 2008 (2008-10-15), pages 958 - 966, XP025478961, ISSN: 0006-2952, [retrieved on 20080807], DOI: 10.1016/J.BCP.2008.07.035
- KIM YOUNG AH ET AL: "Structure-Activity Relationships of 7-Deaza-6-benzylthioinosine Analogues as Ligands of Toxoplasma gondii Adenosine Kinase", JOURNAL OF MEDICINAL CHEMISTRY, vol. 51, no. 13, 1 July 2008 (2008-07-01), US, pages 3934 - 3945, XP055894553, ISSN: 0022-2623, DOI: 10.1021/jm800201s
- PERLÍKOVÁ PAVLA ET AL: "Synthesis and Antitrypanosomal Activity of 6-Substituted 7-Methyl-7-deazapurine Nucleosides", ACS INFECTIOUS DISEASES, vol. 7, no. 4, 9 April 2021 (2021-04-09), US, pages 917 - 926, XP055894552, ISSN: 2373-8227, DOI: 10.1021/acsinfecdis.1c00062

## Description

### FIELD OF THE INVENTION

The present invention relates to novel nucleoside analogues and compositions containing said nucleoside analogues. Moreover, the present invention provides processes for the preparation of the disclosed compounds, as well as methods of using them, for instance as a medicine, in particular for the diagnosis, prevention and/or treatment of parasitic infections, more specifically for use in the diagnosis, prevention and/or treatment of a Trypanosoma infection.

### BACKGROUND TO THE INVENTION

Trypanosomiases are a family of related parasitic protozoal diseases that includes African trypanosomiasis. Tsetse-transmitted African trypanosomiasis is unique to sub-Saharan Africa and affect both people (Human African Trypanosomiasis - HAT) and animals (Animal trypanosomiasis - AT). Outside Africa, non-tsetse-transmitted species may be spread mechanically by blood-sucking flies (tabanids, *Stomoxys*) or vampire bats transferring the infection directly from one host to another. Trypanosome infections affect human health, animal health, agricultural production and rural development.

The human form is almost always fatal (if not treated) and is caused by *Trypanosoma brucei* spp., of which *T. b. gambiense* is prevalent in West and Central Africa and *T. b. rhodesiense* in East and Southern Africa. Initially, parasites reside in the haemolymphatic system (Stage-I) and cause rather non-specific symptoms (*e.g*. general malaise and fever). The second phase (stage-II) consists of parasites crossing the blood-brain barrier (BBB) causing severe and lethal neurological complications (e.g. altered sleep/wake cycles, hence the name 'sleeping sickness').

The animal form can be caused by several *Trypanosoma* species, in Africa most prominently *T. b. brucei, T. congolense* and *T. vivax.* Ruminant (cattle, sheep, goat) trypanosomiasis is widespread and has devastating effects on animal husbandry, preventing satisfactory livestock rearing. Wildlife may serve as a reservoir. Outside the tsetse belt of Africa, *T. evansi* and *T. vivax* are spread mechanically and mainly affect draught animals (horses, camels, water buffalos) used for unmechanized farming and transport in North Africa, South America and Southern Asia. *T. equiperdum* is a pathogen for horses that is transmitted venereally.

Current drugs for HAT suffer from several major limitations, particularly for efficacy against Stage-II which requires adequate Central Nervous System (CNS) drug exposure. For Stage-II disease, currently three drugs / drug combinations are approved: melarsoprol, eflornithine (DFMO) and nifurtimox/eflornithine. Parenteral administration of melarsoprol or eflornithine poses significant logistic challenges for rural areas in Africa while severe toxicity issues with melarsoprol are well-known. Recently, novel entities have phase II/III clinical trials and could greatly improve treatment options, e.g. acoziborole SCYX-7158. Recently, the Drug for Neglected Diseases *initiative* (DNDi, Geneva) obtained global approval for orally administered fexinidazole against *gambiense* HAT, making it the first new HAT therapeutic in three decades as well as the first oral monotherapy. However, fexinidazole is expected to show cross-resistance with nifurtimox, as they exhibit a similar mode-of-action. The various drugs that are currently used for prophylaxis and/or treatment of AT (diminazene aceturate, homidium bromide/chloride, isometamidium, pyrithidium bromide, quinapyrine, suramin) need to be injected and may cause severe local reactions (irritation, oedema, e.a.) as well as systemic side-effects, actually leading to contraindicated use in some animal species (horse, camel, dog). These findings highlight the ever-pressing need for novel therapies in the treatment of parasitic infections in general, especially using molecules from other structural classes.

In that respect, nucleoside analogues have received considerable interest over the past six decades, with respect to many therapeutic areas, with most of the focus being their evaluation as either antiviral or anti-tumor agents (Jordheim et al., 2013; Shelton et al., 2016; WO2005/020885; WO2010/121576).

Parasites in general, and trypanosomes in particular, can be especially vulnerable to the effects of purine nucleoside analogues, because of their sole dependency on purine salvage, as they lack the enzymes for *de novo* purine synthesis. In this regard, potential inhibitors for enzymes of the salvage pathway, as well as so-called 'subversive' substrates (analogues that use the parasite's salvage pathway enzyme(s) to exert their toxic effect(s)), bearing a nucleoside structure, have been conceived or discovered by screening efforts ( Berg et al., 2010). One such subversive analogue, is the naturally occurring nucleoside antibiotic tubercidin. This analogue was found to exert a plethora of biological effects (as it is a close structural mimic of adenosine), but unfortunately, it is overly cytotoxic to mammalian cells and thus non-selective, which makes it of little practical value.

Work by the research group of Hocek showed that certain C-7 substituted 7-deaza-adenosines were only poorly cytotoxic (6-membered rings; versus cytotoxic 5-membered rings) to both tumor as well as fibroblast cells (Snášel et al., 2014; Bourderioux et al., 2011).

Pavia et al., 2021 (Synthesis and antitrypanosomal activity of 6-substituted 7-methyl-7-deazapurine nucleosides - ACS Infectious Diseases, vol. 7 no. 4, Apr, 09 2021 pg. 917-926), WO9618398, WO2008157438 and WO9616664 disclose deazapurine ribose derivatives, 5'-amino derivatives, and adenosine derivatives respectively, however neither of these comply with the requirements of the present invention.

We have now identified that a specific subseries of S-substituted nucleoside analogues as disclosed herein results in a very good potency against parasite infections, with limited toxicity. WO2019076633 discloses closely relating nucleoside compounds for the treatment of parasitic infections, however no S-substituted nucleoside analogues have been specifically disclosed therein. Moreover, it could not have been predicted on beforehand from the disclosure of WO2019076633 that the presently observed effects would have been achieved by the current set of compounds. Moreover, the compounds of the present invention are further characterized in having good potency against multiple parasitic species, which allows for a broader application area.

Accordingly, in the present invention, we explored a further subset of nucleoside compounds to evaluate their *in vitro* and *in vivo* effects, and identified several interesting and potent compounds useful in the treatment of parasitic infection, more specifically *Trypanosoma* infections.

### SUMMARY OF THE INVENTION

In a first aspect, the present invention provides a compound of Formula I or a stereoisomer, tautomer, racemic, salt, hydrate, or solvate thereof, 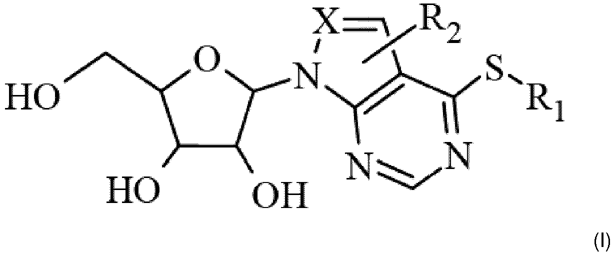 wherein
X is C;
R₁ is selected from the group comprising: -C₂₋₆alkyl, -C₂₋₆alkenyl, -CF₃, -C₁₋₆alkyl-R₃, -Ar₁, -Het₁ and -
Cy₁;
R₂ is selected from the group comprising: -H or -halo;
R₃ is selected from the group comprising: -C₁₋₆alkyl, -C₁₋₆alkenyl, -C₁₋₆alkynyl, -Ar₂, -Het₂ and -Cy₂,
Ar₁ and Ar₂ are each independently selected from a 5- to 10-membered aromatic cycle optionally comprising 1 to 3 heteroatoms selected from O, N and S; each of said Ar₁ and Ar₂ optionally being substituted with one or more substituents independently selected from the list comprising: -halo, - CF₃, -C₁₋₆alkyl, -C₁₋₆alkenyl, -C₁₋₆alkynyl, -O-C₁₋₆alkyl, and -S-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -CF₃, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 3- to 10-membered non-aromatic cycle; each of said Cy₁ and Cy₂ optionally being substituted with one or more substituents independently selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl.

In a further embodiment, the present invention provides a compound as defined herein, wherein:
X is C;
R₁ is selected from the group comprising: -C₂₋₆alkyl, -C₂₋₆alkenyl, -CF₃, -C₁₋₆alkyl-R₃, -Ar₁, -C₂₋₆alkyl-Ar₁, -Het₁ and -Cy₁;
R₂ is -H or -halo;
R₃ is selected from the group comprising: -C₁₋₆alkyl, -C₁₋₆alkenyl, -C₁₋₆alkynyl, -Het₂ and -Cy₂,
Ar₁ is a 5- to 10-membered aromatic cycle optionally comprising 1 to 3 heteroatoms selected from O, N and S; said Ar₁ optionally being substituted with one or more substituents independently selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -CF₃, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 3- to 10-membered non-aromatic cycle; each of said Cy₁ and Cy₂ optionally being substituted with one or more substituents independently selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl.

In another particular embodiment, the present invention provides a compound as defined herein wherein:
R₁ is selected from the group comprising: -C₂₋₆alkyl, -C₁₋₆alkyl-R₃, -C₂₋₆alkyl-Ar₁, -Ar₁, -Het₁ and -Cy₁;
R₂ is H;
R₃ is selected from the group comprising: -C₁₋₆alkyl, -C₁₋₆alkenyl, -Het₂ and -Cy₂,
X is C
Ar₁ is a 5- to 6-membered aromatic cycle, said Ar₁ optionally being substituted with one or more substituents independently selected from the list comprising: -halo, -C₁₋₆alkyl, and -O-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -CF₃, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 5- to 6-membered non-aromatic cycle; each of said Cy₁ and Cy₂ optionally being substituted with one or more substituents independently selected from the list comprising: -halo, -C₁₋₆alkyl, and -O-C₁₋₆alkyl.

In yet another particular embodiment, the present invention provides a compound as defined herein wherein:
R₁ is selected from the group comprising: -C₂₋₆alkyl, -C₁₋₃alkyl-R₃, -C₂₋₃alkyl-Ar₁, -Ar₁, -Het₁ and -Cy₁;
R2 is H;
R₃ is selected from the group comprising: -C₁₋₆alkyl, -C₁₋₆alkenyl, - Het₂ and -Cy₂,
X is C;
Ar₁ is -phenyl; optionally being substituted with one or more substituents selected from the list comprising: -halo, -C₁₋₆alkyl, and -O-C₁₋₆alkyl;
Het₁ and Het₂ are each independently -pyridinyl;
Cy₁ and Cy₂ are each independently selected from - cyclopentane and -cycloheptane.

In another embodiment, the present invention provides a compound as defined herein wherein:
R₁ is selected from the group comprising: -C₂₋₆alkyl, -C₁₋₃alkyl-R₃, -C₂₋₃alkyl-Ar₁, -Ar₁, -Het₁ and -Cy₁;
R₂ is H;
R₃ is selected from the group comprising: -C₁₋₆alkyl, -C₁₋₆alkenyl, and -Cy₂,
X is C;
Ar₁ is -phenyl optionally being substituted with one or more substituents selected from the list comprising: -halo, -C₁₋₆alkyl, and -O-C₁₋₆alkyl;
Het₁ is -pyridinyl;
Cy₁ and Cy₂ are each independently selected from -cyclopentane and -cycloheptane.

In a further embodiment, the present invention provides a compound as defined herein wherein;
R₁ is selected from the group comprising: -C₂₋₆alkyl, -C₁₋₆alkyl-R₃, -Ar₁, and -Cy₁;
R2 is H;
R₃ is selected from the group comprising: -C₁₋₆alkyl, -C₁₋₆alkenyl, -Ar₂, and -Cy₂,
X is C
Ar₁ and Ar₂ are each independently selected from a 5- to 6-membered aromatic cycle; each of said Ar₁ and Ar₂ optionally being substituted with one or more substituents independently selected from the list comprising: -halo, -C₁₋₆alkyl, and -O-C₁₋₆alkyl;
Cy₁ and Cy₂ are each independently selected from a 5- to 6-membered non-aromatic cycle; each of said Cy₁ and Cy₂ optionally being substituted with one or more substituents independently selected from the list comprising: -halo, -C₁₋₆alkyl, and -O-C₁₋₆alkyl.

In yet a further embodiment, the present invention provides a compound as defined herein wherein;
R₁ is selected from the group comprising: -C₂₋₆alkyl, -C₁₋₃alkyl-R₃, -Ar₁, and -Cy₁;
R₃ is selected from the group comprising: -C₁₋₆alkyl, -C₁₋₆alkenyl, -Ar₂, and -Cy₂,
X is C;
Ar₁ and Ar₂ are each independently -phenyl; each of said -phenyl optionally being substituted with a substituent selected from the list comprising: -halo, -C₁₋₆alkyl, and -O-C₁₋₆alkyl;
Cy₁ and Cy₂ are each independently selected from - cyclopentane and -cycloheptane.
In a still further embodiment, the present invention provides a compound as defined herein wherein;
R₁ is selected from the group comprising: -C₂₋₆alkyl, -C₁₋₃alkyl-R₃, -Ar₁, and -Cy₁;
R₃ is selected from the group comprising: -C₁₋₆alkyl, -Ar₂, and -Cy₂,
X is C;
Ar₁ and Ar₂ are each independently -phenyl; each of said -phenyl optionally being substituted with a substituent selected from the list comprising: -halo, -C₁₋₆alkyl, and -O-C₁₋₆alkyl;
Cy₁ and Cy₂ are each independently selected from -cyclopentane and -cycloheptane.

In yet a further embodiment, the present invention provides a compound as defined herein wherein;
R₁ is selected from the group comprising: -C₂₋₆alkyl, -C₁₋₃alkyl-R₃, and -Ar₁;
R₃ is selected from the group comprising: -C₁₋₆alkyl, -C₁₋₆alkenyl, and -Cy₂;
X is C;
Ar₁ is -phenyl optionally being substituted with -O-C₁₋₆alkyl; and
Cy₂ is -cyclopentane.

The present application also provides a compound or reference compound (RC) selected from the list comprising:

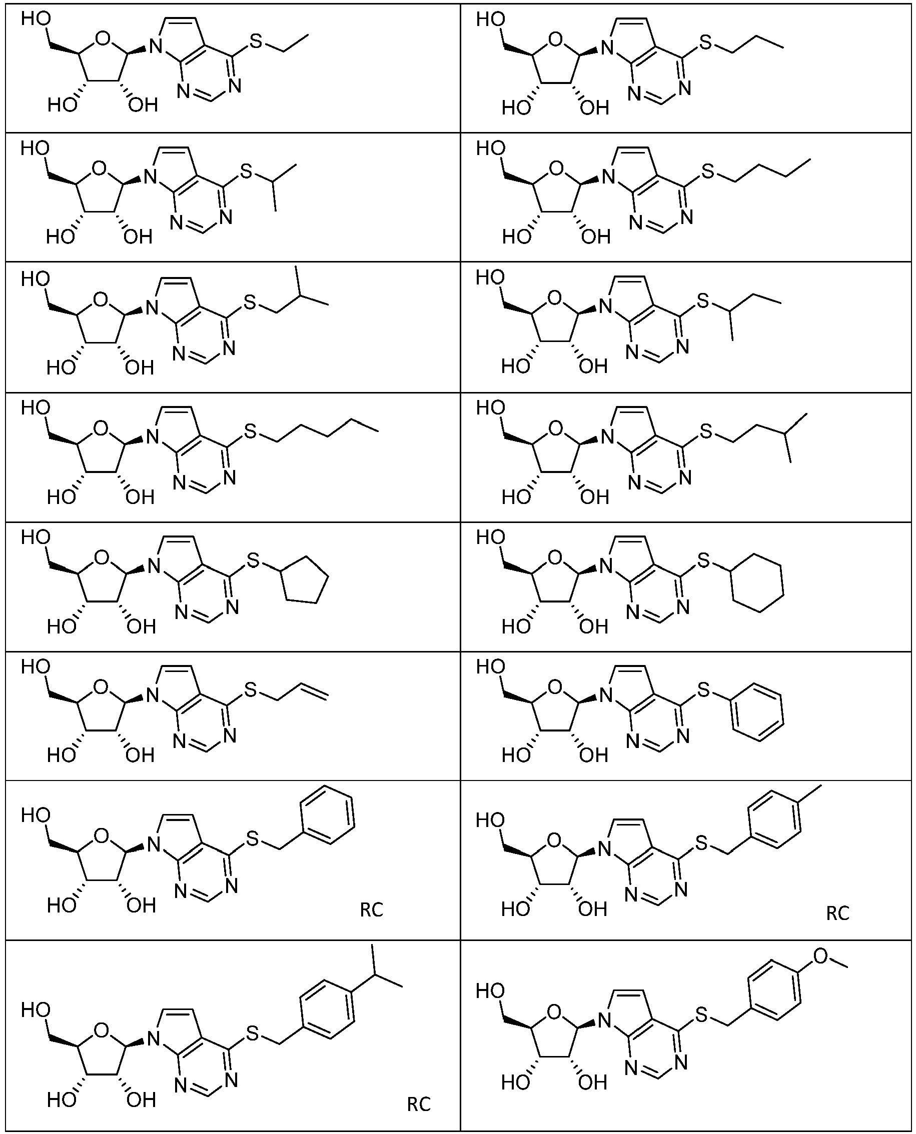

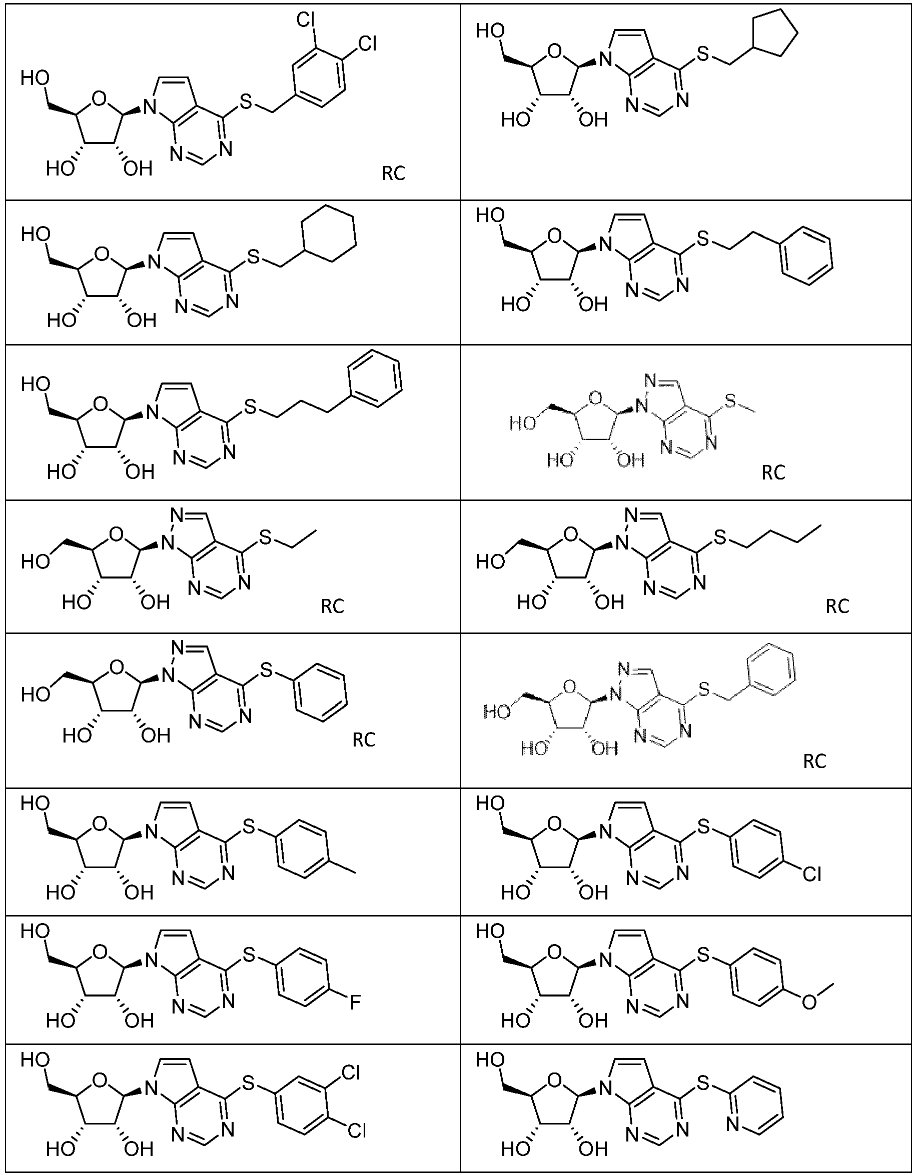

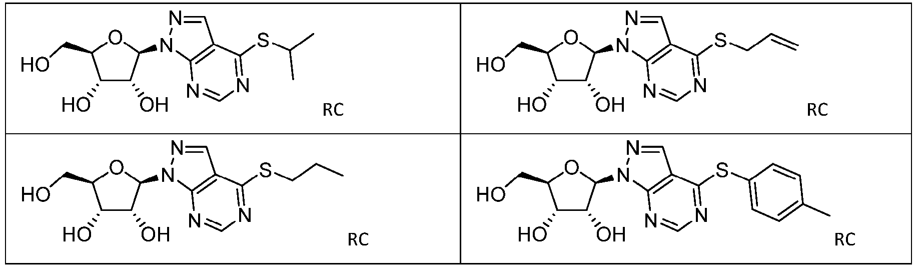

In another particular embodiment, the compounds of the present invention have the stereochemistry as represented in formula II:

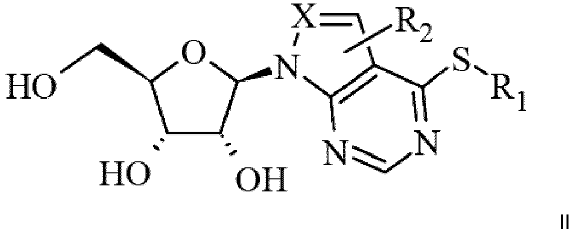

The present invention further provides a pharmaceutical composition comprising a compound as defined herein and at least one pharmaceutically acceptable carrier or diluent.

In a further aspect, the present invention provides a compound or pharmaceutical composition as defined herein for use as a human or veterinary medicine.

In yet a further aspect, the present invention provides a compound or pharmaceutical composition as defined herein for use in the diagnosis, prevention and/or treatment of a parasite infection, in particular *a Trypanosoma* infection; in particular a *T. brucei, T. cruzi, T. congolense, T. vivax, T. evansi* and *T. equiperdum* infection.

In a further aspect, the present invention provides a compound of Formula I or a stereoisomer, tautomer, racemic, salt, hydrate, or solvate thereof, or a pharmaceutical composition comprising such compound, for use in the diagnosis, prevention and/or treatment of a parasite infection, in particular a *Trypanosoma* infection 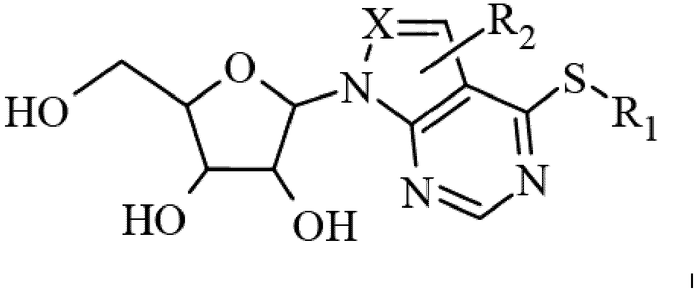 Wherein
X is C;
R₁ is selected from the group comprising: -C₁₋₆alkyl, -C₁₋₆alkyl-R₃, -Ar₁, -Het₁ and -Cy₁;
R₂ is selected from the group comprising: -H or -halo;
R₃ is selected from the group comprising: -C₁₋₆alkyl, -C₁₋₆alkenyl, -C₁₋₆alkynyl, -Ar₂, -Het₂ and -Cy₂,
Ar₁ and Ar₂ are each independently selected from a 5- to 10-membered aromatic cycle optionally comprising 1 to 3 heteroatoms selected from O, N and S; each of said Ar₁ and Ar₂ optionally being substituted with one or more substituents independently selected from the list comprising: -halo, - C₁₋₆alkyl, -C₁₋₆alkenyl, -C₁₋₆alkynyl, -O-C₁₋₆alkyl, and -S-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 3- to 10-membered non-aromatic cycle; each of said Cy₁ and Cy₂ optionally being substituted with one or more substituents independently selected from the list comprising: -halo, -C₁₋₆alkyl, -C₁₋₆alkenyl, -C₁₋₆alkynyl, -O-C₁₋₆alkyl, and -S-C₁₋₆alkyl.

The present application further provides a method for the diagnosis, prevention and/or treatment of a parasite infection, in particular a *Trypanosoma* infection in a subject in need thereof; said method comprising administering to said subject, a therapeutically effective amount of a compound or a pharmaceutical composition as defined herein.

In a very specific embodiment, the *Trypanosoma* infection as defined herein may be selected from the list comprising: *T. brucei, T.cruzi, T. congolense, T. vivax, T. evansi* and *T. equiperdum.*

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.
**Figure 1****: *In vitro* metabolic stability of selected hits in the presence of S9 microsomal fraction of target species.** Accepted metabolic stability requires at least 50% of parent compound remaining after 30 minutes of incubation.
**Figure 2****: *In vivo* evaluation of nucleoside leads in T. *vivax* late curative mouse model.** CL5565 was administrated for 3 days. FH15963 and FH15967 were administrated for 5 consecutive days, in a dosing regimen as indicated for each treatment group. In all groups, treatment started when parasitemia reached ≥10⁶ trypanosome mL⁻¹. Blood parasitemia was evaluated via tail vein blood samples.
**Figure 3****: *In vivo* evaluation of FH15967 in T. *brucei* late curative mouse model.**
   **Panel A:** BLI images of T. b. brucei-infected mice treated with diminazene aceturate (10 mg kg⁻¹ s.i.d. IP), and FH15967 (50 mg kg⁻¹ s.i.d. and b.i.d. IP). Animals were imaged from dorsal and ventral sides.
   **Panel B:** BLI (average signal ± SEM) within a region of interest that corresponds to the abdomen.

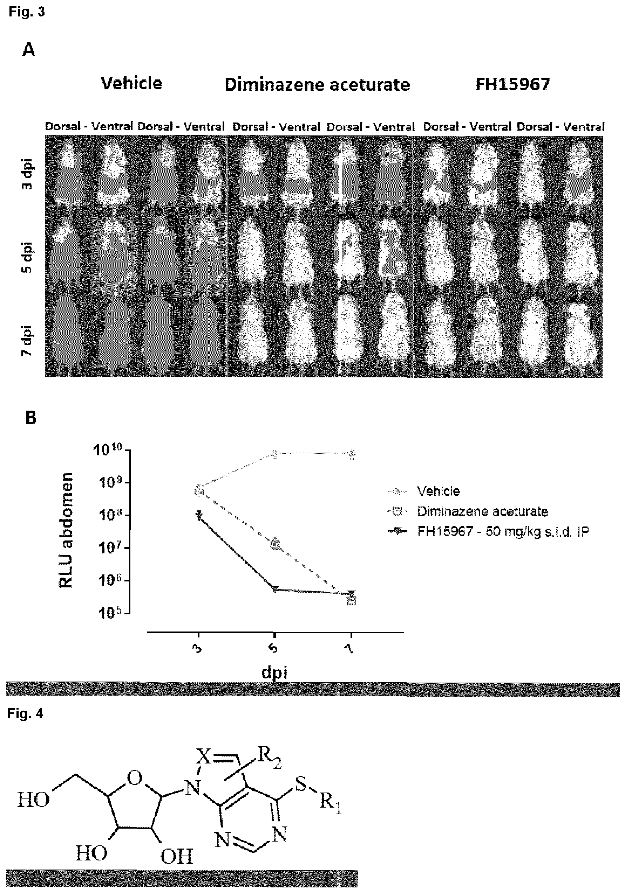

**Figure 4****:Formula I of the present invention**

### DETAILED DESCRIPTION OF THE INVENTION

The invention is set out in the appended set of claims. In addition, any reference to methods of treatment in the subsequent paragraphs of this description is to be interpreted as references to the compounds, pharmaceutical compositions and medicaments of the present invention for use in a method for treatment of the human or animal body by therapy.

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

As used in the specification and the claims, the singular forms "a", "an", and "the" include plural referents unless the context clearly dictates otherwise. By way of example, "a compound" means one compound or more than one compound.

The terms described above and others used in the specification are well understood to those in the art.

Unless a context dictates otherwise, asterisks are used herein to indicate the point at which a mono- or bivalent radical depicted is connected to the structure to which it relates and of which the radical forms part.

### COMPOUNDS

As already defined herein above, in a first aspect, the present invention provides a compound of Formula I or a stereoisomer, tautomer, racemic, salt, hydrate, or solvate thereof, 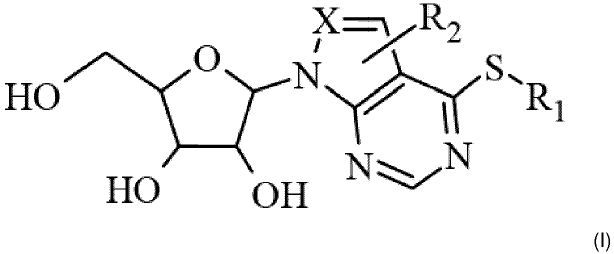 wherein
X is N;
R₁ is selected from the group comprising: -C₂₋₆alkyl, -C₂₋₆alkenyl, -CF₃, -C₁₋₆alkyl-R₃, -Ar₁, -Het₁ and - Cy₁;
R₂ is selected from the group comprising: -H or -halo;
R₃ is selected from the group comprising: -C₁₋₆alkyl, -C₁₋₆alkenyl, -C₁₋₆alkynyl, -Ar₂, -Het₂ and -Cy₂,
Ar₁ and Ar₂ are each independently selected from a 5- to 10-membered aromatic cycle optionally comprising 1 to 3 heteroatoms selected from O, N and S; each of said Ar₁ and Ar₂ optionally being substituted with one or more substituents independently selected from the list comprising: -halo, - CF₃, -C₁₋₆alkyl, -C₁₋₆alkenyl, -C₁₋₆alkynyl, -O-C₁₋₆alkyl, and -S-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -CF₃, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 3- to 10-membered non-aromatic cycle; each of said Cy₁ and Cy₂ optionally being substituted with one or more substituents independently selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl.

In a further aspect, the present invention provides a compound of Formula I or a stereoisomer, tautomer, racemic, salt, hydrate, or solvate thereof, 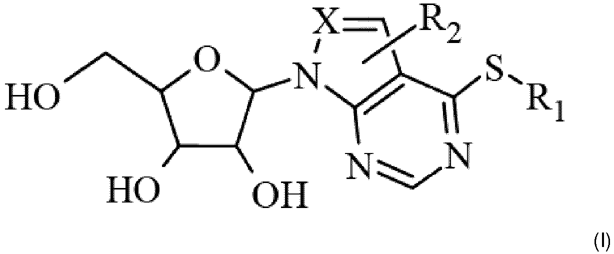 wherein
X is N;
R₁ is selected from the group comprising: -C₂₋₆alkyl, -C₂₋₆alkenyl, -CF₃, -C₁₋₆alkyl-R₃, -Ar₁, -C₂₋₆alkyl-Ar₁, -Het₁ and -Cy₁;
R₂ is -H or -halo;
R₃ is selected from the group comprising: -C₁₋₆alkyl, -C₁₋₆alkenyl, -C₁₋₆alkynyl, -Het₂ and -Cy₂,
Ar₁ is a 5- to 10-membered aromatic cycle optionally comprising 1 to 3 heteroatoms selected from O, N and S; said Ar₁ optionally being substituted with one or more substituents independently selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -CF₃, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 3- to 10-membered non-aromatic cycle; each of said Cy₁ and Cy₂ optionally being substituted with one or more substituents independently selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl.

When describing the compounds of the invention, the terms used are to be construed in accordance with the following definitions, unless a context dictates otherwise.

The term "alkyl" by itself or as part of another substituent refers to a fully saturated hydrocarbon of Formula CₓH₂ₓ₊₁ wherein x is a number greater than or equal to 1. Generally, alkyl groups of this invention comprise from 1 to 20 carbon atoms; more specifically from 1 to 6 carbon atoms. Alkyl groups may be linear or branched and may be substituted as indicated herein. When a subscript is used herein following a carbon atom, the subscript refers to the number of carbon atoms that the named group may contain. Thus, for example, C₁₋₆alkyl means an alkyl of one to six carbon atoms. Examples of alkyl groups are methyl, ethyl, n-propyl, i-propyl, butyl, and its isomers (e.g. n-butyl, i-butyl and t-butyl); pentyl and its isomers, hexyl and its isomers.

The term "alkenyl", as used herein, unless otherwise indicated, means straight-chain, cyclic, or branched-chain hydrocarbon radicals containing at least one carbon-carbon double bond. Examples of alkenyl radicals include ethenyl, E- and Z-propenyl, isopropenyl, E- and Z-butenyl, E- and Z-isobutenyl, E- and Z-pentenyl, E- and Z-hexenyl, E,E-, E,Z-, Z,E-, Z,Z-hexadienyl, and the like.

The term "alkynyl", as used herein, unless otherwise indicated, means straight-chain or branched-chain hydrocarbon radicals containing at least one carbon-carbon triple bond. Examples of alkynyl radicals include ethynyl, propynyl, butynyl, isobutynyl, and pentynyl, hexynyl, and the like.

Where alkyl groups as defined are divalent, i.e., with two single bonds for attachment to two other groups, they are termed "alkylene" groups. Non-limiting examples of alkylene groups includes methylene, ethylene, methylmethylene, trimethylene, propylene, tetramethylene, ethylethylene, 1,2-dimethylethylene, pentamethylene and hexamethylene. Similarly, where alkenyl groups as defined above and alkynyl groups as defined above, respectively, are divalent radicals having single bonds for attachment to two other groups, they are termed "alkenylene" and "alkynylene" respectively.

The term "cycloalkyl" by itself or as part of another substituent is a cyclic alkyl group, that is to say, a monovalent, saturated, or unsaturated hydrocarbyl group having 1, 2, or 3 cyclic structures. Cycloalkyl includes all saturated or partially saturated (containing 1 or 2 double bonds) hydrocarbon groups containing 1 to 3 rings, including monocyclic, bicyclic, or polycyclic alkyl groups. Cycloalkyl groups may comprise 3 or more carbon atoms in the ring and generally, according to this invention comprise from 3 to 10 atoms. Examples of cycloalkyl groups include but are not limited to cyclopropyl, cyclobutyl, cyclopentyl, cyclohexyl, cycloheptyl, cyclooctyl, cyclononyl, adamantanyl and cyclodecyl. An "optionally substituted cycloalkyl" refers to a cycloalkyl having optionally one or more substituents (for example 1 to 3 substituents, for example 1, 2, 3 or 4 substituents), typically selected from the list comprising -halo, -CF₃, -C₁₋₆alkyl, -C₁₋₆alkenyl, -C₁₋₆alkynyl, -O-C₁₋₆alkyl, and -S-C₁₋₆alkyl.

The terms "Het", "heterocyclyl" or "heterocyclo" as used herein by itself or as part of another group refer to non-aromatic cyclic groups (for example, 3 to 13 membered monocyclic, 7 to 17 membered bicyclic, or 10 to 20 membered tricyclic ring systems, or containing a total of 3 to 10 ring atoms) which have at least one heteroatom in at least one carbon atom-containing ring. Each ring of the heterocyclic group containing a heteroatom may have 1, 2, 3 or 4 heteroatoms selected from nitrogen atoms, oxygen atoms and/or sulfur atoms, where the nitrogen and sulfur heteroatoms may optionally be oxidized and the nitrogen heteroatoms may optionally be quaternized. The heterocyclic group may be attached at any heteroatom or carbon atom of the ring or ring system, where valence allows. Exemplary heterocyclic groups include piperidinyl, azetidinyl, imidazolinyl, imidazolidinyl, isoxazolinyl, oxazolidinyl, isoxazolidinyl, thiazolidinyl, isothiazolidinyl, piperidyl, succinimidyl, 3H-indolyl, isoindolinyl, chromenyl, isochromanyl, xanthenyl, 2H-pyrrolyl, 1-pyrrolinyl, 2-pyrrolinyl, 3-pyrrolinyl, pyrrolidinyl, 4H-quinolizinyl, 4aH-carbazolyl, 2-oxopiperazinyl, piperazinyl, homopiperazinyl, 2-pyrazolinyl, 3-pyrazolinyl, pyranyl, dihydro-2H-pyranyl, 4H-pyranyl, 3,4-dihydro-2H-pyranyl, phthalazinyl, oxetanyl, thietanyl, 3-dioxolanyl, 1,3-dioxanyl, 2,5-dioximidazolidinyl, 2,2,4-piperidonyl, 2-oxopiperidinyl, 2-oxopyrrolodinyl, 2-oxoazepinyl, indolinyl, tetrahydropyranyl, tetrahydrofuranyl, tetrahydrothienyl, tetrahydroquinolinyl, tetrahydroisoquinolinyl, thiomorpholinyl, thiomorpholinyl sulfoxide, thiomorpholinyl sulfone, 1,3-dioxolanyl, 1,4-oxathianyl, 1,4-dithianyl, 1,3,5-trioxanyl, 6H-1,2,5-thiadiazinyl, 2H-1,5,2-dithiazinyl, 2H-oxocinyl, 1H-pyrrolizinyl, tetrahydro-1,1-dioxothienyl, N-formylpiperazinyl, and morpholinyl.

The term "aryl" as used herein refers to an aromatic hydrocarbyl group having a single ring (i.e. phenyl) or multiple aromatic rings fused together (e.g. naphthalene or anthracene) or linked covalently, typically containing 5 to 10 atoms; wherein at least one ring is aromatic. The aromatic ring may optionally include one to three additional rings (either cycloalkyl, heterocyclyl, or heteroaryl) fused thereto. Aryl is also intended to include the partially hydrogenated derivatives of the carbocyclic systems enumerated herein. Non-limiting examples of aryl comprise phenyl, biphenylyl, biphenylenyl, 5- or 6-tetralinyl, 1-, 2-, 3-, 4-, 5-, 6-, 7-, or 8-azulenyl, 1- or 2-naphthyl, 1-, 2-, or 3-indenyl, 1-, 2-, or 9-anthryl, 1- 2-, 3-, 4-, or 5-acenaphtylenyl, 3-, 4-, or 5-acenaphtenyl, 1-, 2-, 3-, 4-, or 10-phenanthryl, 1- or 2-pentalenyl, 1, 2-, 3-, or 4-fluorenyl, 4- or 5-indanyl, 5-, 6-, 7-, or 8-tetrahydronaphthyl, 1,2,3,4-tetrahydronaphthyl, 1,4-dihydronaphthyl, dibenzo[a,d]cylcoheptenyl, and 1-, 2-, 3-, 4-, or 5-pyrenyl. The aryl ring can optionally be substituted by one or more substituents. An "optionally substituted aryl" refers to an aryl having optionally one or more substituents (for example 1 to 5 substituents, for example 1, 2, 3 or 4) at any available point of attachment. Non-limiting examples of such substituents are selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl.

Where a carbon atom in an aryl group is replaced with a heteroatom, the resultant ring is referred to herein as a heteroaryl ring.

The term "heteroaryl" as used herein by itself or as part of another group refers but is not limited to 5 to 10 carbon-atom aromatic rings or ring systems containing 1 to 3 rings which are fused together or linked covalently, typically containing 5 to 8 atoms; at least one of which is aromatic in which one or more carbon atoms in one or more of these rings can be replaced by oxygen, nitrogen or sulfur atoms where the nitrogen and sulfur heteroatoms may optionally be oxidized and the nitrogen heteroatoms may optionally be quaternized. Such rings may be fused to an aryl, cycloalkyl, heteroaryl or heterocyclyl ring. Non-limiting examples of such heteroaryl, include: pyrrolyl, furanyl, thiophenyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, 1,2,3-triazinyl, 1,2,4-triazinyl, 1,3,5-triazinyl, 1,2,3,4-tetrazinyl, 1,2,3,5-tetrazinyl, 1,2,4,5-tetrazinyl, oxazinyl, dioxinyl, thiazinyl, triazinyl, imidazo[2,1-b][1,3]thiazolyl, thieno[3,2-b]furanyl, thieno[3,2-b]thiophenyl, thieno[2,3-d][1,3]thiazolyl, thieno[2,3-d]imidazolyl, tetrazolo[1,5-a]pyridinyl, indolyl, indolizinyl, isoindolyl, benzofuranyl, benzopyranyl, 1(4H)-benzopyranyl, 1(2H)-benzopyranyl, 3,4-dihydro-1(2H)-benzopyranyl, 3,4-dihydro-1(2H)-benzopyranyl, isobenzofuranyl, benzothiophenyl, isobenzothiophenyl, indazolyl, benzimidazolyl, 1,3-benzoxazolyl, 1,2-benzisoxazolyl, 2,1-benzisoxazolyl, 1,3-benzothiazolyl, 1,2-benzoisothiazolyl, 2,1-benzoisothiazolyl, benzotriazolyl, 1,2,3-benzoxadiazolyl, 2,1,3-benzoxadiazolyl, 1,2,3-benzothiadiazolyl, 2,1,3-benzothiadiazolyl, thienopyridinyl, purinyl, imidazo[1,2-a]pyridinyl, 6-oxo-pyridazin-1(6H)-yl, 2-oxopyridin-1(2H)-yl, 6-oxo-pyridazin-1(6H)-yl, 2-oxopyridin-1(2H)-yl, 1,3-benzodioxolyl, quinolinyl, isoquinolinyl, cinnolinyl, quinazolinyl, quinoxalinyl, phthalazinyl, 1,2,3-benzotriazinyl, 1,2,4-benzotriazinyl, 7-azaindolyl, 6-azaindolyl, 5-azaindolyl, 4-azaindolyl, 1,5-naphtyridinyl, 1,6-naphtyridinyl, 1,7-naphtyridinyl, 1,8-naphtyridinyl, 2,6-naphtyridinyl, 2,7-naphtyridinyl.

An "optionally substituted heteroaryl" refers to a heteroaryl having optionally one or more substituents (for example 1 to 4 substituents, for example 1, 2, 3 or 4), selected from the list comprising: : -halo, - CF₃, -C₁₋₆alkyl, -C₁₋₆alkenyl, -C₁₋₆alkynyl, -O-C₁₋₆alkyl, and -S-C₁₋₆alkyl.

Whenever the term "substituted" is used in the present invention, it is meant to indicate that one or more hydrogens on the atom indicated in the expression using "substituted" is replaced with a selection from the indicated group, provided that the indicated atom's normal valency is not exceeded, and that the substitution results in a chemically stable compound, i.e. a compound that is sufficiently robust to survive isolation to a useful degree of purity from a reaction mixture, and formulation into a therapeutic agent.

Where groups may be optionally substituted, such groups may be substituted once or more, and preferably once, twice or thrice. Substituents may be selected from, for example, the group comprising: -halo, -CF₃, -C₁₋₆alkyl, -C₁₋₆alkenyl, -C₁₋₆alkynyl, -O-C₁₋₆alkyl, and -S-C₁₋₆alkyl.

As used herein the terms such as "alkyl, aryl, or cycloalkyl, each being optionally substituted with" or "alkyl, aryl, or cycloalkyl, optionally substituted with" refers to optionally substituted alkyl, optionally substituted aryl and optionally substituted cycloalkyl.

The term "halo" or "halogen" as a group or part of a group is generic for fluoro, chloro, bromo, or iodo. In the context of the present invention, chloro is a preferred halo group.

Whenever used in the present invention the term "compounds of the invention" or a similar term is meant to include the compounds of general Formula I, II, and any subgroup thereof. This term also refers to the exemplified compounds as depicted in Table 1, their derivatives, N-oxides, salts, solvates, hydrates, stereoisomeric forms, racemic mixtures, tautomeric forms, optical isomers, analogues, esters, as well as their quaternized nitrogen analogues. The N-oxide forms of said compounds are meant to comprise compounds wherein one or several nitrogen atoms are oxidized to the so-called N-oxide.

In a further embodiment, the present invention provides a compound as defined herein, wherein:
X is C;
R₁ is selected from the group comprising: -C₂₋₆alkyl, -C₂₋₆alkenyl, -CF₃, -C₁₋₆alkyl-R₃, -Ar₁, -C₂₋₆alkyl-Ar₁, -Het₁ and -Cy₁;
R2 is -H;
R₃ is selected from the group comprising: -C₁₋₆alkyl, -C₁₋₆alkenyl, -C₁₋₆alkynyl, -Het₂ and -Cy₂,
Ar₁ is a 5- to 10-membered aromatic cycle optionally comprising 1 to 3 heteroatoms selected from O, N and S; said Ar₁ optionally being substituted with one or more substituents independently selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -CF₃, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 3- to 10-membered non-aromatic cycle; each of said Cy₁ and Cy₂ optionally being substituted with one or more substituents independently selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl.

In another particular embodiment, the present invention provides a compound as defined herein wherein:
R₁ is selected from the group comprising: -C₂₋₆alkyl, -C₁₋₆alkyl-R₃, -C₂₋₆alkyl-Ar₁, -Ar₁, -Het₁ and -Cy₁;
R2 is H;
R₃ is selected from the group comprising: -C₁₋₆alkyl, -C₁₋₆alkenyl, -Het₂ and -Cy₂,
X is C
Ar₁ is a 5- to 6-membered aromatic cycle, said Ar₁ optionally being substituted with one or more substituents independently selected from the list comprising: -halo, -C₁₋₆alkyl, and -O-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -CF₃, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 5- to 6-membered non-aromatic cycle; each of said Cy₁ and Cy₂ optionally being substituted with one or more substituents independently selected from the list comprising: -halo, -C₁₋₆alkyl, and -O-C₁₋₆alkyl.

In yet another particular embodiment, the present invention provides a compound as defined herein wherein:
R₁ is selected from the group comprising: -C₂₋₆alkyl, -C₁₋₃alkyl-R₃, -C₂₋₃alkyl-Ar₁, -Ar₁, -Het₁ and -Cy₁;
R2 is H;
R₃ is selected from the group comprising: -C₁₋₆alkyl, -C₁₋₆alkenyl, - Het₂ and -Cy₂,
X is C;
Ar₁ is -phenyl; optionally being substituted with one or more substituents selected from the list comprising: -halo, -C₁₋₆alkyl, and -O-C₁₋₆alkyl;
Het₁ and Het₂ are each independently -pyridinyl;
Cy₁ and Cy₂ are each independently selected from - cyclopentane and -cycloheptane.

In another embodiment, the present invention provides a compound as defined herein wherein:
R₁ is selected from the group comprising: -C₂₋₆alkyl, -C₁₋₃alkyl-R₃, -C₂₋₃alkyl-Ar₁, -Ar₁, -Het₁ and -Cy₁;
R₂ is H;
R₃ is selected from the group comprising: -C₁₋₆alkyl, -C₁₋₆alkenyl, and -Cy₂,
X is C;
Ar₁ is -phenyl optionally being substituted with one or more substituents selected from the list comprising: -halo, -C₁₋₆alkyl, and -O-C₁₋₆alkyl;
Het₁ is -pyridinyl;
Cy₁ and Cy₂ are each independently selected from -cyclopentane and -cycloheptane.

In a further embodiment, the present invention provides a compound of Formula I or a stereoisomer, tautomer, racemic, salt, hydrate, or solvate thereof, 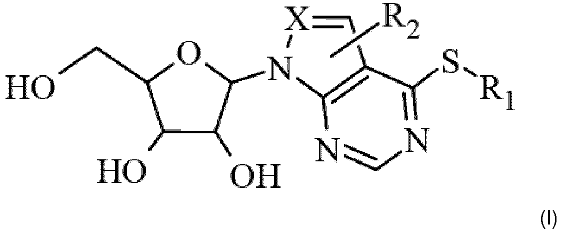 wherein
X is C;
R₁ is selected from the group comprising: -C₂₋₆alkyl, -CF₃, -C₁₋₆alkyl-R₃, -Ar₁, -Het₁ and -Cy₁;
R₂ is selected from the group comprising: -H or -halo;
R₃ is selected from the group comprising: -C₁₋₆alkyl, -C₁₋₆alkenyl, -C₁₋₆alkynyl, -Ar₂, -Het₂ and -Cy₂,
Ar₁ and Ar₂ are each independently selected from a 5- to 10-membered aromatic cycle optionally comprising 1 to 3 heteroatoms selected from O, N and S; each of said Ar₁ and Ar₂ optionally being substituted with one or more substituents independently selected from the list comprising: -halo, - CF₃, -C₁₋₆alkyl, -C₁₋₆alkenyl, -C₁₋₆alkynyl, -O-C₁₋₆alkyl, and -S-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -CF₃, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 3- to 10-membered non-aromatic cycle; each of said Cy₁ and Cy₂ optionally being substituted with one or more substituents independently selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl.

In yet a further embodiment, the present invention provides a compound of Formula I or a stereoisomer, tautomer, racemic, salt, hydrate, or solvate thereof, 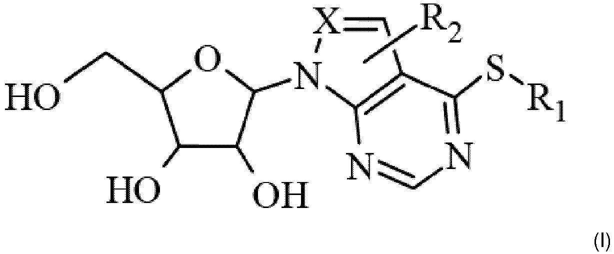 wherein
X is C;
R₁ is selected from the group comprising: -C₂₋₆alkyl, -C₂₋₆alkenyl, -CF₃, -C₁₋₆alkyl-R₃, -Ar₁, -Het₁ and - Cy₁;
R₂ is selected from the group comprising: -H or Cl;
R₃ is selected from the group comprising: -C₁₋₆alkyl, -C₁₋₆alkenyl, -C₁₋₆alkynyl, -Ar₂, -Het₂ and -Cy₂,
Ar₁ and Ar₂ are each independently selected from a 5- to 10-membered aromatic cycle optionally comprising 1 to 3 heteroatoms selected from O, N and S; each of said Ar₁ and Ar₂ optionally being substituted with one or more substituents independently selected from the list comprising: -Cl, - CF₃, -C₁₋₆alkyl, -C₁₋₆alkenyl, -C₁₋₆alkynyl, -O-C₁₋₆alkyl, and -S-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - Cl, -CF₃, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 3- to 10-membered non-aromatic cycle; each of said Cy₁ and Cy₂ optionally being substituted with one or more substituents independently selected from the list comprising: -Cl, -CF₃, -C₁₋₆alkyl, -C₁₋₆alkenyl, -C₁₋₆alkynyl, -O-C₁₋₆alkyl, and -S-C₁₋₆alkyl.

In yet a further embodiment, the present invention provides a compound of Formula I or a stereoisomer, tautomer, racemic, salt, hydrate, or solvate thereof,

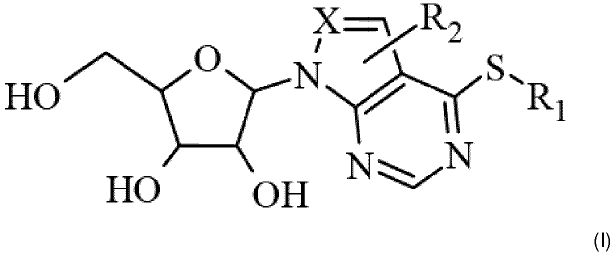

Wherein one or more of the following applies
X is C;
R₁ is selected from the group comprising: -C₂₋₆alkyl, -C₂₋₆alkenyl, -CF₃, -C₁₋₆alkyl-R₃, -Ar₁, -Het₁ and - Cy₁;
R₂ is selected from the group comprising: -H or -halo;
R₃ is selected from the group comprising: -C₁₋₆alkyl, -C₁₋₆alkenyl, -C₁₋₆alkynyl, -Ar₂, -Het₂ and -Cy₂,
Ar₁ and Ar₂ are each independently selected from a 5- to 10-membered aromatic cycle optionally comprising 1 to 3 heteroatoms selected from O, N and S; each of said Ar₁ and Ar₂ optionally being substituted with one or more substituents independently selected from the list comprising: -halo, - CF₃, -C₁₋₆alkyl, -C₁₋₆alkenyl, -C₁₋₆alkynyl, -O-C₁₋₆alkyl, and -S-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -CF₃, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 3- to 10-membered non-aromatic cycle; each of said Cy₁ and Cy₂ optionally being substituted with one or more substituents independently selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl.

In another embodiment, the present invention provides a compound of Formula la or a stereoisomer, tautomer, racemic, salt, hydrate, or solvate thereof, 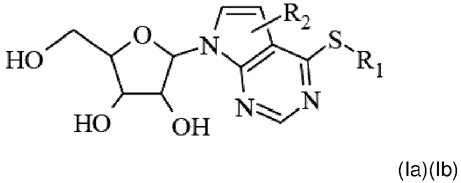 wherein
R₁ is selected from the group comprising: -C₁₋₆alkyl, -C₂₋₆alkenyl, -CF₃, -C₁₋₆alkyl-R₃, -Ar₁, -Het₁ and - Cy₁;
R₂ is selected from the group comprising: -H or -halo;
R₃ is selected from the group comprising: -C₁₋₆alkyl, -C₁₋₆alkenyl, -C₁₋₆alkynyl, -Ar₂, -Het₂ and -Cy₂,
X is selected from the group comprising: C and N;
Ar₁ and Ar₂ are each independently selected from a 5- to 10-membered aromatic cycle optionally comprising 1 to 3 heteroatoms selected from O, N and S; each of said Ar₁ and Ar₂ optionally being substituted with one or more substituents independently selected from the list comprising: -halo, - CF₃, -C₁₋₆alkyl, -C₁₋₆alkenyl, -C₁₋₆alkynyl, -O-C₁₋₆alkyl, and -S-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -CF₃, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 3- to 10-membered non-aromatic cycle; each of said Cy₁ and Cy₂ optionally being substituted with one or more substituents independently selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl.

In another embodiment, the present invention provides a compound of Formula la or a stereoisomer, tautomer, racemic, salt, hydrate, or solvate thereof, 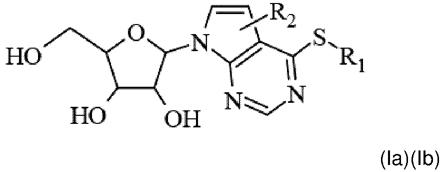 wherein
R₁ is selected from the group comprising: -C₁₋₆alkyl, -C₂₋₆alkenyl, -CF₃, -C₁₋₆alkyl-R₃, -C₁₋₆alkyl-Ar₁, - Ar₁, -Het₁ and -Cy₁;
R₂ is -H or -halo;
R₃ is selected from the group comprising: -C₁₋₆alkyl, -C₁₋₆alkenyl, -C₁₋₆alkynyl, -Het₂ and -Cy₂,
X is selected from the group comprising: C and N;
Ar₁ is selected from a 5- to 10-membered aromatic cycle optionally comprising 1 to 3 heteroatoms selected from O, N and S; said Ar₁ optionally being substituted with one or more substituents independently selected from the list comprising: -halo, -CF₃, -C₁₋₆alkyl, -C₁₋₆alkenyl, -C₁₋₆alkynyl, - O-C₁₋₆alkyl, and -S-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -CF₃, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 3- to 10-membered non-aromatic cycle; each of said Cy₁ and Cy₂ optionally being substituted with one or more substituents independently selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl.

In a further embodiment, the present invention provides a compound as defined herein wherein;
R₁ is selected from the group comprising: -C₂₋₆alkyl, -C₁₋₆alkyl-R₃, -Ar₁, and -Cy₁;
R2 is H;
R₃ is selected from the group comprising: -C₁₋₆alkyl, -C₁₋₆alkenyl, -Ar₂, and -Cy₂,
X is C
Ar₁ and Ar₂ are each independently selected from a 5- to 6-membered aromatic cycle; each of said Ar₁ and Ar₂ optionally being substituted with one or more substituents independently selected from the list comprising: -halo, -C₁₋₆alkyl, and -O-C₁₋₆alkyl;
Cy₁ and Cy₂ are each independently selected from a 5- to 6-membered non-aromatic cycle; each of said Cy₁ and Cy₂ optionally being substituted with one or more substituents independently selected from the list comprising: -halo, -C₁₋₆alkyl, and -O-C₁₋₆alkyl.

In yet a further embodiment, the present invention provides a compound as defined herein wherein;
R₁ is selected from the group comprising: -C₂₋₆alkyl, -C₁₋₃alkyl-R₃, -Ar₁, and -Cy₁;
R₃ is selected from the group comprising: -C₁₋₆alkyl, -C₁₋₆alkenyl, -Ar₂, and -Cy₂,
X is C;
Ar₁ and Ar₂ are each independently -phenyl; each of said -phenyl optionally being substituted with a substituent selected from the list comprising: -halo, -C₁₋₆alkyl, and -O-C₁₋₆alkyl;
Cy₁ and Cy₂ are each independently selected from - cyclopentane and -cycloheptane.

In a still further embodiment, the present invention provides a compound as defined herein wherein;
R₁ is selected from the group comprising: -C₂₋₆alkyl, -C₁₋₃alkyl-R₃, -Ar₁, and -Cy₁;
R₃ is selected from the group comprising: -C₁₋₆alkyl, -Ar₂, and -Cy₂,
X is C;
Ar₁ and Ar₂ are each independently -phenyl; each of said -phenyl optionally being substituted with a substituent selected from the list comprising: -halo, -C₁₋₆alkyl, and -O-C₁₋₆alkyl;
Cy₁ and Cy₂ are each independently selected from -cyclopentane and -cycloheptane.

In yet a further embodiment, the present invention provides a compound as defined herein wherein;
R₁ is selected from the group comprising: -C₂₋₆alkyl, -C₁₋₃alkyl-R₃, and -Ar₁;
R₃ is selected from the group comprising: -C₁₋₆alkyl, -C₁₋₆alkenyl, and -Cy₂;
X is C;
Ar₁ is -phenyl optionally being substituted with -O-C₁₋₆alkyl; and
Cy₂ is -cyclopentane.

As described herein, some of the compounds of the invention may contain one or more asymmetric carbon atoms that serve as a chiral center, which may lead to different optical forms (e.g. enantiomers or diastereoisomers). The invention comprises all such optical forms in all possible configurations, as well as mixtures thereof. It will be clear to the skilled person that the compounds of the invention may exist in the form of different isomers and/or tautomers, including but not limited to geometrical isomers, conformational isomers, E/Z-isomers, stereochemical isomers (i.e. enantiomers and diastereoisomers) and isomers that correspond to the presence of the same substituents on different positions of the rings present in the compounds of the invention. All such possible isomers, tautomers and mixtures thereof are included within the scope of the invention.

In another particular embodiment, the compounds of the present invention have the stereochemistry as represented in formula II:

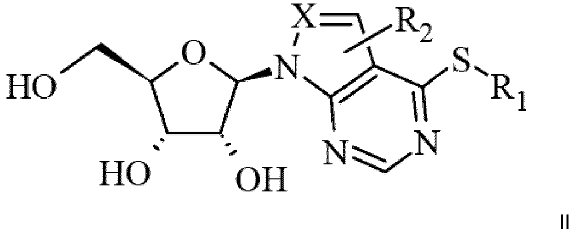

In the context of the present invention, in particular in respect of formula I**,** la, and/or II, one or more of the following applies alone or in combination with one another:
R₁ is selected from the group comprising: -C₂₋₆alkyl, -C₂₋₆alkenyl, -CF₃, -C₁₋₆alkyl-R₃, -Ar₁, -Het₁ and -Cy₁;
alternatively R₁ is selected from the group comprising: -C₂₋₆alkyl, -C₂₋₆alkenyl, -CF₃, -C₁₋₆alkyl-R₃,
-Ar₁, -C₂₋₆alkyl-Ar₁, -Het₁ and -Cy_{1;}
alternatively R₁ is selected from the group comprising: -C₂₋₆alkyl, --C₁₋₃alkyl-R₃, -C₂₋₃alkyl-Ar₁, -Ar₁, -Het₁ and -Cy₁
alternatively R₁ is selected from the group comprising: -C₂₋₆alkyl, -C₁₋₆alkyl-R₃, -Ar₁, and -Cy₁;
alternatively R₁ is selected from the group comprising: -C₂₋₆alkyl, -C₁₋₃alkyl-R₃, -Ar₁, and -Cy₁;

R₂ is selected from the group comprising: -H or -halo;
   alternatively R₂ is -Cl or -H
   alternatively R₂ is -H;
   alternatively R₂ is -halo;
R₃ is selected from the group comprising: -C₁₋₆alkyl, -C₁₋₆alkenyl, -C₁₋₆alkynyl, -Ar₂, -Het₂ and -Cy₂,
   alternatively R₃ is selected from the group comprising: -C₁₋₆alkyl, -C₁₋₆alkenyl, -C₁₋₆alkynyl, -Het₂ and -Cy₂
   alternatively R₃ is selected from the group comprising: -C₁₋₆alkyl, -C₁₋₆alkenyl, -Ar₂, and -Cy₂,
   alternatively R₃ is selected from the group comprising: -C₁₋₆alkyl, -Ar₂, and -Cy₂;
   alternatively R₃ is selected from the group comprising: -C₁₋₆alkyl, -C₁₋₆alkenyl, -Het₂ and -Cy₂
   alternatively R₃ is selected from the group comprising: -C₁₋₆alkyl, -C₁₋₆alkenyl and -Cy₂
   alternatively R₃ is selected from the group comprising: -C₁₋₆alkyl, and -C₁₋₆alkenyl,
X is C
Ar₁ and Ar₂ are each independently selected from a 5- to 10-membered aromatic cycle optionally comprising 1 to 3 heteroatoms selected from O, N and S; each of said Ar₁ and Ar₂ optionally being substituted with one or more substituents independently selected from the list comprising: -halo, -
   CF₃, -C₁₋₆alkyl, -C₁₋₆alkenyl, -C₁₋₆alkynyl, -O-C₁₋₆alkyl, and -S-C₁₋₆alkyl;
   alternatively, Ar₁ and Ar₂ are each independently selected from a 5- to 6-membered aromatic cycle; each of said Ar₁ and Ar₂ optionally being substituted with one or more substituents independently selected from the list comprising: -halo, -C₁₋₆alkyl, and -O-C₁₋₆alkyl;
   alternatively Ar₁ and Ar₂ are each independently -phenyl; each of said -phenyl optionally being substituted with a substituent selected from the list comprising: -halo, -C₁₋₆alkyl, and -O-C₁₋₆alkyl; alternatively Ar₁ is -phenyl optionally being substituted with -O-C₁₋₆alkyl;
   alternatively Ar₁ is -phenyl optionally being substituted with -C₁₋₆alkyl
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -CF₃, -C₁₋₆alkyl, -C₁₋₆alkenyl, -C₁₋₆alkynyl, -O-C₁₋₆alkyl, and -S-C₁₋₆alkyl;
   alternatively Het₁ and Het₂ are -pyridinyl
   alternatively Het₁ and Het₂ are absent
Cy₁ and Cy₂ are each independently selected from a 3- to 10-membered non-aromatic cycle; each of said Cy₁ and Cy₂ optionally being substituted with one or more substituents independently selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl;
   alternatively Cy₁ and Cy₂ are each independently selected from a 5- to 6-membered non-aromatic cycle; each of said Cy₁ and Cy₂ optionally being substituted with one or more substituents independently selected from the list comprising: -halo, -C₁₋₆alkyl, and -O-C₁₋₆alkyl;
   alternatively Cy₁ and Cy₂ are each independently selected from -cyclopentane and -cycloheptane; alternatively Cy₂ is -cyclopentane.

The present application also provides a compound or reference compound (RC) selected from the list comprising:

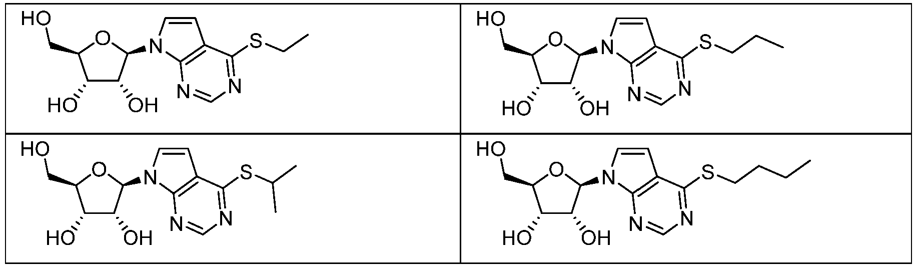

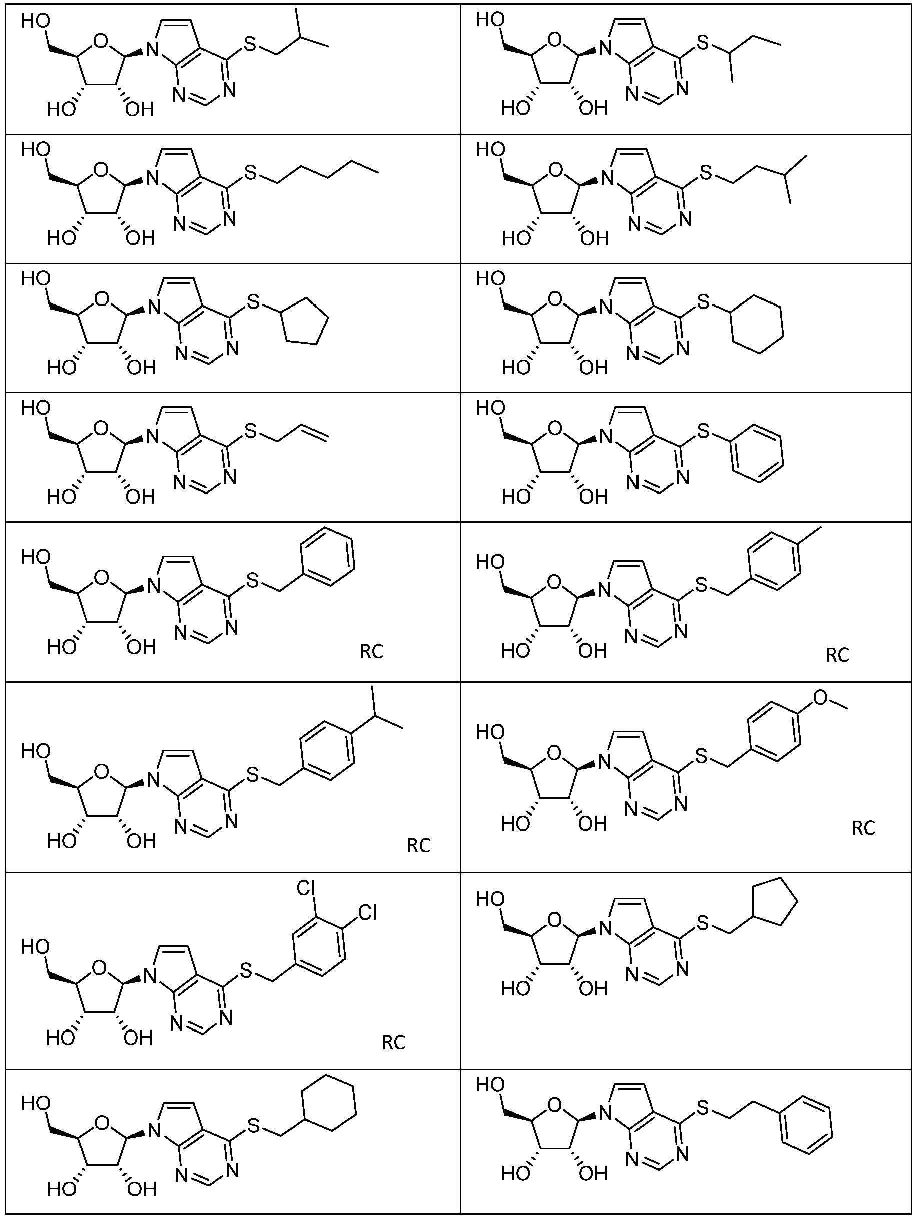

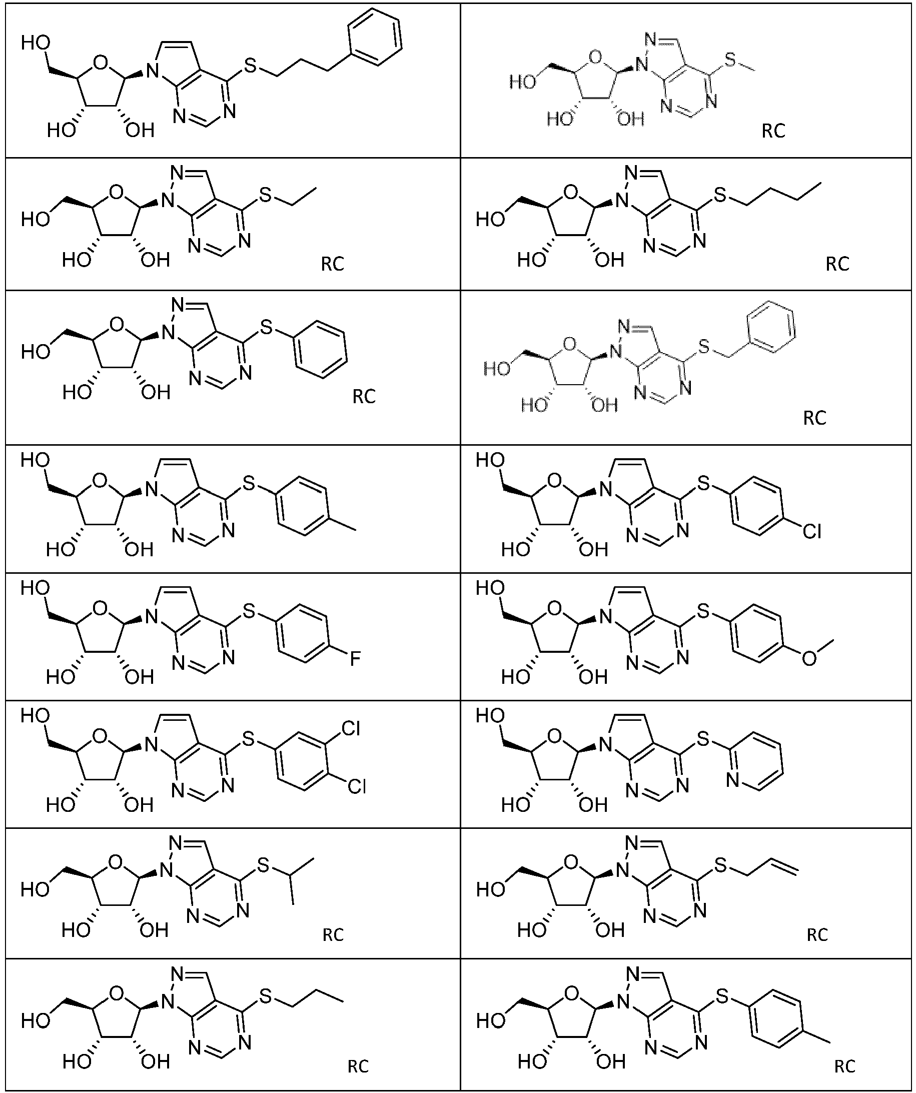

Alternatively, the present invention also provides a compound selected from the list comprising:

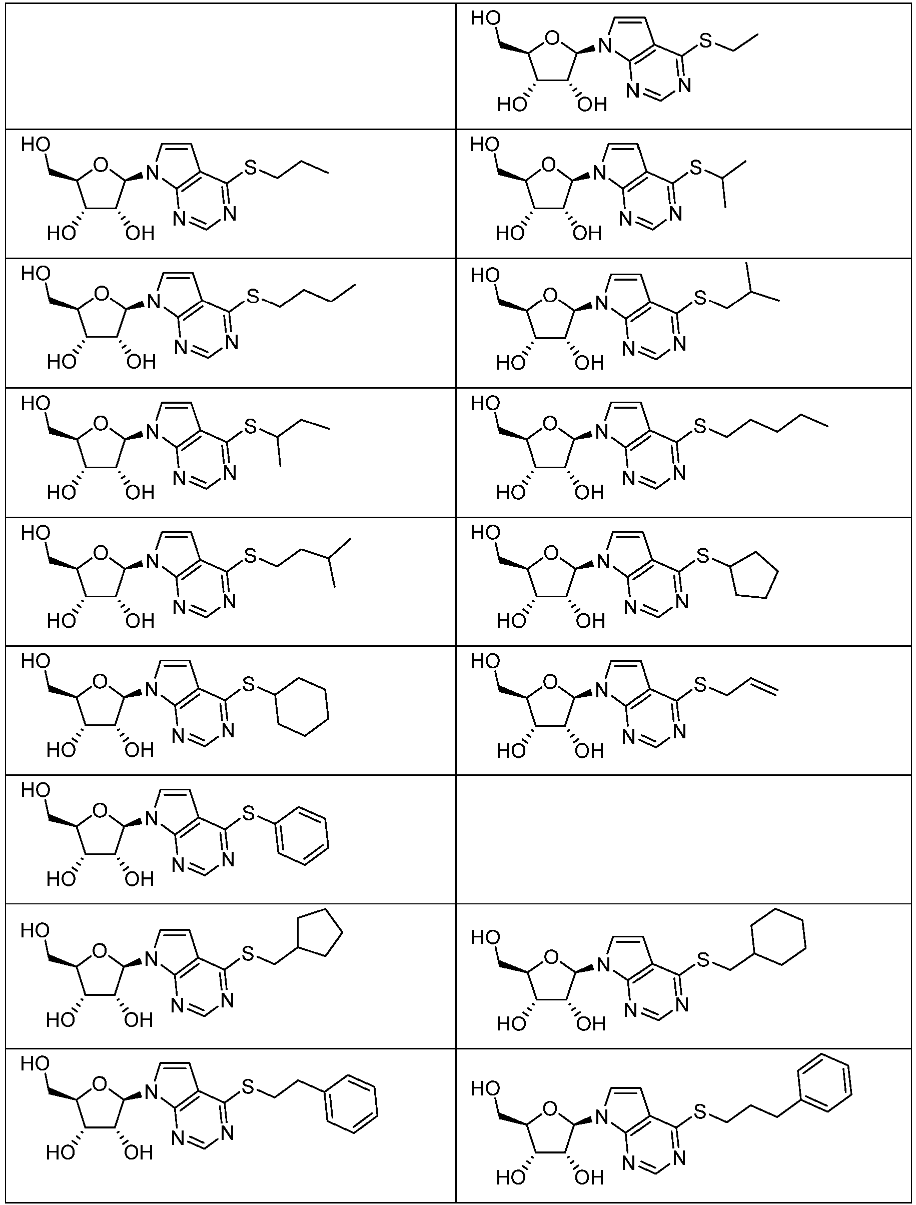

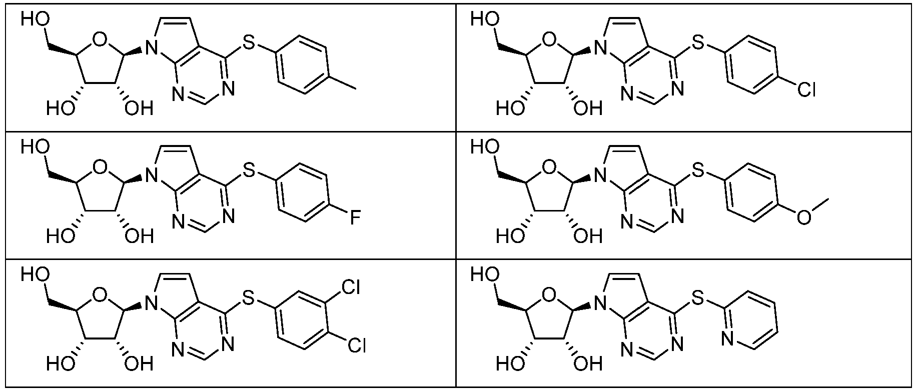

The present invention further provides a pharmaceutical composition comprising a compound as defined herein and at least one pharmaceutically acceptable carrier or diluent.

In a further aspect, the present invention provides a compound of Formula I or a stereoisomer, tautomer, racemic, salt, hydrate, or solvate thereof, or a pharmaceutical composition comprising such compound, for use in the diagnosis, prevention and/or treatment of a parasite infection, in particular a *Trypanosoma* infection 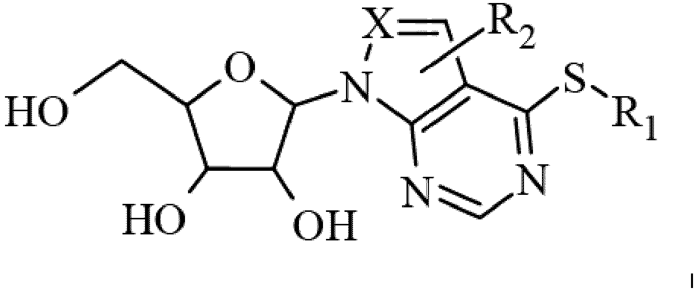 wherein
X is C;
R₁ is selected from the group comprising: -C₁₋₆alkyl, -C₁₋₆alkyl-R₃, -Ar₁, -Het₁ and -Cy₁;
R₂ is selected from the group comprising: -H or -halo;
R₃ is selected from the group comprising: -C₁₋₆alkyl, -C₁₋₆alkenyl, -C₁₋₆alkynyl, -Ar₂, -Het₂ and -Cy₂,
Ar₁ and Ar₂ are each independently selected from a 5- to 10-membered aromatic cycle optionally comprising 1 to 3 heteroatoms selected from O, N and S; each of said Ar₁ and Ar₂ optionally being substituted with one or more substituents independently selected from the list comprising: -halo, - C₁₋₆alkyl, -C₁₋₆alkenyl, -C₁₋₆alkynyl, -O-C₁₋₆alkyl, and -S-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 3- to 10-membered non-aromatic cycle; each of said Cy₁ and Cy₂ optionally being substituted with one or more substituents independently selected from the list comprising: -halo, -C₁₋₆alkyl, -C₁₋₆alkenyl, -C₁₋₆alkynyl, -O-C₁₋₆alkyl, and -S-C₁₋₆alkyl.

Accordingly, in a further aspect, the present invention provides a compound of Formula I or a stereoisomer, tautomer, racemic, salt, hydrate, or solvate thereof, or a pharmaceutical composition comprising such compound, for use in the diagnosis, prevention and/or treatment of a parasite infection, in particular a *Trypanosoma* infection; wherein said compound is selected from the below table;

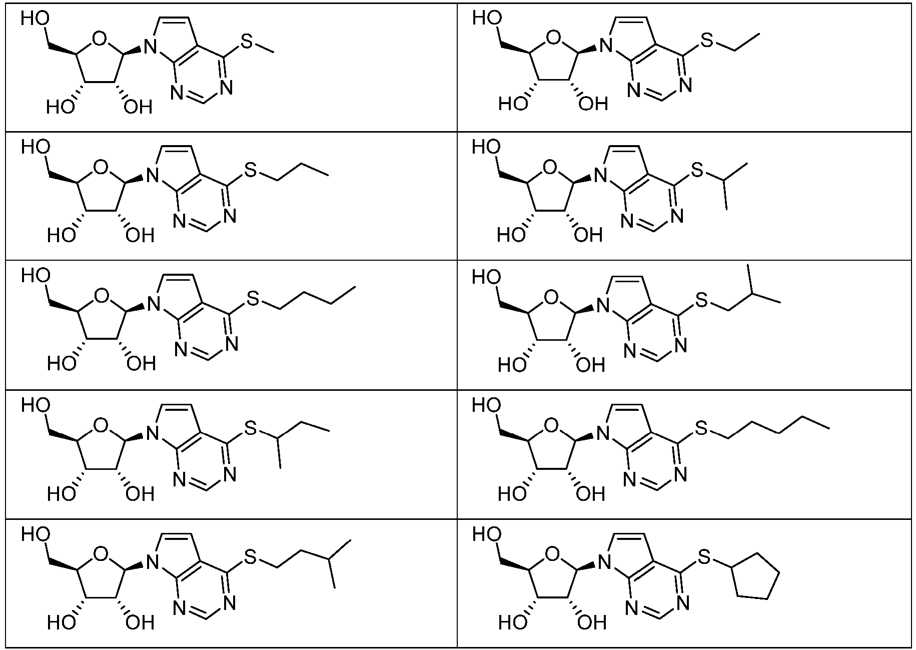

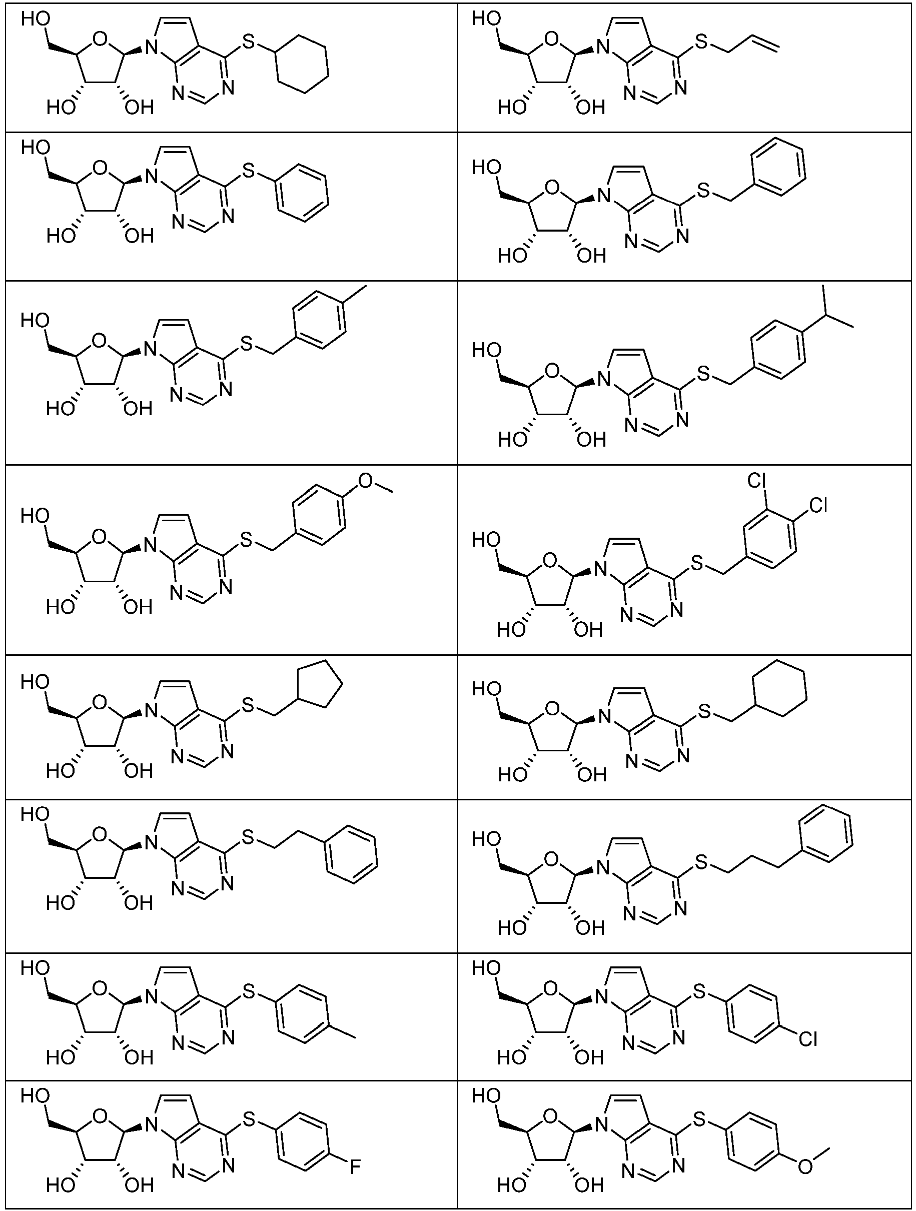

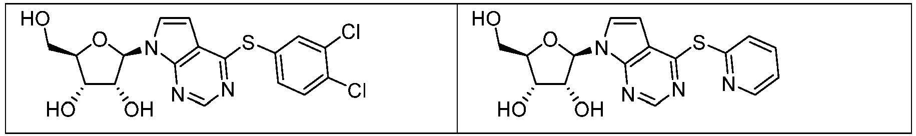

### USES

In one embodiment, the compounds of the present invention are useful as a medicament, specifically a human or veterinary medicine: in particular as anti-parasitic agents, more in particular as anti-protozoal agents. More in particular, compounds as provided herein are useful in the treatment of parasitic diseases such African trypanosomiasis and Chagas disease.

The present invention further provides a compound as defined herein or a (pharmaceutical) composition comprising said compound, for use as a human or veterinary medicine, in particular for use in the prevention and/or treatment of parasitic infections, more in particular for preventing and/or treating at least one infection, disease or disorder selected from the group comprising trypanosome infections (sleeping sickness or African trypanosomiasis, Chagas disease or American trypanosomiasis), leishmaniasis, malaria, trichomoniasis, cryptosporidiosis, histomoniasis or toxoplasmosis. A "trypanosome" refers to a protozoan parasite, which may infect a broad range of hosts, including mammals. In certain embodiments, a trypanosome is *Trypanosoma brucei* spp. Exemplary trypanosomal parasites include, but are not limited to, *Trypanosoma brucei gambiense, Trypanosoma brucei rhodesiense, Trypanosoma evansi (including T evansi type A and T. evansi type B), Trypanosoma congolense, Trypanosoma vivax,* and *Trypanosoma equiperdum.* In certain embodiments, a trypanosome is *Trypanosoma cruzi.*

In a preferred embodiment, the invention provides methods and uses of a compound as defined hereinbefore or of a composition comprising said compound(s) for the prevention and/or treatment of parasitic infections.

Human African trypanosomiasis (HAT), also known as "sleeping sickness", is a vector-borne parasitic disease. It is caused by infection with protozoan parasites belonging to the genus *Trypanosoma* (*T. brucei* spp.). They are transmitted to humans by tsetse fly (*Glossina* genus) bites which have acquired their infection from human beings or from animals harbouring human pathogenic parasites. In the first stage, the trypanosomes multiply in subcutaneous tissues, blood and lymph. This is also called the haemo-lymphatic stage, which entails bouts of fever, headaches, joint pains and itching. In the second stage the parasites cross the blood-brain barrier to infect the central nervous system. This is known as the neurological or meningo-encephalic stage (stage II). In general this is when more obvious signs and symptoms of the disease appear: changes of behaviour, confusion, sensory disturbances and poor coordination. Disturbance of the sleep cycle, which gives the disease its name, is an important feature. Without treatment, sleeping sickness is considered fatal although cases of healthy carriers have been reported. The compounds of the present invention are particularly useful for treating said "stage II" of HAT disease or sleeping sickness.

Animal trypanosomiasis is caused by a variety of trypanosomes species (e.g. *T. brucei, T. congolense, T. vivax, T. evansi* and *T. equiperdum*)*. T. vivax* causes *Souma* disease in several hosts including cattle, horses, sheep and dogs. Transmission of these parasites occurs mainly through the *Glossina* vector but can also occur mechanically by other hematophagous insects such as horse flies (fam. Tabanidae) or stable flies (*Stomoxys sp*.)*. T. congolense,* is another major causative agent of livestock trypanosome infections and also transmitted by tsetse flies. *T. evansi* is a hemoflagellate that is mechanically transmitted by hematophagous flies and causes *Surra* disease in livestock and wildlife. *T. equiperdum* causes *Dourine* in horses and is transmitted venereally. In general, all AT species can cause high morbidity and mortality in livestock, severely compromising animal husbandry and livestock rearing.

"Chagas disease" refers to a parasitic disease associated with or caused by infection with the protozoan parasite *Trypanosoma cruzi.* Chagas disease can comprise an acute phase which typically lasts from weeks to months and is often symptom-free. When symptoms do occur they can include swelling at the infection site, fever, fatigue, rash, body aches, eyelid swelling, headache, loss of appetite, nausea, diarrhea, vomiting, swollen glands and enlargement of the liver or spleen. The disease can also include a chronic phase that typically occurs 10 to 20 years after initial infection. In the chronic stage symptoms can includes irregular heartbeat, congestive heart failure, sudden cardiac arrest, difficulty swallowing due to enlargement of the esophagus, and/or abdominal pain or constipation due to enlargement of the colon. The compounds of the present invention are particularly useful for treating the "chronic" stage" of Chagas disease.

The present invention further provides a method for the prevention and/or treatment of at least one parasitic infection or infestation in a mammalian subject, in particular a human being or an animal, such as horses, catlle, sheep, goats..., more in particular in livestock. the method comprising the step of administering to the subject a therapeutically or prophylactically effective amount of a compound in accordance with the present invention.

A "parasitic infection" as used herein refers to an infection caused by any of the following parasites: *Plasmodium* spp., *P. falciparum, P. berghei, P. malariae, P. vivax, P. ovale, Cryptosporidium* spp., *Cryptosporidium parvum, C. hominis, Acanthanmoeba* spp., *Trypanosoma* spp. *Trypanosoma brucei brucei, T. b. rhodesiense, T. b. gambiense, T. evansi, T. equiperdum, T. simiae, T. vivax, T. congolense, T. cruzi, Leishmania* spp., *Leishmania donovani, L. major, L. mexicana, L. infantum, L. tropica, L. brazeliensis, Schistosoma* spp., *S. mansoni, S. haematobium, S. japonicum, Toxoplasma gondii, Trichomonas* spp., *Trichomonas vaginalis, Entamoeba invadens, Giardia* spp., *Giardia lamblia; Tritrichomonas* spp., *Histomonas meleagridis, Entamoeba* spp., *Limax* spp., *Acanthamoeba* spp., or *Eimeria* spp.

In a preferred embodiment, the invention provides a method for the prevention and/or treatment of parasitic infections, such as Trypanosoma infections, in particular infections by *Trypanosoma brucei, Trypanosoma cruzi* and/or *Trypanosoma vivax.*

Particularly preferred are the compounds of the invention having a nanomolar activity or having an IC50 value of less than 1 µM, in particular less than 0.5 µM, more in particular less than 0.1 µM, when tested in the *in vitro* assay as described herein.

For pharmaceutical use, the compounds of the invention may be used as a free acid or base, and/or in the form of a pharmaceutically acceptable acid-addition and/or base-addition salt (e.g. obtained with non-toxic organic or inorganic acid or base), in the form of a hydrate, solvate and/or complex. As used herein and unless otherwise stated, the term "solvate" includes any combination which may be formed by a compound of this invention with a suitable inorganic solvent (e.g. hydrates) or organic solvent. Such salts, hydrates, solvates, etc. and the preparation thereof will be clear to the skilled person; reference is for instance made to the salts, hydrates, solvates, etc.

The pharmaceutically acceptable salts of the compounds according to the invention, i.e. in the form of water-, oil-soluble, or dispersible products, include the conventional non-toxic salts or the quaternary ammonium salts which are formed, e.g., from inorganic or organic acids or bases. **In** addition, the basic nitrogen-containing groups may be quaternized with such agents as lower alkyl halides.

The present invention also relates to a pharmaceutical composition comprising one or more of the compounds as provided herein in admixture or in combination with a pharmaceutically acceptable carrier, diluent and/or excipient, and optionally an adjuvant or one or more further pharmaceutically active compounds.

The invention also provides a method of making a pharmaceutical composition comprising mixing one or more compounds of the invention with at least one pharmaceutically acceptable carrier, diluent or excipient and/or adjuvant, and optionally one or more further pharmaceutically active compounds (in particular anti-parasitic or anti-protozoal compounds).

By means of non-limiting examples, such a formulation may be in a form suitable for oral administration, for parenteral administration (such as by intravenous, intramuscular or subcutaneous injection or intravenous infusion), for topical administration (including ocular), for administration by inhalation, by a skin patch, by an implant, by a suppository, etc. Such suitable administration forms - which may be solid, semi-solid or liquid, depending on the manner of administration - as well as methods and carriers, diluents and excipients for use in the preparation thereof, will be clear to the skilled person.

Some preferred, but non-limiting examples of such preparations include tablets, pills, powders, lozenges, sachets, cachets, elixirs, suspensions, emulsions, solutions, syrups, aerosols, ointments, creams, lotions, soft and hard gelatin capsules, suppositories, eye drops, sterile injectable solutions and sterile packaged powders (which are usually reconstituted prior to use) for administration as a bolus and/or for continuous administration, which may be formulated with carriers, excipients, and diluents that are suitable per se for such formulations.

The preparations may be prepared in a manner known per se, which usually involves mixing at least one compound according to the invention with the one or more pharmaceutically acceptable carriers, and, if desired, in combination with other pharmaceutical active compounds, when necessary under aseptic conditions.

The pharmaceutical preparations of the invention are preferably in a unit dosage form, and may be suitably packaged, for example in a box, blister, vial, bottle, sachet, ampoule or in any other suitable single-dose or multi-dose holder or container (which may be properly labeled); optionally with one or more leaflets containing product information and/or instructions for use.

The compounds or compositions can be administered by a variety of routes including the oral, rectal, ocular, transdermal, subcutaneous, intravenous, intramuscular or intranasal routes, depending mainly on the specific preparation used and the condition to be treated or prevented. For humans, an oral administration is preferred whereas parenteral treatment is preferred for livestock. The at least one compound of the invention will generally be administered in an "effective amount", by which is meant any amount of a compound of the Formula I or II, or any subgroup thereof that, upon suitable administration, is sufficient to achieve the desired prophylactic or therapeutic benefit in the treatment of a condition or to delay or minimize (the development of) one or more symptoms associated with the condition in the subject to which it is administered. Usually, depending on the condition to be prevented or treated and the route of administration.

The amount(s) to be administered, the route of administration and the further treatment regimen may be determined by the treating clinician, depending on factors such as the age, gender and general condition of the patient and the nature and severity of the disease/symptoms to be treated. The term "therapeutically effective amount" can encompass an amount that improves overall therapy, reduces or avoids symptoms, , and/or enhances signs, or causes of the condition the therapeutic efficacy of another therapeutic agent. In certain embodiments, a therapeutically effective amount is effective for treating a disease. In certain embodiments, a therapeutically effective amount is effective for treating an infectious disease. In certain embodiments, a therapeutically effective amount is effective for treating Chagas disease. In certain embodiments, a therapeutically effective amount is effective for treating sleeping sickness. In certain embodiments, a therapeutically effective amount is effective for treating malaria. In certain embodiments, a therapeutically effective amount is effective for treating a parasitic infection. In certain embodiments, a therapeutically effective amount is effective for treating a protozoan infection. In certain embodiments, a therapeutically effective amount is effective for treating a trypanosomal infection. In certain embodiments, a therapeutically effective amount is effective for treating a *T*. *cruzi* infection. In certain embodiments, a therapeutically effective amount is effective for treating a *T. brucei* infection. In certain embodiments, a therapeutically effective amount is effective for treating a *T*. *vivax* infection. In certain embodiments, a therapeutically effective amount is effective for treating a *T. congolense* infection, In certain embodiments, a therapeutically effective amount is effective for treating a *T. evansi* infection, In certain embodiments, a therapeutically effective amount is effective for treating a *T. equiperdum* infection.

In accordance with the method of the present invention, said pharmaceutical composition can be administered separately at different times during the course of therapy or concurrently in divided or single combination forms. The present invention is therefore to be understood as embracing all such regimes of simultaneous or alternating treatment and the term "administering" is to be interpreted accordingly.

**In** the context of the present invention, the term 'preventing' is meant to encompass reducing the risk of becoming infected by a parasite and/or reducing the risk of becoming heavily sick from a parasitic infection.

In addition to a human application, the compounds and compositions of the present invention are also of value in the veterinary field, which for the purposes herein not only includes the prevention and/or treatment of diseases in animals, but also - for economically important animals such as chickens / turkeys / ducks / pigs / cattle and equine-species, etc. - enhancing the growth and/or weight of the animal and/or the amount and/or the quality of the meat or other products obtained from the animal. Thus, in a further aspect, the invention relates to a compound or composition for veterinary use that contains at least one compound of the invention and at least one suitable carrier (i.e. a carrier suitable for veterinary use). The invention also relates to the use of a compound of the invention in the preparation of such a composition.

The invention will now be illustrated by means of the following synthetic and biological examples, which do not limit the scope of the invention in any way.

### EXAMPLES

### SYNTHESIS ROUTES AND PHYSICOCHEMICAL PROPERTIES

The table below, provides exemplary compounds of the present application, and their corresponding molecular weight:

| | |
|---|---|
| **FH15961/CL5565** | 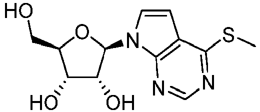 |
| | Chemical Formula: C₁₂H₁₅N₃O₄S |
| | Molecular Weight: 297,3290 |
| **FH15938** | 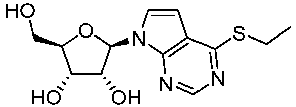 |
| | Chemical Formula: C₁₃H₁₇N₃O₄S |
| | Molecular Weight: 311,36 |
| **FH15935** | 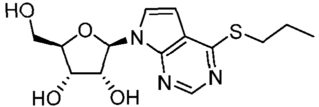 |
| | Chemical Formula: C₁₄H₁₉N₃O₄S |
| | Molecular Weight: 325,383 |
| **FH15958** | 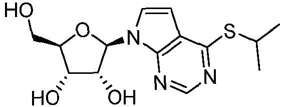 |
| | Chemical Formula: C₁₄H₁₉N₃O₄S |
| | Molecular Weight: 325,383 |
| **FH15936** | 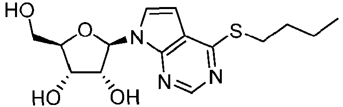 |
| | Chemical Formula: C₁₅H₂₁N₃O₄S |
| | Molecular Weight: 339,410 |
| **FH15955** | 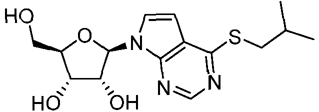 |
| | Chemical Formula: C₁₅H₂₁N₃O₄S |
| | Molecular Weight: 339,410 |
| **FH15956** | 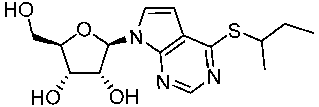 |
| | Chemical Formula: C₁₅H₂₁N₃O₄S |
| | Molecular Weight: 339,410 |
| **FH15937** | 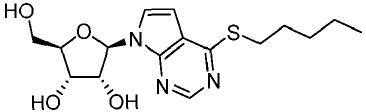 |
| | Chemical Formula: C₁₆H₂₃N₃O₄S |
| | Molecular Weight: 353,437 |
| **FH15954** | 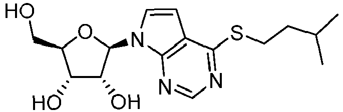 |
| | Chemical Formula: C₁₆H₂₃N₃O₄S |
| | Molecular Weight: 353,437 |
| **FH15952** | 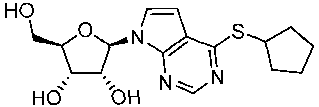 |
| | Chemical Formula: C₁₆H₂₁N₃O₄S |
| | Molecular Weight: 351,421 |
| **FH15957** | 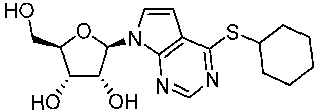 |
| | Chemical Formula: C₁₇H₂₃N₃O₄S |
| | Molecular Weight: 365,448 |
| **FH15963** | 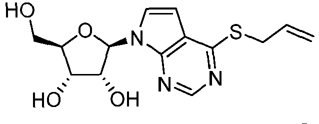 |
| | Chemical Formula: C₁₄H₁₇N₃O₄S |
| | Molecular Weight: 323,3670 |
| **FH15967** | 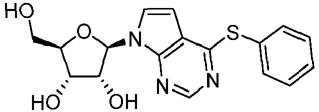 |
| | Chemical Formula: C₁₇H₁₇N₃O₄S |
| | Molecular Weight: 359,4000 |
| **EV1098** | 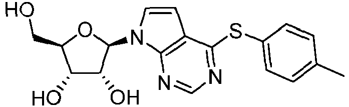 |
| | Chemical Formula: C₁₈H₁₉N₃O₄S |
| | Molecular Weight: 373.4270 |
| **EV1104** | 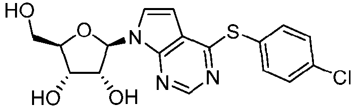 |
| | Chemical Formula: C₁₇H₁₆ClN₃O₄S |
| | Molecular Weight: 393.8420 |
| **EV1108** | 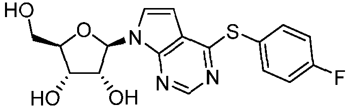 |
| | Chemical Formula: C₁₇H₁₆FN₃O₄S |
| | Molecular Weight: 377.3904 |
| **EV1109** | 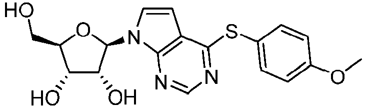 |
| | Chemical Formula: C₁₈H₁₉N₃O₅S |
| | Molecular Weight: 389.4260 |
| **EV1110** | 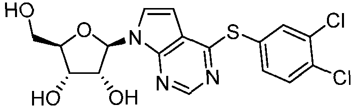 |
| | Chemical Formula: C₁₇H₁₅Cl₂N₃O₄S |
| | Molecular Weight: 428.2840 |
| **EV1111** | 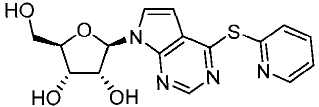 |
| | Chemical Formula: C₁₆H₁₆N₄O₄S |
| | Molecular Weight: 360.3880 |
| **FH14905** | 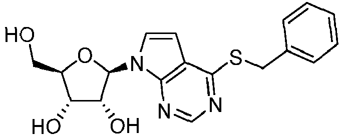 |
| | Chemical Formula: C₁₈H₁₉N₃O₄S |
| | Molecular Weight: 373,43 |
| **FH15959** | 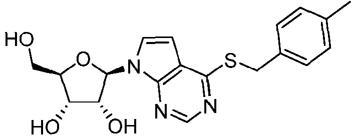 |
| | Chemical Formula: C₁₉H₂₁N₃O₄S |
| | Molecular Weight: 387,454 |
| **FH15941** | 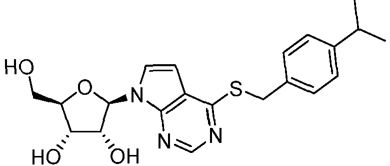 |
| | Chemical Formula: C₂₁H₂₅N₃O₄S |
| | Molecular Weight: 415,51 |
| **FH15960** | 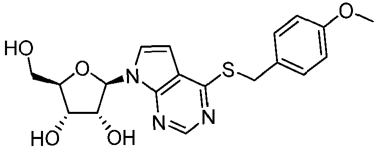 |
| | Chemical Formula: C₁₉H₂₁N₃O₅S |
| | Molecular Weight: 403,453 |
| **FH15934** | 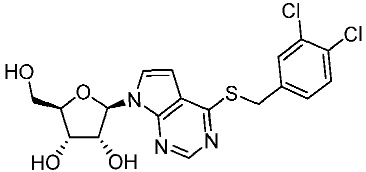 |
| | Chemical Formula: C₁₈H₁₇Cl₂N₃O₄S |
| | Molecular Weight: 442,311 |
| **FH15939** | 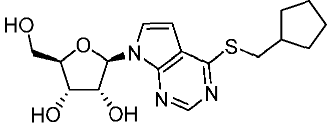 |
| | Chemical Formula: C₁₇H₂₃N₃O₄S |
| | Molecular Weight: 365,448 |
| **FH15962** | 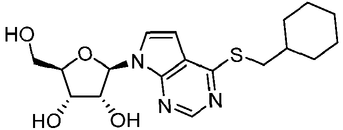 |
| | Chemical Formula: C₁₈H₂₅N₃O₄S |
| | Molecular Weight: 379,4750 |
| **FH15953** | 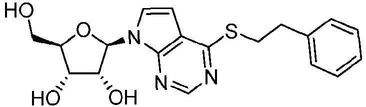 |
| | Chemical Formula: C₁₉H₂₁N₃O₄S |
| | Molecular Weight: 387,454 |
| **FH15940** | 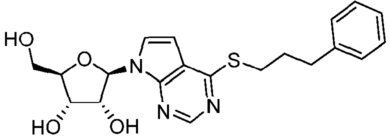 |
| | Chemical Formula: C₂₀H₂₃N₃O₄S |
| | Molecular Weight: 401,481 |
| **JB1031** | 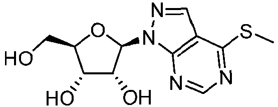 |
| | Chemical Formula: C₁₁H₁₄N₄O₄S |
| | Exact Mass: 298,0736 |
| | Molecular Weight: 298,3170 |
| **JB1032** | 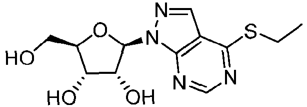 |
| | Chemical Formula: C₁₂H₁₆N₄O₄S |
| | Exact Mass: 312,0892 |
| | Molecular Weight: 312,3440 |
| **EV2092** | 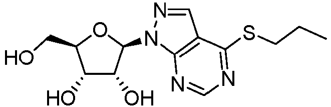 |
| | Chemical Formula: C₁₃H₁₈N₄O₄S |
| | Molecular Weight: 326.3710 |
| **EV2093** | 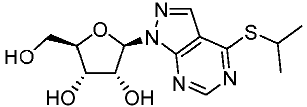 |
| | Chemical Formula: C₁₃H₁₈N₄O₄S |
| | Molecular Weight: 326.3710 |
| **EV2094** | 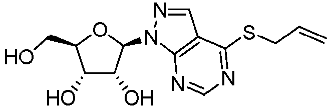 |
| | Chemical Formula: C₁₃H₁₆N₄O₄S |
| | Molecular Weight: 324.3550 |
| **JB1033** | 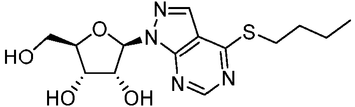 |
| | Chemical Formula: C₁₄H₂₀N₄O₄S |
| | Exact Mass: 340,1205 |
| | Molecular Weight: 340,3980 |
| **JB1034** | 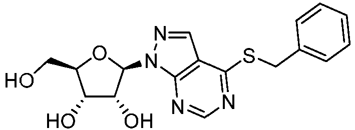 |
| | Chemical Formula: C₁₇H₁₈N₄O₄S |
| | Exact Mass: 374,1049 |
| | Molecular Weight: 374,4150 |

**FH14905** was prepared according to literature. [Structure-Activity Relationships of 7-Deaza-6-benzylthioinosine Analogues as Ligands of Toxoplasma gondii Adenosine Kinase, J. Med. Chem. 2008, 51, 13, 3934-3945]
**FH14903** was prepared according to literature. [Structure-Activity Relationships of 7-Deaza-6-benzylthioinosine Analogues as Ligands of Toxoplasma gondii Adenosine Kinase, J. Med. Chem. 2008, 51, 13, 3934-3945 // Glycosylation of Pyrrolo[2,3- d]pyrimidines with 1- O-Acetyl-2,3,5-tri- O-benzoyl-beta-d-ribofuranose: Substituents and Protecting Groups Effecting the Synthesis of 7-Deazapurine Ribonucleosides, J. Org. Chem. 2018, 83, 15, 8589-8595]

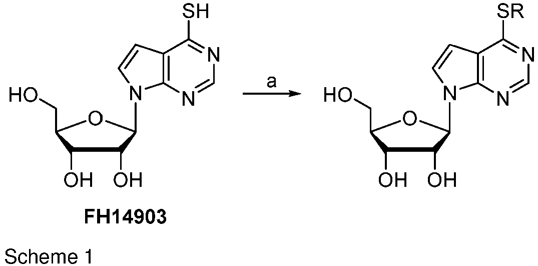

### General procedure for S-alkylation (Scheme 1):

**FH14903** (1 eq.) was suspended in water (12.5 mL/mmol SM) and conc. NH₄OH solution (0.17 mL/mmol SM) was added, resulting in a clear solution. Next, the corresponding alkyl iodide (1.3 eq.), benzyl bromide (1.3 eq.) or benzyl chloride was added and the reaction mixture stirred at the indicated temperature for the indicated time. Then, the mixture was evaporated to dryness and the residue evaporated with Celite^{®}. The residue was purified by FCC eluting with a gradient of 0-10% MeOH/DCM+0.1%NH₄OH to give the desired product.

### 4-S-ethyl-N7-(β-D-ribofuranosyl)pyrrolo[2,3-d]pyrimidine (FH15938)

**FH15938** was prepared according to the general procedure for S-alkylation (T=60°C, 3days). **FH14903** (0.050 g, 0.18 mmol) gave rise to **FH15938** (0.036 g, 0.12 mmol) as a white solid in 65 % yield. Melting point: 138 °C. ¹H NMR (400 MHz, DMSO-d₆) δ: 1.35 (t, *J =* 7.2 Hz, 3H, CH₃), 3.33 (t, *J =* 7.2 Hz, 2H, SCH₂), 3.55 (ddd, *J =* 11.7, 5.6, 4.1 Hz, 1H, H-5'), 3.64 (dt, *J* = 11.8, 4.7 Hz, 1H, H-5"), 3.92 (q, *J =* 3.7 Hz, 1H, H-4'), 4.09 - 4.12 (m, 1H, H-3'), 4.38 - 4.43 (m, 1H, H-2'), 5.07 (t, *J =* 5.3 Hz, 1H, OH-5'), 5.15 (d, *J* = 5.0 Hz, 1H, OH-3'), 5.35 (d, *J =* 6.7 Hz, 1H, OH-2'), 6.15 (d, *J =* 6.2 Hz, 1H, H-1'), 6.57 (d, *J* = 3.6 Hz, 1H, H-5), 7.76 (d, *J =* 3.7 Hz, 1H, H-6), 8.62 (s, 1H, H-2). ¹³C NMR (100 MHz, DMSO-d₆) δ: 15.0 (CH₃), 22.5 (CH₂), 61.5 (C-5'), 70.5 (C-3'), 74.1 (C-2'), 85.2 (C-4'), 86.9 (C-1'), 99.1 (C-5), 116.0 (C-4a), 126.2 (C-6), 148.4 (C7a), 150.4 (C-2), 160.1 (C-4). HRMS calculated for C₁₃H₁₈N₃O₄S [M+H⁺]: 312.1013, found: 312.1024

### 4-S-propyl-N7-(β-D-ribofuranosyl)pyrrolo[2,3-d]pyrimidine (FH15935)

**FH15935** was prepared according to the general procedure for S-alkylation (T=60°C, 3days). **FH14903** (0.050 g, 0.18 mmol) gave rise to **FH15935** (0.017 g, 0.053 mmol) as a white solid in 30 % yield. Melting point: 115 °C. ¹H NMR (400 MHz, DMSO-d₆) δ: 1.00 (t, *J* = 7.5 Hz, 3H, CH₃), 1.72 (sextet, *J* = 7.1 Hz, 2H, CH₂), 3.32 (t, *J =* 7.0 Hz, 2H, SCH₂), 3.54 (ddd, *J =* 12.1, 5.7, 4.2 Hz, 1H, H-5'), 3.63 (dt, *J =* 12.0, 4.7 Hz, 1H, H-5"), 3.92 (q, *J* = 3.5 Hz, 1H, H-4'), 4.09 - 4.12 (m, 1H, H-3'), 4.38 - 4.43 (m, 1H, H-2'), 5.07 (t, *J =* 5.5 Hz, 1H, OH-5'), 5.16 (d, *J =* 4.8 Hz, 1H, OH-3'), 5.35 (d, *J =* 6.3 Hz, 1H, OH-2'), 6.15 (d, *J* = 6.1 Hz, 1H, H-1'), 6.58 (d, *J =* 3.7 Hz, 1H, H-5), 7.76 (d, *J =* 3.9 Hz, 1H, H-6), 8.67 (s, 1H, H-2). ¹³C NMR (100 MHz, DMSO-d₆) δ: 13.2 (CH₃), 22.6 (CH₂), 29.9 (SCH₂), 61.5 (C-5'), 70.5 (C-3'), 74.1 (C-2'), 85.2 (C-4'), 86.9 (C-1'), 99.2 (C-5), 116.0 (C-4a), 126.2 (C-6), 148.3 (C7a), 150.4 (C-2), 160.2 (C-4). HRMS calculated for C₁₄H₂₀N₃O₄S [M+H⁺]: 326.1169, found: 326.1176.

### 4-S-2-methylpropyl-N7-(β-D-ribofuranosyl)pyrrolo[2,3-d]pyrimidine (FH15955)

**FH15955** was prepared according to the general procedure for S-alkylation. **FH14903** (0.015 g, 0.043 mmol) gave rise to **FH15955** (0.049 g, 0.16 mmol) as a white solid in 25 % yield. Melting point: 120 °C.

¹H NMR (400 MHz, DMSO-d₆) δ: 1.01 (d, *J* = 6.7 Hz, 6H, CH₃), 1.96 (sept., *J* = 6.7 Hz, 1H, CH), 3.28 (dd, *J* = 6.7, 1.5 Hz, 2H, SCH₂), 3.54 (ddd, *J =* 11.4, 5.6, 4.2 Hz, 1H, H-5'), 3.63 (dt, *J* = 11.9, 4.7 Hz, 1H, H-5"), 3.92 (q, *J =* 3.2 Hz, 1H, H-4'), 4.09-4.12 (m, 1H, H-3'), 4.38-4.43 (m, 1H, H-2'), 5.07 (t, *J* = 5.4 Hz, 1H, OH-5'), 5.16 (d, *J =* 4.6 Hz, 1H, OH-3'), 5.37 (d, *J =* 6.3 Hz, 1H, OH-2'), 6.15 (d, *J* = 6.2 Hz, 1H, H-1'), 6.60 (d, *J =* 3.6 Hz, 1H, H-5), 7.76 (d, *J =* 3.8 Hz, 1H, H-6), 8.61 (s, 1H, H-2). ¹³C NMR (100 MHz, DMSO-d₆) δ: 21.60 (CH₃), 21.61 (CH₃), 28.2 (CH), 36.1 (SCH₂), 61.5 (C-5'), 70.5 (C-3'), 74.1 (C-2'), 85.2 (C-4'), 86.9 (C-1'), 99.2 (C-5), 116.0 (C-4a), 126.2 (C-6), 148.3 (C7a), 150.3 (C-2), 160.2 (C-4). HRMS calculated for C₁₅H₂₂N₃O₄S [M+H⁺]: 340.1326, found: 340.1327.

### 4-S-sec-butyl-N7-(β-D-ribofuranosyl)pyrrolo[2,3-d]pyrimidine (FH15956)

**FH15956** was prepared according to the general procedure for S-alkylation. **FH14903** (0.015 g, 0.043 mmol) gave rise to **FH15956** (0.038 g, 0.11 mmol) as a white solid in 63 % yield. Melting point: 108 °C.
¹H NMR (400 MHz, DMSO-d₆) δ: 1.00 (td, *J* = 7.4, 1.2 Hz, 3H, CH₃), 1.41 (d, *J =* 6.8 Hz, 3H, CH₃), 1.66-1.82 (m, 2H, CH₂), 3.54 (ddd, *J* = 11.6, 5.6, 4.0 Hz, 1H, H-5'), 3.63 (dt, *J =* 12.0, 4.7 Hz, 1H, H-5"), 3.92 (q, *J* = 3.9 Hz, 1H, H-4'), 4.09-4.12 (m, 1H, H-3'), 4.14-4.23 (m, 1H, SCH), 4.38-4.43 (m, 1H, H-2'), 5.07 (t, J = 5.5 Hz, 1H, OH-5'), 5.16 (d, *J =* 4.9 Hz, 1H, OH-3'), 5.35 (d, *J =* 6.5 Hz, 1H, OH-2'), 6.14 (d, *J* = 6.2 Hz, 1H, H-1'), 6.55 (d, *J =* 3.5 Hz, 1H, H-5), 7.76 (d, *J =* 3.8 Hz, 1H, H-6), 8.61 (s, 1H, H-2). ¹³C NMR (100 MHz, DMSO-d₆) δ: 11.2 (CH₃), 11.3 (CH₃), 20.7 (CH₃), 20.8 (CH₃), 28.98 (CH₂), 29.02 (CH₂), 40.0 (SCH), 40.2 (SCH), 61.5 (C-5'), 70.5 (C-3'), 74.1 (C-2'), 85.2 (C-4'), 86.9 (C-1'), 99.2 (C-5), 116.1 (C-4a), 126.2 (C-6), 148.4 (C7a), 150.4 (C-2), 160.4 (C-4). HRMS calculated for C₁₅H₂₂N₃O₄S [M+H⁺]: 340.1326, found: 340.1339

### 4-S-pentyl-N7-(β-D-ribofuranosyl)pyrrolo[2,3-d]pyridimidine (FH15937)

**FH15937** was prepared according to the general procedure for S-alkylation (T=60°C, 4days). **FH14903** (0.050 g, 0.18 mmol) gave rise to **FH15937** (0.027 g, 0.078 mmol) as a white solid in 44 % yield. Melting point: 86 °C. ¹H NMR (400 MHz, DMSO-d₆) δ: 0.87 (t, *J =* 7.2 Hz, 3H, CH₃), 1.30 - 1.44 (m, 4H, 2xCH₂), 1.67 - 1.74 (m, 2H, CH₂), 3.33 (t, *J* = 7.5 Hz, 2H, SCH₂), 3.54 (ddd, *J* = 11.9, 5.7, 3.9 Hz, 1H, H-5'), 3.63 (dt, *J* = 11.8, 4.7 Hz, 1H, H-5"), 3.92 (q, *J* = 3.8 Hz, 1H, H-4'), 4.09 - 4.12 (m, 1H, H-3'), 4.38 - 4.43 (m, 1H, H-2'), 5.06 (t, *J =* 5.4 Hz, 1H, OH-5'), 5.16 (d, *J =* 5.3 Hz, 1H, OH-3'), 5.34 (d, *J =* 6.2 Hz, 1H, OH-2'), 6.15 (d, *J* = 6.1 Hz, 1H, H-1'), 6.57 (d, *J =* 3.6 Hz, 1H, H-5), 7.76 (d, *J =* 3.9 Hz, 1H, H-6), 8.61 (s, 1H, H-2). ¹³C NMR (100 MHz, DMSO-d₆) δ: 13.8 (CH₃), 21.7 (CH₂), 27.9 (CH₂), 28.9 (CH₂), 30.4 (SCH₂), 61.5 (C-5'), 70.5 (C-3'), 74.1 (C-2'), 85.2 (C-4'), 86.9 (C-1'), 99.2 (C-5), 116.0 (C-4a), 126.2 (C-6), 148.3 (C7a), 150.4 (C-2), 160.2 (C-4). HRMS calculated for C₁₆H₂₄N₃O₄S [M+H⁺]: 354.1482, found: 354.1509

### 4-S-isopentyl-N7-(β-D-ribofuranosyl)pyrrolo[2,3-d]pyrimidine (FH15954)

**FH15954** was prepared according to the general procedure for S-alkylation. **FH14903** (0.050 g, 0.18 mmol) gave rise to **FH15954** (0.049 g, 0.16 mmol) as a white solid in 78 % yield. Melting point: 80 °C. ¹H NMR (400 MHz, DMSO-d₆) δ: 0.92 (s, 3H, CH₃), 0.93 (s, 3H, CH₃), 1.59 (q, *J* = 7.4 Hz, 2H, CH₂), 1.72 (sept., *J* = 6.6 Hz, 1H, CH), 3.35 (dd, *J* = 7.8, 6.3 Hz, 2H, SCH₂), 3.54 (ddd, *J =* 12.0, 5.5, 4.0 Hz, 1H, H-5'), 3.63 (dt, *J* = 12.0, 4.5 Hz, 1H, H-5"), 3.92 (q, *J* = 3.9 Hz, 1H, H-4'), 4.09-4.12 (m, 1H, H-3'), 4.38-4.42 (m, 1H, H-2'), 5.06 (t, *J =* 5.6 Hz, 1H, OH-5'), 5.15 (d, *J =* 4.8 Hz, 1H, OH-3'), 5.34 (d, *J* = 6.4 Hz, 1H, OH-2'), 6.15 (d, *J =* 6.2 Hz, 1H, H-1'), 6.57 (d, *J =* 3.7Hz, 1H, H-5), 7.76 (d, *J* = 3.8 Hz, 1H, H-6), 8.62 (s, 1H, H-2). ¹³C NMR (100 MHz, DMSO-d₆) δ: 22.1 (2xCH₃), 26.1 (CH), 27.0 (CH₂), 38.1 (SCH₂), 61.5 (C-5'), 70.5 (C-3'), 74.1 (C-2'), 85.2 (C-4'), 86.9 (C-1'), 99.1 (C-5), 116.0 (C-4a), 126.2 (C-6), 148.3 (C7a), 150.4 (C-2), 160.1 (C-4). HRMS calculated for C₁₆H₂₄N₃O₄S [M+H⁺]: 354.1482, found: 354.1468

### 4-S-cyclopentyl-N7-(β-D-ribofuranosyl)pyrrolo[2,3-d]pyrimidine (FH15952)

**FH15952** was prepared according to the general procedure for S-alkylation. **FH14903** (0.050 g, 0.18 mmol) gave rise to **FH15952** (0.024 g, 0.069 mmol) as a white solid in 38 % yield. Melting point: 130 °C.
¹H NMR (400 MHz, DMSO-d₆) δ: 1.57-1.78 (m, 6H, 3xCH₂), 2.19-2.25 (m, 2H, CH₂), 3.54 (ddd, *J* = 11.8, 5.2, 4.5 Hz, 1H, H-5'), 3.64 (dt, *J* = 11.9, 4.2 Hz, 1H, H-5"), 3.91 (q, *J =* 3.7 Hz, 1H, H-4'), 4.09 - 4.12 (m, 1H, H-3'), 4.27-4.34 (m, 1H, SCH), 4.38 - 4.43 (m, 1H, H-2'), 5.07 (t, *J =* 5.3 Hz, 1H, OH-5'), 5.15 (d, *J* = 4.9 Hz, 1H, OH-3'), 5.34 (d, *J* = 6.5 Hz, 1H, OH-2'), 6.15 (d, *J =* 6.1 Hz, 1H, H-1'), 6.55 (d, *J* = 3.6 Hz, 1H, H-5), 7.76 (d, *J =* 3.8 Hz, 1H, H-6), 8.61 (s, 1H, H-2). ¹³C NMR (100 MHz, DMSO-d₆) δ: 24.3 (2xCH₃), 33.1 (CH₂), 33.2 (CH₂), 41.6 (SCH), 61.5 (C-5'), 70.5 (C-3'), 74.1 (C-2'), 85.2 (C-4'), 86.9 (C-1'), 99.2 (C-5), 115.8 (C-4a), 126.2 (C-6), 148.4 (C7a), 150.5 (C-2), 160.9 (C-4). HRMS calculated for C₁₆H₂₂N₃O₄S [M+H⁺]: 352.1326, found: 352.1367

### 4-S-cyclohexyl-N7-(β-D-ribofuranosyl)pyrrolo[2,3-d]pyrimidine (FH15957)

**FH15957** was prepared according to the general procedure for S-alkylation. **FH14903** (0.050 g, 0.18 mmol) gave rise to **FH15957** (0.010 g, 0.027 mmol) as a white solid in 15 % yield. Melting point: 167 °C.
¹H NMR (400 MHz, DMSO-d₆) δ: 1.27-1.36 (m, 1H, CH₂), 1.41-1.54 (m, 4H, 2xCH₂₎, 1.57-1.62 (m, 1H, CH₂), 1.72-1.75 (m, 2H, CH₂), 2.07-2.09 (m, 2H, CH₂), 3.54 (ddd, *J* = 11.8, 5.7, 4.2 Hz, 1H, H-5'), 3.63 (dt, *J* = 12.0, 4.7 Hz, 1H, H-5"), 3.91 (q, *J =* 3.9 Hz, 1H, H-4'), 4.09-4.12 (m, 1H, H-3'), 4.13-4.18 (m, 1H, SCH), 4.38 - 4.43 (m, 1H, H-2'), 5.06 (t, *J =* 5.5 Hz, 1H, OH-5'), 5.15 (d, *J* = 4.5 Hz, 1H, OH-3'), 5.34 (d, *J* = 6.4 Hz, 1H, OH-2'), 6.14 (d, *J* = 5.9 Hz, 1H, H-1'), 6.54 (d, *J* = 3.6 Hz, 1H, H-5), 7.75 (d, *J* = 3.7 Hz, 1H, H-6), 8.61 (s, 1H, H-2). ¹³C NMR (100 MHz, DMSO-d₆) δ: 25.2 (CH₂), 25.5 (3xCH₂), 32.7 (CH₂), 32.8 (CH₂), 41.2 (SCH), 61.5 (C-5'), 70.5 (C-3'), 74.1 (C-2'), 85.2 (C-4'), 86.9 (C-1'), 99.2 (C-5), 115.9 (C-4a), 126.2 (C-6), 148.4 (C7a), 150.5 (C-2), 160.0 (C-4). HRMS calculated for C₁₇H₂₄N₃O₄S [M+H⁺]: 366.1482, found: 366.1478

### 4-S-allyl-N7-(β-D-ribofuranosyl)pyrrolo[2,3-d]pyrimidine (FH15963)

**FH15963** was prepared according to the general procedure for S-alkylation. **FH14903** (0.050 g, 0.18 mmol) gave rise to **FH15963** (0.024 g, 0.073 mmol) as a white solid in 41 % yield. Melting point: 137 °C.

¹H NMR (400 MHz, DMSO-d₆) δ: 3.55 (ddd, *J* = 11.7, 5.6, 4.1 Hz, 1H, H-5'), 3.64 (dt, *J* = 11.8, 4.5 Hz, 1H, H-5"), 3.92 (q, *J =* 3.5 Hz, 1H, H-4'), 4.04 (d, *J =* 6.5 Hz, 2H, SCH₂), 4.09-4.12 (m, 1H, H-3'), 4.39-4.43 (m, 1H, H-2'), 5.06 (t, *J* = 5.7 Hz, 1H, OH-5'), 5.13 (dd, *J =* 10.2, 1.5 Hz, 1H, Hallyl), 5.16 (d, *J* = 4.8 Hz, 1H, OH-3'), 5.35 (d, *J =* 6.3 Hz, 1H, OH-2'), 5.35 (dq, *J* = 17.0, 1.5 Hz, 1H, Hallyl), 5.94-6.04 (m, 1H, Hallyl), 6.16 (d, *J =* 6.3 Hz, 1H, H-1'), 6.60 (d, *J =* 4.2 Hz, 1H, H-5), 7.78 (d, *J* = 3.8 Hz, 1H, H-6), 8.63 (s, 1H, H-2). ¹³C NMR (100 MHz, DMSO-d₆) δ: 30.6 (SCH₂), 61.5 (C-5'), 70.5 (C-3'), 74.1 (C-2'), 85.2 (C-4'), 86.9 (C-1'), 99.1 (C-5), 115.9 (C-4a), 118.1 (allyl-CH₂) 126.3 (C-6), 133.8 (allyl-CH), 148.5 (C-7a) 150.4 (C-2), 159.3 (C-4). HRMS calculated for C₁₄H₁₈N₃O₅S [M+H⁺]: 324.1013, found: 324.0988.

### 4-S-phenyl-N7-(β-D-ribofuranosyl)pyrrolo[2,3-d]pyrimidine (FH15967)

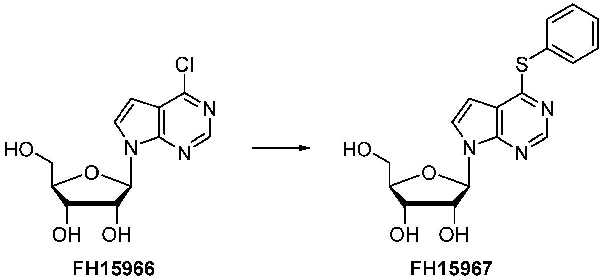

**FH15966** (0.070 g, 0.25 mmol, 1 eq.), K₃PO₄ (0.13 g, 0.32 mmol, 2.4 eq.), Pd(OAc)2 (0.0010 g, 0.0025 mmol, 0.010 eq.), PTABS (0.0030 g, 0.0098 mmol, 0.02 eq.), were added to a round bottom flask. Next, DMF (0.80 mL, 3.0 mL/mmol SM) and thiophenol (0.033 mL, 0.32 mmol, 1.0 eq.) were added and the resulting mixture heated to 50 °C. After full consumption of the starting material (~4h), the mixture was cooled to ambient temperature and evaporated. The residue was purified by column chromatography (0 → 10 % MeOH/DCM) to give **FH15967** (0.055 g, 0.153 mmol) as a slight yellow solid in 62 % yield. Melting point: 161°C. ¹H NMR (400 MHz, DMSO-d₆) δ: 3.53 (ddd, *J* = 11.9, 5.5, 4.4 Hz, 1H, H-5'), 3.62 (dt, *J* = 11.9, 4.8 Hz, 1H, H-5"), 3.91 (q, *J* = 3.6 Hz, 1H, H-4'), 4.08-4.11 (m, 1H, H-3'), 4.36-4.40 (m, 1H, H-2'), 5.04 (t, *J =* 5.7 Hz, 1H, OH-5'), 5.15 (d, *J* = 4.9 Hz, 1H, OH-"), 5.35 (d, *J* = 6.5 Hz, 1H, OH-2'), 6.10 (d, *J =* 4.0 Hz, 1H, H-5), 6.15 (d, *J* = 6.4 Hz, 1H, H-1'), 7.48-7.55 (m, 3H, HPh), 7.65-7.68 (m, 2H, HPh), 7.76 (d, *J =* 3.9 Hz, 1H, H-6), 8.52 (s, 1H, H-2). ¹³C NMR (100 MHz, DMSO-d₆) δ: 61.5 (C-5'), 70.5 (C-3'), 74.1 (C-2'), 85.2 (C-4'), 86.8 (C-1'), 99.2 (C-5), 115.7 (C-4a), 126.7 (C-6), 127.7 (CPh), 129.5 (2xCPh), 129.7 (CPh), 135.3 (2xCPh), 149.4 (C-7a) 150.3 (C-2), 160.2 (C-4). HRMS calculated for C₁₇H₁₈N₃O₅S [M+H⁺]: 360.1013, found: 360.1010.

### 4-S-(4-methylphenyl)-N7-(β-D-ribofuranosyl)pyrrolo[2,3-d]pyrimidine (EV1098)

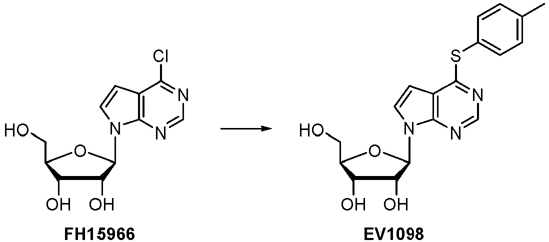

This procedure is based on literature [HFIP Promoted Low-Temperature SNAr of Chloroheteroarenes Using Thiols and Amines, Bhujabal et al, J. Org. Chem. 2019, 84, 23, 15343-15354]
In an oven dried pressure tube, **FH15966** (20 mg, 0.07 mmol) was dissolved in HFIP (1 ml) after which 4-methylbenzenethiol (10 mg, 0.084 mmol) was added. The mixture was stirred for 16 to 48 hours to completion. Volatiles were evaporated and the residue was purified by flash column chromatography (0 → 10 % MeOH in DCM). **EV1098** was obtained as a white solid (6 mg, 23%)
¹H NMR (400 MHz, DMSO-d6) δ 2.39 (s, 3H, CH3), 3.48-3.58 (m, 1H, H-5'), 3.58-3.67 (m, 1H, H-5"), 3.91 (quart, J=3.5 Hz, 1H, H-4'), 4.09 (quart, J=3.7 Hz, 1H, H-3'), 4.37 (quart, J=5.6 Hz, 1H, H-2'), 5.04 (t, J=5.5 Hz, 1H, OH (C-5')), 5.17 (d, J=4.6 Hz, 1H, OH (C-3')), 5.34 (d, J=6.2 Hz, 1H, OH (C-2')), 6.06 (d, J=3.8 Hz, 1H, H-1'), 6.15 (d, J=6.0 Hz, 1H, H-2), 7.32 (d, J=8.2 Hz, 2H, Ph-H (meta to S) ), 7.54 (d, J=8.2 Hz, 2H, Ph-H (ortho to S)), 7.74 (d, J=3.8 Hz, 1H, H-3), 8.50 (s, 1H, H-6)
¹³C NMR (101 MHz, DMSO-d6) δ 21.7 (CH3), 62.0 (C-5'), 71.0 (C-3'), 74.6 (C-2'), 85.7 (C-4'), 87.3 (C-1'), 99.7 (C-3), 116.1 (C-q), 124.6 (C-q), 127.1 (C-2), 130.7 (C_{Ph}), 136.0 (C_{Ph}), 140.1 (C-q), 149.8 (C-q), 150.8 (C-6), 161.2 (C-q)

### 4-S-(4-chlorophenyl)-N7-(β-D-ribofuranosyl)pyrrolo[2,3-d]pyrimidine (EV1104)

**EV1104** was obtained as a white solid (14 mg, 53%) by means of the same procedure as **EV1098. FH15966** (20 mg) and 4-chlorobenzenethiol (12 mg) was used.
¹H NMR (400 MHz, DMSO-d6) δ 3.50-3.59 (m, 1H, H-5'), 3.59-3.69 (m, 1H, H-5"), 3.92 (quart, J=3.6 Hz, 1H, H-4'), 4.07-4.14 (m, 1H, H-3'), 4.40 (quart, J=5.3Hz, 1H, H-2'), 5.05 (t, J=5.4 Hz, 1H, OH (C-5')), 5.19 (d, J=4.5 Hz, 1H, OH (C-2'), 5.38 (d, J=6.1 Hz, 1H, OH (C-2')), 6.17 (d, J=6.0 Hz, 1H, H-1'), 6.30 (d, J=4.0 Hz, 1H, H-3), 7.56 (t, J=8.5 Hz, 2H, Ph-H), 7.67 (t, J=8.5 Hz, 2H, Ph-H), 7.81 (d, J=3.5 Hz, 1H, H-2), 8.53 (s, 1H, H-6)
¹³C NMR (101 MHz, DMSO-d6) δ 62.0 (C-5'), 71.0 (C-3'), 74.6 (C-2'), 85.7 (C-4'), 87.4 (C-1'), 99.6 (C-3), 116.2 (C-q), 127.2 (C-q), 127.5 (C-2), 130.0 (C_{Ph}), 135.1 (C-q), 137.4 (C_{Ph}), 149.8 (C-q), 150.9 (C-6), 159.8 (C-q)

### 4-S-(4-fluorophenyl)-N7-(β-D-ribofuranosyl)pyrrolo[2,3-d]pyrimidine (EV1108)

**EV1108** was obtained as a white solid (36 mg, 87%) by means of the same procedure as **EV1098. FH15966** (31 mg) and 4-fluorobenzenethiol (17 mg) was used.
¹H NMR (400 MHz, DMSO-d6) δ 3.50-3.58 (m, 1H, H-5'), 3.59-3.67 (m, 1H, H-5"), 3.91 (quart, J=3.6 Hz, 1H, H-4'), 4.10 (quart, J=3.6 Hz, 1H, H-3'), 4.39 (quart, J=5.6 Hz, 1H, H-2'), 5.05 (t, J=5.3 Hz, 1H, OH (C-5')), 5.16 (d, J=4.7 Hz, 1H, OH (C-3')), 5.35 (d, J=6.2 Hz, 1H, OH (C-2')), 6.16 (d, J=6.2 Hz, 1H, H-1'), 6.23 (d, J=3.7 Hz, 1H, H-3), 7.36 (tt, J=8.9;2.0 Hz, 2H, Ph-H), 7.68-7.74 (m, 2H, Ph-H), 7.79 (d, J=3.7 Hz, 1H, H-2), 8.51 (s, 1H, H-6)
¹³C NMR (101 MHz, DMSO-d6) δ 62.0 (C-5'), 71.0 (C-3'), 74.6 (C-2'), 85.7 (C-4'), 87.4 (C-1'), 99.6 (C-3), 116.0 (C-q), 117.0 (C_{Ph}), 117.2 (C_{Ph}), 123.7 (C-q), 127.3 (C-2), 138.4 (C_{Ph}), 138.5 (C_{Ph}), 149.8 (C-q), 150.9 (C-6), 159.8 (C-q), 160.5 (C-q), 162.3 (C-q), 164.7 (C-q)

### 4-S-(4-methoxyphenyl)-N7-(β-D-ribofuranosyl)pyrrolo[2,3-d]pyrimidine (EV1109)

**EV1109** was obtained as a white solid (14 mg, 33%) by means of the same procedure as **EV1098. FH15966** (32 mg) and 4-chlorobenzenethiol (19 mg) was used.
1H NMR (400 MHz, DMSO-d6) δ 3.49-3.57 (m, 1H, H-5'), 3.57-3.67 (m, 1H, H-5"), 3.84 (s, 3H, O-CH3), 3.91 (quart, J=3.6 Hz, 1H, H-4'), 4.09 (dd, J=10.3;4.7 Hz, 1H, H-3'), 4.37 (quart, J=5.6 Hz, 1H, H-2'), 5.04 (t, J=5.4 Hz, 1H, OH (C-5')), 5.16 (d, J=5.1 Hz, 1H, OH (C-3')), 5.35 (d, J=6.4 Hz, 1H, OH (C-2')), 6.00 (d, J=3.7 Hz, 1H, H-3), 6.15 (d, J=6.2 Hz, 1H, H-1'), 7.07 (dt, J=8.7;2.0 Hz, 2H, Ph-H), 7.58 (dt, J=8.7;1.9 Hz, 2H, Ph-H), 7.73 (d, J=3.9 Hz, 1H, H-2), 8.49 (s, 1H, H-6)
¹³C NMR (101 MHz, DMSO-d6): δ 55.9 (CH3), 62.0 (C-5'), 71.0 (C-3'), 74.6 (C-2'), 85.6 (C-4'), 87.3 (C-1'), 99.7 (C-3), 115.6 (C_{Ph}), 115.8 (C-q), 118.3 (C-q), 127.0 (C-2), 137.9 (C_{Ph}), 149.8 (C_{Ph}), 150.8 (C-6), 161.1 (C-q), 161.9 (C-q)

### 4-S-(3,4-dichlorophenyl)-N7-(β-D-ribofuranosyl)pyrrolo[2,3-d]pyrimidine (EV1110)

**EV1110** was obtained as a white solid (31 mg, 66%) by means of the same procedure as **EV1098. FH15966** (30 mg) and 3,4-dichlorobenzenethiol (23 mg) was used.
1H NMR (400 MHz, DMSO-d6) δ 3.50-3.59 (m, 1H, H-5'), 3.59-3.68 (m, 1H, H-5"), 3.92 (quart, J=3.6 Hz, 1H, H-4'), 4.11 (dd, J=10.3;4.8 Hz, 1H, H-3'), 4.40 (quart, J=5.7 Hz, 1H, H-2'), 5.05 (t, J=5.4 Hz, 1H, OH (C-5')), 5.18 (d, J=4.4 Hz, 1H, OH (C-3')), 5.37 (d, J=6.4 Hz, 1H, OH (C-2')), 6.17 (d, J=6.1 Hz, 1H, H-1'), 6.44 (d, J=3.9 Hz, 1H, H-3), 7.64 (dd, J=8.3;2.0 Hz, 1H, Ph-H), 7.77 (d, J=8.3 Hz, 1H, Ph-H), 7.85 (d, J=3.8 Hz, 1H, H-2), 7.97 (d, J=2.1 Hz, 1H, Ph-H), 8.55 (s, 1H, H-6)
¹³C NMR (101 MHz, DMSO-d6): δ 55.9 (CH3), 62.0 (C-5'), 71.0 (C-3'), 74.6 (C-2'), 85.6 (C-4'), 87.3 (C-1'), 99.6 (C-3), 116.3 (C-q), 127.7 (C-2), 129.1 (C-q), 131.8 (C_{Ph}), 132.2 (C-q), 133.1 (C-q), 135.7 (C_{Ph}), 136.8 (C_{Ph}), 149.8 (C_{Ph}), 151.0 (C-6), 158.9 (C-q)

### 4-S-(2-pyridinyl)-N7-(β-D-ribofuranosyl)pyrrolo[2,3-d]pyrimidine (EV1111)

**EV1111** was obtained as a yellow solid (25 mg, 63%) by means of the same procedure as **EV1098. FH15966** (30 mg) and 2-pyridinethiol (14 mg) was used.
¹H NMR (400 MHz, DMSO-d6) δ 3.49-3.59 (m, 1H, H-5'), 3.59-3.68 (m, 1H, H-5"), 3.93 (quart, J=3.6 Hz, 1H, H-4'), 4.11 (dd, J=10.5;4.6 Hz, 1H, H-3'), 4.41 (quart, J=5.8 Hz, 1H, H-2'), 5.05 (t, J=5.4 Hz, 1H, OH (C-5')), 5.19 (d, J=4.6 Hz, 1H, OH (C-3')), 5.39 (d, J=6.6 Hz, 1H, OH (C-2')), 6.19 (d, J=6.3 Hz, 1H, H-1'), 6.25 (d, J=3.6 Hz, 1H, H-3), 7.42 (dd, J=7.5;0.9 Hz, 1H, Ar-H), 7.77 (d, J=8.1 Hz, 1H, Ph-H), 7.81-7.90 (m, 2H, Ar-H + H-2), 8.55-8.59 (m, 1H, Ar-H), 8.62 (s, 1H, H-6)
¹³C NMR (101 MHz, DMSO-d6): δ 55.9 (CH3), 62.0 (C-5'), 71.0 (C-3'), 74.6 (C-2'), 85.7 (C-4'), 87.3 (C-1'), 100.0 (C-3), 123.8 (C_{pyr}), 127.8 (C_{pyr}), 129.1 (C_{pyr}), 138.2 (C_{pyr})< 150.2 (C-q), 150.8 (C_{pyr}), 151.0 (C-6), 153.3 (C-q)

### 4-S-(4-methylbenzyl)-N7-(β-D-ribofuranosyl)pyrrolo[2,3-d]pyrimidine (FH15959)

**FH15959** was prepared according to the general procedure for S-alkylation. **FH14903** (0.050 g, 0.18 mmol) gave rise to **FH15959** (0.046 g, 0.12 mmol) as a white solid in 67 % yield.

¹H NMR (400 MHz, DMSO-d₆) δ: 2.26 (s, 3H, CH₃), 3.54 (ddd, *J* = 11.8, 5.7, 4.1 Hz, 1H, H-5'), 3.63 (dt, *J* = 12.1, 4.9 Hz, 1H, H-5"), 3.92 (q, *J =* 3.5 Hz, 1H, H-4'), 4.09-4.12 (m, 1H, H-3'), 4.38-4.42 (m, 1H, H-2'), 4.60 (s, 2H, SCH₂), 5.06 (t, *J* = 5.6 Hz, 1H, OH-5'), 5.16 (d, *J* = 5.1 Hz, 1H, OH-3'), 5.34 (d, *J =* 6.2 Hz, 1H, OH-2'), 6.16 (d, *J =* 6.2 Hz, 1H, H-1'), 6.57 (d, *J =* 3.8 Hz, 1H, H-5), 7.11 (d, *J* = 7.7 Hz, 2H, HPh), 7.33 (d, *J =* 7.9 Hz, 2H, HPh), 7.77 (d, *J =* 3.8 Hz, 1H, H-6), 8.67 (s, 1H, H-2). ¹³C NMR (100 MHz, DMSO-d₆) δ: 20.7 (CH₃), 31.6 (SCH₂), 61.5 (C-5'), 70.5 (C-3'), 74.1 (C-2'), 85.2 (C-4'), 87.0 (C-1'), 99.2 (C-5), 115.7 (C-4a), 126.3 (C-6), 128.9 (2xCPh), 129.0 (2xCPh), 134.7 (CPh), 136.3 (CPh), 148.5 (C7a), 150.4 (C-2), 159.6 (C-4). HRMS calculated for C₁₉H₂₂N₃O₄S [M+H⁺]: 388.1326, found: 388.1343.

### 4-S-(4-isopropylbenzyl)-N7-(β-D-ribofuranosyl)pyrrolo[2,3-d]pyrimidine (FH15941)

**FH15941** was prepared according to the general procedure for S-alkylation (T=60°C, 5 days). **FH14903** (0.050 g, 0.18 mmol) gave rise to **FH15941** (0.071 g, 0.17 mmol) as a white solid in 97 % yield. Melting point: 128 °C.
¹H NMR (400 MHz, DMSO-d₆) δ: 1.16 (s, 3H, CH₃), 1.17 (s, 3H, CH₃), 2.84 (sept., *J* = 6.8 Hz, 1H, CH), 3.55 (dt, *J* = 11.6, 4.5 Hz, 1H, H-5'), 3.64 (dt, *J =* 12.0, 4.6 Hz, 1H, H-5"), 3.92 (q, *J* = 3.5 Hz, 1H, H-4'), 4.09 - 4.12 (m, 1H, H-3'), 4.38 - 4.43 (m, 1H, H-2'), 4.60 (t, *J* = 14.1 Hz, 2H, SCH2), 5.06 (t, *J =* 5.6 Hz, 1H, OH-5'), 5.16 (d, *J =* 4.7 Hz, 1H, OH-3'), 5.34 (d, *J =* 6.4 Hz, 1H, OH-2'), 6.16 (d, *J* = 6.1 Hz, 1H, H-1'), 6.57 (d, *J =* 3.7 Hz, 1H, H-5), 7.16-7.18 (m, 2H, H-Ph), 7.35-7.37 (m, 2H, H-Ph), 7.77 (d, *J* = 3.8 Hz, 1H, H-6), 8.68 (s, 1H, H-2). ¹³C NMR (100 MHz, DMSO-d₆) δ: 23.8 (2xCH₃), 31.6 (CH), 33.1 (SCH₂), 61.5 (C-5'), 70.5 (C-3'), 74.1 (C-2'), 85.2 (C-4'), 87.0 (C-1'), 99.1 (C-5), 115.7 (C-4a), 126.3 (C-Ph), 126.4 (2xC-Ph), 126.2 (C-6), 128.9 (2xC-Ph), 135.1 (C-Ph), 147.4 (C-Ph), 148.5 (C7a), 150.4 (C-2), 159.6 (C-4). HRMS calculated for C₂₁H₂₆N₃O₄S [M+H⁺]: 416.1639, found: 416.1630

### 4-S-(4-methylbenzyl)-N7-(β-D-ribofuranosyl)pyrrolo[2,3-d]pyrimidine (FH15960)

**FH15960** was prepared according to the general procedure for S-alkylation. **FH14903** (0.050 g, 0.18 mmol) gave rise to **FH15960** (0.027 g, 0.067 mmol) as a white solid in 38 % yield.

¹H NMR (400 MHz, DMSO-d₆) δ: 3.53-3.56 (m, 1H, H-5'), 3.64 (dd, *J =* 11.8, 3.6 Hz, 1H, H-5"), 3.71 (s, 3H, OCH₃), 3.92 (q, *J =* 3.8 Hz, 1H, H-4'), 4.10-4.11 (m, 1H, H-3'), 4.38-4.42 (m, 1H, H-2'), 4.59 (s, 2H, SCH₂), 5.06 (br. s, 1H, OH-5'), 5.15 (br. s, 1H, OH-3'), 5.34 (d, *J =* 5.0 Hz, 1H, OH-2'), 6.16 (d, *J* = 6.1 Hz, 1H, H-1'), 6.56 (d, *J* = 3.7 Hz, 1H, H-5), 6.85-6.88 (m, 2H, HPh), 7.35-7.39 (m, 2H, HPh), 7.77 (d, *J =* 3.8 Hz, 1H, H-6), 8.67 (s, 1H, H-2). ¹³C NMR (100 MHz, DMSO-d₆) δ: 31.4 (SCH₂), 55.1 (OCH3), 61.5 (C-5'), 70.5 (C-3'), 74.1 (C-2'), 85.2 (C-4'), 86.9 (C-1'), 99.2 (C-5), 113.9 (2xCPh), 115.7 (C-4a), 126.3 (C-6), 129.6 (CPh), 130.2 (2xCPh), 148.5 (C-7a) 150.4 (C-2), 158.4 (CPh), 159.7 (C-4). HRMS calculated for C₁₉H₂₂N₃O₅S [M+H⁺]: 404.1275, found: 404.1309.

### 4-S-(3,4-dichlorobenzyl)-N7-(β-D-ribofuranosyl)pyrrolo[2,3-d]pyrimidine (FH15934)

**FH15934** was prepared according to the general procedure for S-alkylation. **FH14903** (0.050 g, 0.18 mmol) gave rise to **FH15934** (0.034 g, 0.076 mmol) as a white solid in 43 % yield.

¹H NMR (400 MHz, DMSO-d₆) δ: 3.55 (ddd, *J* = 11.8, 5.6, 4.2 Hz, 1H, H-5'), 3.63 (dt, *J* = 12.1, 4.7 Hz, 1H, H-5"), 3.92 (q, *J =* 3.8 Hz, 1H, H-4'), 4.09 - 4.12 (m, 1H, H-3'), 4.38 - 4.43 (m, 1H, H-2'), 4.65 (dd, *J= 14.0,* 1.2 Hz, 2H, SCH₂), 5.06 (t, *J* = 5.3 Hz, 1H, OH-5'), 5.16 (d, *J* = 4.8 Hz, 1H, OH-3'), 5.35 (d, *J* = 6.1 Hz, 1H, OH-2'), 6.16 (d, *J =* 6.0 Hz, 1H, H-1'), 6.60 (d, *J* = 3.8 Hz, 1H, H-5), 7.46 (dd, *J* = 8.3, 1.9 Hz, 1H, H-Ph), 7.57 (d, *J* = 8.3 Hz, 1H, H-Ph), 7.75 (d, *J* = 1.9 Hz, 1H, H-Ph), 7.80 (d, *J* = 3.9 Hz, 1H, H-6), 8.67 (s, 1H, H-2). ¹³C NMR (100 MHz, DMSO-d₆) δ: 30.4 (SCH₂), 61.5 (C-5'), 70.5 (C-3'), 74.1 (C-2'), 85.2 (C-4'), 87.0 (C-1'), 99.1 (C-5), 115.7 (C-4a), 126.5 (C-6), 129.3 (CPh), 129.7 (CPh), 130.6 (CPh), 130.8 (CPh), 130.9 (CPh), 139.7 (CPh), 148.5 (C7a), 150.3 (C-2), 159.7 (C-4). HRMS calculated for C₁₈H₁₈Cl₂N₃O₄S [M+H⁺]: 442.0390, found: 442.0402.

### 4-cylopentylmethylthio-N7-(β-D-ribofuranosyl)pyrrolo[2,3-d]pyrimidine (FH15939)

**FH15939** was prepared according to the general procedure for S-alkylation (T=60°C, 6days). **FH14903** (0.050 g, 0.18 mmol) gave rise to **FH15939** (0.016 g, 0.043 mmol) as a white solid in 24 % yield. Melting point: 181 °C.
¹H NMR (400 MHz, DMSO-d₆) δ: 1.26-1.35 (m, 2H, CH₂), 1.46-1.56 (m, 2H, CH2), 1.58-1.68 (m, 2H, CH₂), 1.76-1.84 (m, 2H, CH₂), 2.22 (sept., *J* = 7.5 Hz, 1H, CH), 3.38 (ddd, *J* = 13.3, 7.3, 1.5 Hz, 2H, SCH₂), 3.55 (ddd, *J* = 11.8, 5.6, 4.2 Hz, 1H, H-5'), 3.64 (dt, *J =* 12.0, 4.7 Hz, 1H, H-5"), 3.92 (q, *J =* 3.7 Hz, 1H, H-4'), 4.09 - 4.12 (m, 1H, H-3'), 4.38 - 4.43 (m, 1H, H-2'), 5.07 (t, *J =* 5.2 Hz, 1H, OH-5'), 5.16 (d, *J* = 4.8 Hz, 1H, OH-3'), 5.35 (d, *J* = 6.2 Hz, 1H, OH-2'), 6.15 (d, *J =* 6.3 Hz, 1H, H-1'), 6.59 (d, *J* = 3.8 Hz, 1H, H-5), 7.76 (d, *J =* 3.8 Hz, 1H, H-6), 8.61 (s, 1H, H-2). ¹³C NMR (100 MHz, DMSO-d₆) δ: 24.8 (2xCH₂), 31.7 (2xCH₂), 33.5 (SCH₂), 39.3* (CH), 61.5 (C-5'), 70.5 (C-3'), 74.1 (C-2'), 85.2 (C-4'), 87.0 (C-1'), 99.2 (C-5), 115.9 (C-4a), 126.2 (C-6), 148.3 (C7a), 150.4 (C-2), 160.3 (C-4). *value was obtained from a ¹H-¹³C gHSQC experiment. HRMS calculated for C₁₇H₂₄N₃O₄S [M+H⁺]: 366.1482, found: 366.1508

### 4-S-cyclohexylmethyl-N7-(β-D-ribofuranosyl)pyrrolo[2,3-d]pyrimidine (FH15962)

**FH15962** was prepared according to the general procedure for S-alkylation. **FH14903** (0.050 g, 0.18 mmol) gave rise to **FH15962** (0.057 g, 0.15 mmol) as a white solid in 85 % yield.

¹H NMR (400 MHz, DMSO-d₆) δ: 0.99-1.10 (m, 2H, CH₂), 1.11-1.24 (m, 3H, CH₂), 1.58-1.60 (m, 1H, CH₂), 1.60-1.62 (m, 1H, CH), 1.64-1.71 (m, 2H, CH₂), 3.28 (dd, *J* = 6.9, 2.3 Hz, 2H, SCH₂), 3.51-3.58 (m, 1H, H-5'), 3.61-3.66 (m, 1H, H-5"), 3.91 (q, *J* = 3.9 Hz, 1H, H-4'), 4.09-4.12 (m, 1H, H-3'), 4.38-4.43 (m, 1H, H-2'), 5.06 (t, *J* = 5.7 Hz, 1H, OH-5'), 5.15 (d, *J* = 4.6 Hz, 1H, OH-3'), 5.34 (d, *J* = 5.7 Hz, 1H, OH-2'), 6.15 (d, *J* = 5.7 Hz, 1H, H-1'), 6.59 (d, *J* = 4.0 Hz, 1H, H-5), 7.76 (d, *J* = 3.7 Hz, 1H, H-6), 8.61 (s, 1H, H-2). ¹³C NMR (100 MHz, DMSO-d₆) δ: 25.5 (2xCH₂), 25.8 (CH₂), 32.0 (2xCH₂), 34.5 (SCH₂), 37.5 (CH), 61.5 (C-5'), 70.5 (C-3'), 74.1 (C-2'), 85.2 (C-4'), 86.9 (C-1'), 99.2 (C-5), 116.0 (C-4a), 126.2 (C-6), 148.3 (C-7a) 150.3 (C-2), 160.3 (C-4). HRMS calculated for C₁₈H₂₆N₃O₅S [M+H⁺]: 380.1639, found: 380.1690.

### 4-S-phenethyl-N7-(β-D-ribofuranosyl)pyrrolo[2,3-d]pyrimidine (FH15953)

**FH15953** was prepared according to the general procedure for S-alkylation. **FH14903** (0.050 g, 0.18 mmol) gave rise to **FH15953** (0.055 g, 0.14 mmol) as a white waxy solid in 91 % yield. Melting point: 130 °C.

¹H NMR (400 MHz, DMSO-d₆) δ: 3.02 (t, *J* = 7.7 Hz, 2H, CH₂), 3.52-3.58 (m, 1H, H-5'), 3.60 (t, *J* = 7.4 Hz, 2H, CH₂), 3.60-3.66 (m, 2H, CH₂), 3.92 (q, *J* = 3.7 Hz, 1H, H-4'), 4.09-4.13 (m, 1H, H-3'), 4.39-4.43 (m, 1H, H-2'), 5.07 (t, *J* = 5.4 Hz, 1H, OH-5'), 5.15 (d, *J* = 4.8 Hz, 1H, OH-3'), 5.35 (d, *J* = 6.3 Hz, 1H, OH-2'), 6.16 (d, *J* = 6.11 Hz, 1H, H-1'), 6.57 (d, *J =* 3.9 Hz, 1H, H-5), 7.21-7.25 (m, 1H, HPh), 7.31-7.32 (m, 4H, HPh), 7.77 (d, *J* = 3.8 Hz, 1H, H-6), 8.66 (s, 1H, H-2). ¹³C NMR (100 MHz, DMSO-d₆) δ: 29.3 (CH₂), 35.1 (SCH₂), 61.5 (C-5'), 70.5 (C-3'), 74.1 (C-2'), 85.2 (C-4'), 86.9 (C-1'), 99.2 (C-5), 116.0 (C-4a), 126.2 (C-6), 126.4 (CPh),128.4 (2xCPh), 128.6 (2xCPh), 140.1 (CPh), 148.4 (C7a), 150.5 (C-2), 159.9 (C-4). HRMS calculated for C₁₉H₂₂N₃O₄S [M+H⁺]: 388.1326, found: 388.1350.

### 4-(3-phenylpropyl)thio-N7-(β-D-ribofuranosyl)pyrrolo[2,3-d]pyrimidine (FH15940)

**FH15940** was prepared according to the general procedure for S-alkylation (T=60°C, 2 days). **FH14903** (0.050 g, 0.18 mmol) gave rise to **FH15940** (0.052 g, 0.13 mmol) as a white solid in 73 % yield. Melting point: 90 °C.

¹H NMR (400 MHz, DMSO-d₆) δ: 2.02 (quint., *J* = 8.2 Hz, 2H, CH₂), 2.75 (t, *J =* 7.7 Hz, 2H, CH₂), 3.34 (t, *J* = 7.1 Hz, 2H, CH₂), 3.54 (ddd, *J* = 11.8, 5.8, 4.1 Hz, 1H, H-5'), 3.64 (dt, *J =* 12.0, 4.7 Hz, 1H, H-5"), 3.92 (q, *J* = 3.9 Hz, 1H, H-4'), 4.09 - 4.12 (m, 1H, H-3'), 4.38 - 4.43 (m, 1H, H-2'), 5.06 (t, *J* = 5.2 Hz, 1H, OH-5'), 5.15 (d, *J* = 5.2 Hz, 1H, OH-3'), 5.34 (d, *J =* 6.4 Hz, 1H, OH-2'), 6.15 (d, *J =* 6.4 Hz, 1H, H-1'), 6.59 (d, *J =* 3.6 Hz, 1H, H-5), 7.16-7.31 (m, 5H, H-Ph), 7.77 (d, *J =* 3.8 Hz, 1H, H-6), 8.61 (s, 1H, H-2). ¹³C NMR (100 MHz, DMSO-d₆) δ: 27.6 (CH₂), 30.8 (CH₂), 34.1 (CH₂), 61.5 (C-5'), 70.5 (C-3'), 74.1 (C-2'), 85.2 (C-4'), 86.9 (C-1'), 99.2 (C-5), 116.0 (C-4a), 125.9 (C-Ph), 126.2 (C-6), 128.3 (4xC-Ph), 141.1 (C-Ph), 148.4 (C7a), 150.4 (C-2), 160.0 (C-4). HRMS calculated for C₂₀H₂₄N₃O₄S [M+H⁺]: 402.1482, found: 402.1470.

### 6-S-alkyl pyrazolo[3,4-d]pyrimidine nucleosides were synthesized from JB944 (Scheme 2)

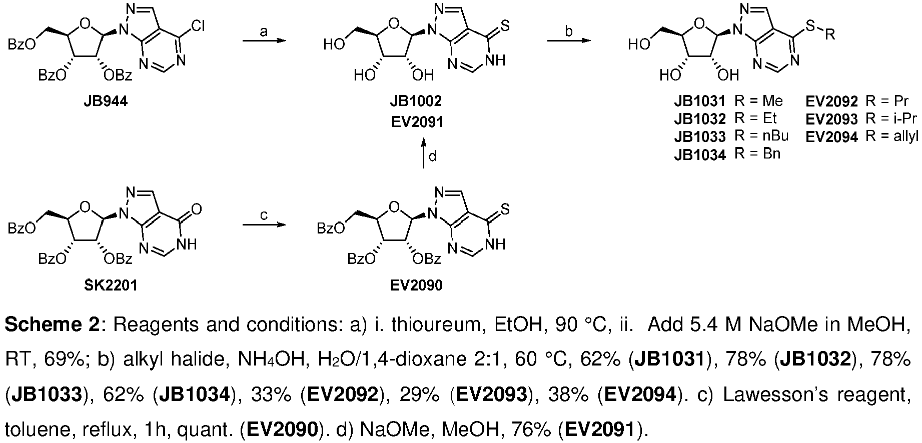

The 6-chloro group of **JB944** was exchanged for a sulfur via reaction with thioureum in ethanol at elevated temperature. In situ deprotection with sodium methoxide then afforded thiopurinol riboside **JB1002.** Alkylation of the sulfur atom with different alkyl halides led to compounds **JB1031-JB1034.**

### 4-methylthio-1-β-D-ribofuranosyl-1H-pyrazolo[3,4-d]pyrimidine (JB1031)

**JB1002** (0.071 g, 0.25 mmol) was subjected to general procedure D with methyl iodide as the electrophile. Purification of the obtained residue via flash column chromatography (2 →20% MeOH in CH₂Cl₂) afforded **JB1031** (46 mg, 0.154 mmol, 62% yield) as a white solid.

¹H NMR (400 MHz, DMSO-d₆) δ 2.72 (s, 3 H, CH₃), 3.43 (dt, *J*=11.7, 6.0 Hz, 1 H, H-5'), 3.58 (dt, *J*=11.7, 5.1 Hz, 1 H, H-5"), 3.93 (dd, *J*=10.4, 4.9 Hz, 1 H, H-4'), 4.24 (dd, *J*=10.0, 5.0 Hz, 1 H, H-3'), 4.64 (dd, *J*=10.3, 5.0 Hz, 1 H, H-2'), 4.75 (t, *J*=5.8 Hz, 1 H, OH), 5.19 (d, *J*=5.6 Hz, 1 H, OH), 5.44 (d, *J*=5.9 Hz, 1 H, OH), 6.22 (d, *J*=4.5 Hz, 1 H, H-1'), 8.45 (s, 1 H, H-3), 8.81 (s, 1 H, H-6) ppm. ¹³C NMR (101 MHz, DMSO-d₆) δ 11.6 (CH₃), 62.2 (C-5'), 70.8 (C-2'), 73.2 (C-3'), 85.3 (C-4'), 88.3 (C-1'), 112.0 (C-3a), 132.9 (C-3), 151.5 (C-7a), 154.4 (C-6), 165.4 (C-4) ppm. HRMS (ESI): calculated for C₁₁H₁₅N₄O₄S ([M+H]⁺): 299.0814, found: 299.0821.

### 4-ethylthio-1-β-D-ribofuranosyl-1H-pyrazolo[3,4-d]pyrimidine (JB1032)

**JB1002** (0.071 g, 0.25 mmol) was subjected to general procedure D with ethyl iodide as the electrophile. Purification of the obtained residue via flash column chromatography (2 →20% MeOH in CH₂Cl₂) afforded **JB1032** (61 mg, 0.195 mmol, 78% yield) as a white solid.

¹H NMR (400 MHz, DMSO-d₆) δ 1.37 (t, *J*=7.3 Hz, 3 H, CH₃), 3.35 - 3.47 (m, 3 H, CH₂, H-5'), 3.58 (dt, *J*=11.8, 5.1 Hz, 1 H, H-5"), 3.93 (dd, *J*=10.4, 4.9 Hz, 1 H, H-4'), 4.19 - 4.29 (m, 1 H, H-3'), 4.64 (m, *J*=3.4 Hz, 1 H, H-2'), 4.75 (t, *J*=5.7 Hz, 1 H, OH), 5.20 (br. s., 1 H, OH), 5.44 (br. s, 1 H, OH), 6.21 (d, *J*=4.5 Hz, 1 H, H-1'), 8.41 (s, 1 H, H-3), 8.81 (s, 1 H, H-6) ppm. ¹³C NMR (101 MHz, DMSO-d₆) δ 14.6 (CH₃), 23.0 (CH₂), 62.2 (C-5'), 70.8 (C-2'), 73.2 (C-3'), 85.3 (C-4'), 88.4 (C-1'), 112.1 (C-3a), 132.9 (C-3), 151.6 (C-7a), 154.4 (C-6), 164.9 (C-4) ppm. HRMS (ESI): calculated for C₁₂H₁₇N₄O₄S ([M+H]⁺): 313.0971, found: 313.0970.

### 4-propylthio-1-β-D-ribofuranosyl-1H-pyrazolo[3,4-d]pyrimidine (EV2092)

**EV2091** (0.120 g, 0.42 mmol) was subjected to general procedure D with n-propyl iodide as the electrophile. Purification of the obtained residue via flash column chromatography (2 →10% MeOH in CH₂Cl₂) afforded **EV2092** (46 mg, 0.14 mmol, 33% yield) as a white solid.
¹H NMR (400 MHz, methanol-d4) δ 1.07 (t, J=7 Hz, 3H, CH3), 1.80 (sext, J=7.5 Hz, 2H, CH2(-CH3)), 3.37 (t, J=7 Hz, 2H, CH2(-S)), 3.78 (dd, J=12;4 Hz, 1H, H-5'), 3.94 (dd, J=12;3 Hz, 1H, H-5"), 4.20 (m, 1H, H-4'), 4.38 (t, J=5.5 Hz, 1H, H-3'), 4.52 (m, 1H, H-2'), 6.04 (d, J=2.5 Hz, 1H, H-1'), 8.66 (s, 1H, H-6), 8.87 (s, 1H, H-3) ppm
¹³C NMR (101 MHz, methanol-d4) δ 13.6 (CH₃), 23.8 (CH₂ (- CH₃)), 31.9 (CH₂ - S), 62.6 (C-5'), 71.4 (C-3'), 77.5 (C-2'), 87.2 (C-4'), 97.4 (C-1'), 112.6 (C-3a), 125.6 (C-3), 155.7 (C-6), 158.2 (C-7a), 170.7 (C-4)

### 4-isopropylthio-1-β-D-ribofuranosyl-1H-pyrazolo[3,4-d]pyrimidine (EV2093)

**EV2091** (0.120 g, 0.42 mmol) was subjected to general procedure D with isopropyl iodide as the electrophile. Purification of the obtained residue via flash column chromatography (2 →8% MeOH in CH₂Cl₂) afforded **EV2093** (38 mg, 0.12 mmol, 29% yield) as a white solid.
¹H NMR (400 MHz, methanol-d4) δ 1.48 (d, J=7 Hz, 6H, 2xCH3), 3.78 (dd, J=12;4 Hz, 1H, H-5'), 3.94 (dd, J=12;2.5 Hz, 1H, H-5"), 4.20 (m, 1H, H-4'), 4.27-4.45 (m, 2H, H-3' + CH (isopropyl)), 4.51 (m, 1H, H-2'), 6.03 (d, J=2.5 Hz, 1H, H-1'), 8.67 (s, 1H, H-6), 8.84 (s, 1H, H-3)
¹³C NMR (101 MHz, methanol-d4) δ 23.2 (CH₃), 35.1 (CH - S), 62.6 (C-5'), 71.4 (C-3'), 77.5 (C-2'), 87.2 (C-4'), 97.4 (C-1'), 112.5 (C-3a), 125.6 (C-3), 155.8 (C-6), 158.2 (C-7a), 170.7 (C-4)

### 4-allylthio-1-β-D-ribofuranosyl-1H-pyrazolo[3,4-d]pyrimidine (EV2094)

**EV2091** (0.120 g, 0.42 mmol) was subjected to general procedure D with allyl iodide as the electrophile. Purification of the obtained residue via flash column chromatography (2 →10% MeOH in CH₂Cl₂) afforded **EV2094** (51 mg, 0.16 mmol, 38% yield) as a white solid.
1H NMR (400 MHz, DMSO-d6) δ 3.56-3.67 (m, 1H, H-5'), 3.70-3.81 (m, 1H, H-5"), 4.00-4.14 (m, 3H, H-4' + CH2(-S)), 4.20 (quart, J=5.5 Hz, 1H, H-3'), 4.93 (dd, J=10.5;4 Hz, 1H, H-2'), 5.09 (t, J=5 Hz, 1H, OH (C-5')), 5.17 (d, J=10 Hz, 1H, H geminal), 5.22 (d, J=6 Hz, 1H, OH (C-3')), 5.39 (d, J=16 Hz, 1H, H geminal), 5.69 (d, J=5 Hz, 1H, H-1'), 5.94-6.07 (m, 2H, H allyl (non-geminal) + OH (C-2')), 8.75 (s, 1H, H-6), 9.07 (s, 1H, H-3)
¹³C NMR (101 MHz, DMSO-d6) δ 31.3 (CH2 - S), 61.3 (C-5'), 70.1 (C-3'), 76.0 (C-2'), 86.0 (C-4'), 95.8 (C-1'), 111.0 (C-3a), 119.1 (CH2 allyl geminal), 124.8 (C-3), 133.6 (CH allyl non-geminal), 154.7 (C-6), 157.4 (C-7a) 166.8 (C-4).

### 4-butylthio-1-β-D-ribofuranosyl-1H-pyrazolo[3,4-d]pyrimidine (JB1033)

**JB1002** (0.071 g, 0.25 mmol) was subjected to general procedure D with butyl iodide as the electrophile. Purification of the obtained residue via flash column chromatography (2 →20% MeOH in CH₂Cl₂) afforded **JB1033** (66 mg, 0.193 mmol, 78% yield) as a white solid.

¹H NMR (400 MHz, DMSO-*d*₆) δ 0.92 (t, *J*=7.4 Hz, 3 H, CH₃), 1.37 - 1.53 (m, 2 H, CH₂), 1.63 - 1.77 (m, 2 H, CH₂), 3.35 - 3.47 (m, 3 H, CH₂, H-5'), 3.57 (dt, *J*=11.5, 5.1 Hz, 1 H, H-5"), 3.93 (dd, *J*=10.1, 5.0 Hz, 1 H, H-4'), 4.24 (dd, *J*=10.0, 5.0 Hz, 1 H, H-3'), 4.64 (dd, *J*=10.4, 5.0 Hz, 1 H, H-2'), 4.75 (t, *J*=5.8 Hz, 1 H, OH), 5.19 (d, *J*=5.5 Hz, 1 H, OH), 5.43 (d, *J*=5.9 Hz, 1 H, OH), 6.21 (d, *J*=4.6 Hz, 1 H, H-1'), 8.42 (s, 1 H, H-3), 8.80 (s, 1 H, H-6) ppm. ¹³C NMR (101 MHz, DMSO-d₆) δ 13.5 (CH₃), 21.3 (CH₂), 28.0 (CH₂), 30.9 (CH₂), 62.2 (C-5'), 70.8 (C-2'), 73.2 (C-3'), 85.3 (C-4'), 88.4 (C-1'), 112.1 (C-3a), 132.9 (C-3), 151.6 (C-7a), 154.4 (C-6), 165.0 (C-4) ppm. HRMS (ESI): calculated for C₁₄H₂₁N₄O₄S ([M+H]⁺): 341.1284, found: 341.1297.

### 4-benzylthio-1-β-D-ribofuranosyl-1H-pyrazolo[3,4-d]pyrimidine (JB1034)

**JB1002** (0.071 g, 0.25 mmol) was subjected to general procedure D with benzyl bromide as the electrophile. Purification of the obtained residue via flash column chromatography (2 →20% MeOH in CH₂Cl₂) afforded **JB1034** (58 mg, 0.155 mmol, 62% yield) as a white solid.

¹H NMR (400 MHz, DMSO-d₆) δ 3.43 (dt, *J*=11.8, 5.9 Hz, 1 H, H-5'), 3.57 (dt, *J*=10.5, 5.3 Hz, 1 H, H-5"), 3.93 (dd, *J*=10.1, 4.9 Hz, 1 H, H-4'), 4.24 (dd, *J*=10.1, 5.0 Hz, 1 H, H-3'), 4.64 (dd, *J*=10.3, 5.1 Hz, 1 H, H-2'), 4.70 (s, 2 H, CH₃), 4.74 (t, *J*=5.8 Hz, 1 H, OH), 5.19 (d, *J*=5.6 Hz, 1 H, OH), 5.44 (d, *J*=5.9 Hz, 1 H, OH), 6.22 (d, *J*=4.5 Hz, 1 H, H-1'), 7.23 - 7.36 (m, 3 H, H-Phe), 7.47 (d, *J*=7.1 Hz, 2 H, H-Phe), 8.43 (s, 1 H, H-3), 8.86 (s, 1 H, H-6) ppm. ¹³C NMR (101 MHz, DMSO-d₆) δ 32.1 (CH₂-Bn), 62.2 (C-5'), 70.8 (C-2'), 73.2 (C-3'), 85.3 (C-4'), 88.4 (C-1'), 111.8 (C-3a), 127.4 (C-Phe), 128.1 (C-Phe), 128.6 (C-Phe), 129.0 (C-Phe), 132.9 (C-3), 137.1 (C-Phe), 151.7 (C-7a), 154.4 (C-6), 164.2 (C-4) ppm. HRMS (ESI): calculated for C₁₇H₁₉N₄O₄S ([M+H]⁺): 375.1127, found: 375.1133.

### S-aryl pyrazolo[3,4-d]pyrimidine nucleosides were synthesized from JB944 (scheme 3)

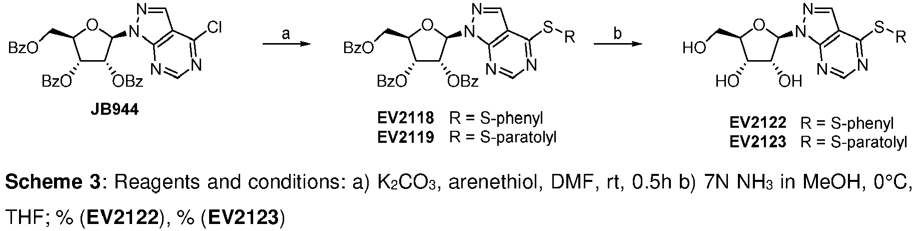

### 4-phenylthio-1-β-D-ribofuranosyl-1H-pyrazolo[3,4-d]pyrimidine (EV2122)

**JB944** (500 mg, 0.84 mmol) was dissolved in DMF (5 ml). K₂CO₃ (138 mg, 1 mmol) and benzenethiol (102 µL, 1 mmol) were added to the solution. After 30 min, the reaction was finished and volatiles were evaporated. The residue was purified via flash column chromatography (15 →40% ethyl acetate in petroleum ether). **EV2118** (416 mg, 0.62 mmol, 74%) was obtained as a clear oil. The identification and purity was assessed via LC-MS, before continuing to the next step.

**EV2118** (416 mg, 0.62 mmol) was dissolved in THF (3 ml) and added to 7N NH₃ in MeOH (12 ml) in a pressure tube at 0°C. The solution was stirred until the reaction no longer progressed (monitored via LC-MS). Cs₂CO₃ (60 mg, 0.19 mmol) was added to carefully run the reaction to completion. The product will degrade if the reaction mixture is stirred too long after the addition of Cs₂CO₃. Acetic acid (5 ml, 87.5 mmol) was added to quench the reaction. Volatiles were evaporated and the residue was purified by flash column chromatography (1 →10% MeOH in CH₂Cl₂). **EV2122** (20 mg, 0.055 mmol, 9% yield) was obtained as a white solid.

1H NMR (400 MHz, methanol-d4): δ 3.71 (dd, J=12.2;4.6 Hz, 1H, H-5'), 3.86 (dd, J=12.2;3.2 Hz, 1H, H-5"), 4.14-4.20 (m, 1H, H-4'), 4.28 (t, J=5.4 Hz, 1H, H-3'), 4.47 (dd, J=5.0;3.2 Hz, 1H, H-2'), 7.50-7.64 (m, 2H, Ph-H), 7.67-7.74 (m, 2H, Ph-H), 7.99 (s, 1H, H-3), 8.56 (s, 1H, H-6)

### 4-paratolylthio-1-β-D-ribofuranosyl-1H-pyrazolo[3,4-d]pyrimidine (EV2123)

**EV2119** (765 mg, 1.11 mmol, 92% yield) was obtained using the same procedure as **EV2118,** with **JB944** (703 mg, 1.21 mmol), K₂CO₃ (217 mg, 1.57 mmol) and 4-methylbenzenethiol (226 mg, 1.82 mmol).

**EV2123** () was obtained as a white solid via the same procedure as **EV2122,** with **EV2119** (765 mg, 1.11), THF (4 ml), 7N NH3 in MeOH (15 ml), Cs₂CO₃ (108 mg, 0.33 mmol) and AcOH (7 ml). 1H NMR (400 MHz, DMSO-d6) δ 2.41 (s, 1H, CH3), 3.54 (dt, J=12 ; 4 Hz, 1H, H-5'), 3.70 (dt, J=12; 5 Hz, 1H, H-5"), 4.02 (quart, J=3.5 Hz, 1H, H-4'), 4.12 (quart, J=5 Hz, 1H, H-3'), 4.34 (quart, J=4.5 Hz, 1H, H-2'), 5.02 (t, J=5 Hz, 1H, OH(C-5')), 5.22 (d, J=6 Hz, 1H, OH(C-3')), 5.68 (d, J=5.5 Hz, 1H, OH(C-2')), 5.93 (d, J=3 Hz, 1H, H-1'), 7.36 (d, J=8 Hz, 2H, Ar-H (meta to S)), 7.78 (d, J=8 Hz, 2H, Ar-H (ortho to S)), 8.49 (s, 1H, H-3), 8.69 (s, 1H, H-6)

### EXPERIMENTAL BIOLOGICAL EVALUATION

### Methods

### Animals and parasites

Female Swiss mice were purchased from Janvier (France). Food for laboratory rodents (Carfil, Arendonk, Belgium) and drinking water were available *ad libitum.* The animals were kept in quarantine for at least 5 days before infection and were randomly allocated to the experimental units.

*T. congolense* (IL3000), the isometamidium (ISM)-resistant *T. congolense* strain (MSORO M7H), *T. evansi type A* (RoTat 1.2), *T. evansi type B* (KETRI 2479) and *T. equiperdum* (BoTat 1.1) used for *in vitro, ex vivo* and *in vivo* experiments were kindly provided by the Institute of Tropical Medicine (ITM), and *T. vivax* (ILRAD700) and *T. congolense* (TC13) used for *ex vivo* and *in vivo* experiments, were kindly provided by the University of Brussel (VUB).

### Cytotoxicity assay on MRC-5 fibroblasts

MRC-5_{SV2} (Sigma-Aldrich/ECACC, catalogue number 84100401) human embryonic lung fibroblasts were used to perform cytotoxicity assays. The cells were cultured in minimum essential medium (MEM, Life Technologies) with Earle's salt medium, supplemented with L-glutamine, NaHCO3 and 5% inactivated fetal bovine serum (iFBS). Cells were seeded at 1.5×10⁴ cells/well and 4-fold compound dilutions were immediately added with a highest in-test concentration of 64 µM. After a 3-day incubation, resazurin (Sigma-Aldrich) was added to each well (final concentration of 10 µg mL⁻¹), and cell viability was assessed by fluorescent reading (Tecan^{®}, GENios) following 4h of incubation with resazurin (37°C/5% CO₂). The results were expressed as percentage reduction in cell growth/viability compared to control wells and the 50% cytotoxic concentration (CC₅₀) was calculated for each of the compounds.

### In vitro drug susceptibility assays

Drug susceptibility assays with *T. brucei* (Squib 427), *T. evansi type A* (RoTat 1.2) and *T. equiperdum* (BoTat 1.1) were performed under similar conditions. Parasites were cultured in HMI-9 culture medium supplemented with 10% iFBS. To determine *in vitro* susceptibility of the abovementioned *Trypanosoma* species, 10 concentrations of a 4-fold dilution series (with a highest in-test concentration of 64 µM) was prepared for each compound, and parasites were seeded at 1.5×10⁴ parasite/well. After 72h of drug exposure at 37°C with 5% CO₂, resazurin was added to the wells (final concentration of 10 µg mL⁻¹), followed by another incubation for 24h. Cell viability was determined by fluorescence reading (Tecan^{®}, GENios) and 50% inhibitory concentration (IC₅₀) was calculated for each compound.

Drug susceptibility assays for *T. congolense* (IL3000) were performed with parasites cultured in HMI-9 medium supplemented with 15% horse serum. Parasites were seeded at a concentration of 1.5×10⁴ parasite/well and exposed to a 4-fold compound dilution series starting at a highest in-test concentration of 64 µM. Plates were incubated for 72h at 34°C and 5% CO₂, followed by the addition of resazurin (Sigma, Aldrich) and a further incubation period of 24h. Cell viability was quantified by fluorescence reading (Tecan^{®}, GENios), and IC₅₀ values were calculated by non-linear regression (dose-response curve with variable slope) using Prism 8.0 (GraphPad Software Inc., San Diego, CA, USA).

### Ex vivo drug susceptibility assays for T. vivax, T. congolense ISM-resistant, and T. evansi type B

For *Trypanosoma* species/strains that were not adapted to culture conditions, donor mice were infected intraperitoneally with 10⁴ parasites. Around day 5-6 post infection, blood was collected via cardiac puncture and parasites were isolated using mini-anion exchange centrifugation technique (mAECT). 4-fold dilutions of the test compounds were prepared with the highest concentration of 64 µM, and parasites were seeded at 1x10⁵ parasite/well. Following 24h of drug exposure at 37°C and 5% CO₂, resazurin was added to the wells and plates were incubated (37°C/5% CO₂) for another 24h. Cell viability was determined by fluorescence reading (Tecan^{®}, GENios), and IC₅₀ values were calculated by non-linear regression (dose-response curve with variable slope) using Prism 8.0 (GraphPad Software Inc., San Diego, CA, USA).

### In vitro metabolic stability

The metabolic stability of selected compounds was determined in the presence of mice, horse, and bovine liver microsome fractions (S9) through phase-I (CYP₄₅₀ and NADPH dependent enzymes) and Phase-II (UGT enzymes) metabolisms. Samples were collected after 0, 15, 30, and 60 minutes of incubation and the corresponding loss of parent compound was determined via liquid chromatography (UPLC) (Waters Aquity^{™}) coupled with tandem quadrupole mass spectrometry (MS²) (Waters Xevo^{™}), equipped with an electrospray ionization (ESI) interface and operated in multiple reaction monitoring (MRM) mode. For some compounds, the mouse microsome was pre-incubated with 1-aminobenzotriazole (1-ABT) 30 minutes prior to the addition of test compounds, to assess whether it adequately block phase-I metabolic degradation.

### In vivo experiments

A late curative mouse model was designed for each of three AAT species (*T. vivax, T. congolense, T. evansi*). For each model, female Swiss mice (Janvier Labs, France) were randomly distributed to groups of three animals, and intraperitoneally infected with 10⁴ of *T.vivax (ILRAD700)* or *T. congolense* (TC13) or *T.evansi* (RoTat 1.2) parasites derived from heavily infected donor mice. Compounds were formulated in 10% (v/v) polyethylene glycol 400 (PEG₄₀₀) in water at 2 or 2.5 mg mL⁻¹. Each compound was freshly prepared before administration and was intraperitoneally administrated s.i.d. (i.e. once daily) for 5 days at 50 mg mL⁻¹. 1-ABT was formulated in 0.5% (v/v) Tween₈₀ in water at 25 mg mL⁻¹ and was orally administrated at 100 mg kg⁻¹ s.i.d. two hours before the administration of test compounds (CL5565, FH15963 and FH15967). The reference drug diminazene aceturate was formulated in phosphate-buffered saline (PBS) at 2.5 mg ml⁻¹ and administrated s.i.d. i.p. for 5 days at 10 mg kg⁻¹. All treatments were initiated at day 3 or day 5 post infection, when the blood parasitemia reached 10⁶ parasites mL⁻¹. The occurrence of clinical or adverse effects were monitored and blood parasitemia was determined through the microscopic evaluation of tail vein blood samples on daily basis until 14dpi, twice a week until 28dpi, and once a week until 60dpi (pre-set endpoint). The body weigh was also recorded at the same timepoints.

For pharmacokinetic analyses, blood samples were collected at 30 mins, 1, 2, 4, 8, and 24 hours following the first treatment dose. 15 µL of blood was dropped on Whatman^{®} FTA^{®} DMPK A cards, dried out at room temperature for at least 2 hours, and processed for analysis via liquid chromatography (UPLC) coupled with tandem quadrupole mass spectrometry (MS²) (Waters Xevo^{™}).

### In vivo bioluminescence imaging

Female Swiss mice (BW ~ 20-24g; Janvier Labs, France) were randomly allocated to group of 3 animals and were intraperitoneally infected with 2x10³ *T. b. brucei* AnTAR1.1 PPYRE9 derived from a heavily infected donor mouse. FH15967 was formulated in 10% (v/v) polyethylene glycol 400 (PEG400) in water at 2 mg mL⁻¹ and was administrated to separate groups as single dose s.i.d. i.p. at 50 mg kg-1 (n=3), and b.i.d i.p. at 50 mg kg⁻¹ (n=3). The reference drug diminazene aceturate was formulated in PBS at 2.5 mg mL⁻¹ and administrated s.i.d i.p. as a single dose at 10 mg kg⁻¹. Treatment was initiated at 3dpi when parasitemia reached 10⁶ parasites mL⁻¹, and parasitemia was microscopically determined via Neubaur improved haemocytometer on a daily basis until 7dpi. Bioluminescent images were obtained at 3, 5 and 7dpi to determine parasite burdens in the body. Accordingly, D-luciferin was injected i.p. at 15 mg kg⁻¹, animals were anaesthetised using isoflurane, and luminescent signals were acquired from both the dorsal and ventral sides of the animals. Bioluminescent images were acquired at exposure time of 5 seconds from all animals prior to treatment, and also from animals in the vehicle control groups during the experiment with high parasite burdens. Animals in the treatment groups were additionally imaged at exposure times of 15, 60, 180 and 300 seconds. BLI images that obtained from IVIS Spectrum In vivo Imaging System (Perkin Elmer) were quantified with Living Image V4.3.1 SoftWoRx Suite 2.0 for microscopy, then processing using the Fiji software.

### Results

### 6-substituted S-alkyl derivatives of tubercidin display highly potent in vitro activity

Based on the reported activity of tubercidin analogues against African trypanosomes, an enriched library subset of 93 antiprotozoal nucleosides was evaluated for *in vitro* activity against *T. brucei* and *T. vivax* in search of pan-active compounds against the five most relevant AT species. Selectivity was assessed by evaluating cytotoxicity against the human MRC-5 fibroblast cell line. Substantial activity was recorded in a series of 6-substituted S-alkyl derivatives of tubercidin (Table 1).

**Table 1: In vitro evaluation of S-alkyl derivatives of tubercidin against T. brucei and T. vivax. MRC-5 fibroblasts were included as a read-out for off-target cytotoxicity. Results are expressed in µM and represent the mean and SEM of two independent experiments.**

| **Compound** | **MRC-5** | ***T. brucei*** | ***T. vivax*** |
|---|---|---|---|
| **FH14905** | >64 | 0.019 ± 0.002 | 32.3 ± 4.85 |
| **FH15934** | 13.9 ± 0.97 | 0.085 ± 0.001 | 8.87 ± 0.09 |
| **FH15935** | >64 | 0.030 ± 0.0009 | 0.023 ± 0.005 |
| **FH15936** | >64 | 0.030 ± 0.0001 | 0.024 ± 0.007 |
| **FH15937** | 35.3 ± 1 | 0.031 ± 0.001 | 2.19 ± 0.61 |
| **FH15938** | 28.25 ± 2.03 | **0.25 ± 0.05** | 0.031 ± 0.009 |
| **FH15939** | 32.30 ± 1 | 0.032 ± 0.021 | 0.69 ± 0.25 |
| **FH15940** | 24.3 ± 0.94 | 0.106 ± 0.004 | 8.71 ± 1.19 |
| **FH15941** | 26.9 ± 1.38 | 0.44 ± 0.02 | 2.37 ± 1.47 |
| **FH15952** | 45.5 ± 1.87 | 0.091 ± 0.001 | 0.62 ± 0.16 |
| **FH15953** | 28.9 ± 1.34 | 0.41 ± 0.01 | 4.93 ± 0.59 |
| **FH15954** | 43.2 ± 2.63 | 0.030 ± 0.004 | 0.23 ± 0.02 |
| **FH15955** | >64 | 0.032 ± 0.001 | 0.021 ± 0.002 |
| **FH15956** | >64 | 0.36 ± 0.04 | 1.98 ± 0.94 |
| **FH15957** | 58.8 ± 1.37 | 0.19 ± 0.04 | 3.16 ± 0.71 |
| **FH15958** | >64 | 0.54 ± 0.04 | 0.46 ± 0.25 |
| **FH15959** | >64 | 0.031 ± 0.001 | 18.8 ± 0.65 |
| **FH15960** | >64 | 0.13 ± 0.008 | 0.82 ± 0.27 |
| **FH15961/CL5565** | >64 | 0.44 ± 0.001 | 0.005 ± 0.01 |
| **FH15962** | 19 | 0.031 ± 0.01 | 2.86 ± 0.26 |
| **FH15963** | >64 | 0.034 ± 0.001 | 0.034 ± 0.004 |
| **FH15967** | >64 | 0.031 ± 0.008 | 0.044 ± 0.005 |
| **JB1032** | >64 | 0.51 ± 0.05 | 1.36 ± 0.30 |
| **JB1033** | >64 | 0.26 ± 0.01 | 1.21 ± 0.42 |

Based on the activity of compounds against *T. brucei* and *T. vivax,* 16 compounds without non-specific cytotoxicity were shortlisted for further evaluation against all other relevant AT species [a sensitive and ISM-resistant *T. congolense* strain, *T. evansi* type A and B, *T. equiperdum].* This analysis resulted in a particularly interesting set of 3 compounds with confirmed *in vitro* pan-AT activity (Table 2), while other compounds may be less suitable for pan-AT applications, evidently they still have their value for specific AT applications, as being highly active against one or more *Trypanosoma* species, as evident from table 1..

**Table 2: In vitro evaluation results of confirmed nucleoside leads [IC₅₀(µM)]. IC₅₀ values are from two independent repeat experiments of a serial dilution (4-fold, 10 dilutions) from 64 to 0.00006 µM in technical duplicate.**

| **Comp.** | **MRC-5** | ***T. brucei*** | ***T. vivax*** | ***T. congolense*** | ***T. congolense ISM-R*** | ***T. evansi (type A)*** | ***T. evanst (type B)*** | ***T.* equiperdum** |
|---|---|---|---|---|---|---|---|---|
| **CL5565** | >64 | 0.49 ± 0.1 | 0.004 ± 0.002 | 0.043 ± 0.008 | 0.43 ± 0.02 | 0.76 ± 0.03 | 0.016 ± 0.003 | 0.04 ± 0.0002 |
| **FH15963** | >64 | 0.034 ± 0.001 | 0.034 ± 0.004 | 0.18 + 0.008 | 0.14 ± 0.05 | 0.10 ± 0.005 | 0.014 ± 0.004 | 0.025 ± 0.004 |
| **FH15967** | >64 | 0.031 ± 0.008 | 0.044 ± 0.005 | 0.10 ± 0.02 | 0.18 ± 0.009 | 0.033 ± 0.006 | 0.018 ± 0.006 | 0.026 ± 0.005 |

### Compounds CL5565, FH15963 and FH15967 are metabolically stable

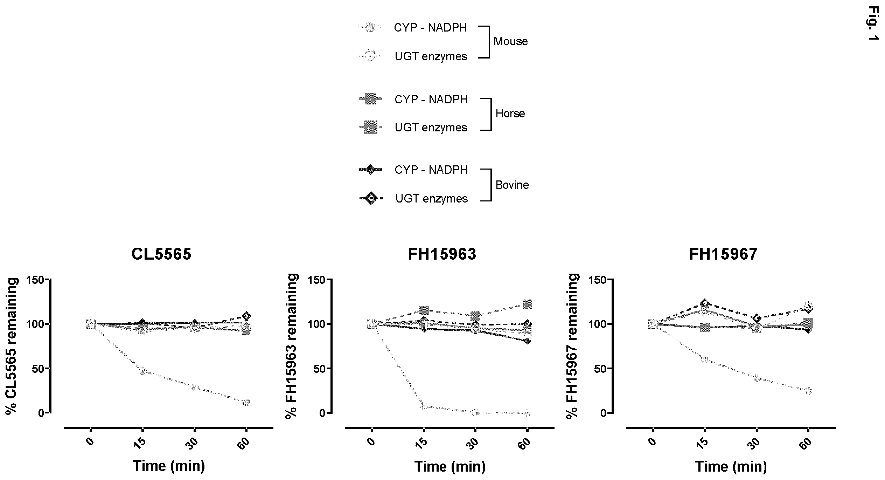

Incubation of selected analogues with mouse, horse, and bovine liver microsome (S9) fractions revealed that all 3 compounds are metabolically stable in the target (horse, bovine) species, defined as ≥50% of parent compound remaining after 30 minutes. Although CL5565, FH15963 and FH15967 showed phase-I metabolic degradation in mouse liver microsomes, the non-selective cytochrome inhibitor 1-aminobenzotriazole (1-ABT) was found to adequately block phase-I metabolic decay of these three compounds hereby demonstrating that co-administration of 1-ABT enables proper *in vivo* evaluation of selected compounds for potency against AT in our mouse models (Figure 1).

### FH15967 shows excellent efficacy in late curative AAT models

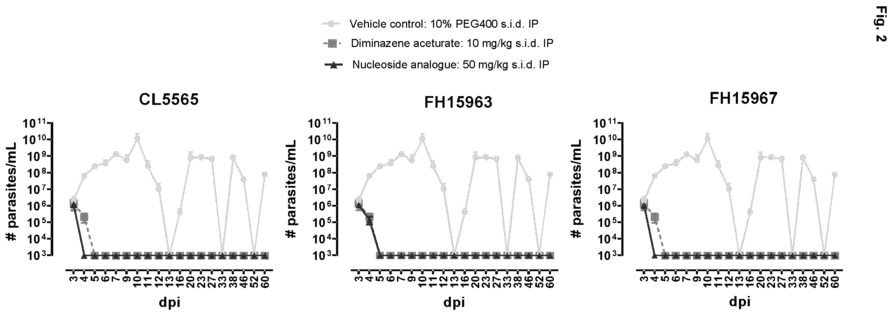

The selected compounds were further investigated for their *in vivo* efficacies in *T. congolense, T. vivax* and *T. evansi* late curative mouse models, where treatment (50 mg kg⁻¹ s.i.d. i.p. for 5 consecutive days) starts when parasitemia reaches ≥10⁶ trypanosome mL⁻¹. All three compounds provide full cure against *T. vivax,* confirmed by microscopic evaluation of tail vein blood samples (Figure 2). Notably, all three compounds displayed equipotent or more potent activity against *T. vivax* than the reference compound, diminazene aceturate, at the tested doses.

In a next step, the *in vivo* efficacy of FH15967 was evaluated in a bioluminescent *T. brucei* late curative model with a single-dose i.p. treatment at 50 mg kg⁻¹. All treated animals showed negative blood parasitemia until day 7 post infection. Bioluminescent imaging and signal quantification revealed a rapid parasitemia decline in the test group, even faster than the diminazene aceturate reference group (Figure 3).

### Experimental data of the compounds wherein X = N

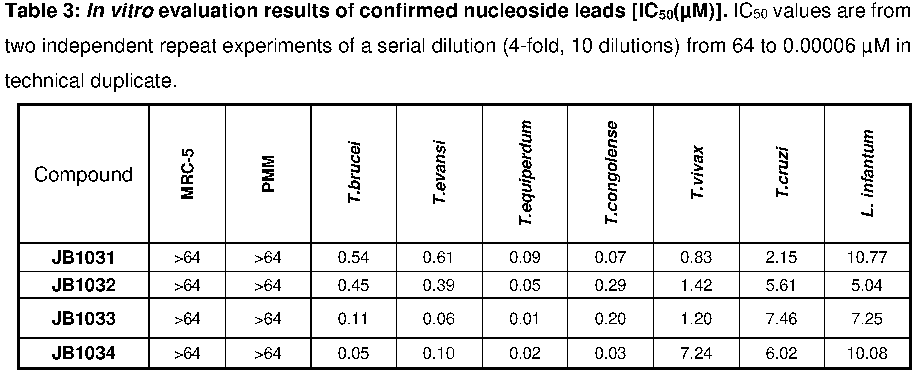

## Claims

1. A compound of Formula I or a stereoisomer, tautomer, racemic, salt, hydrate, or solvate thereof, 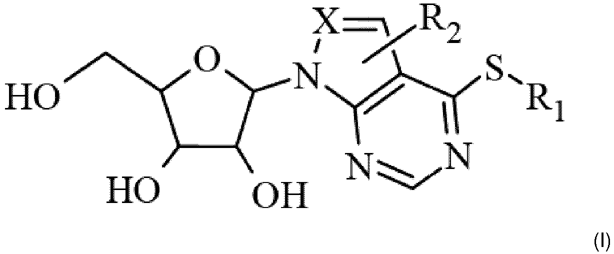 wherein
X is C;
R₁ is selected from the group comprising: -C₂₋₆alkyl, -C₂₋₆alkenyl, -CF₃, -C₁₋₆alkyl-R₃, -Ar₁, -C₂₋₆alkyl-Ar₁, -Het₁ and -Cy₁;
R₂ is selected from the group comprising: -H or -halo;
R₃ is selected from the group comprising: -C₁₋₆alkyl, -C₁₋₆alkenyl, -C₁₋₆alkynyl, -Het₂ and -Cy₂,
Ar₁ is a 5- to 10-membered aromatic cycle optionally comprising 1 to 3 heteroatoms selected from O, N and S; said Ar₁ optionally being substituted with one or more substituents independently selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -CF₃, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 3- to 10-membered non-aromatic cycle; each of said Cy₁ and Cy₂ optionally being substituted with one or more substituents independently selected from the list comprising: -halo, -CF₃, -C₁₋₆alkyl, -C₁₋₆alkenyl, -C₁₋₆alkynyl, -O-C₁₋₆alkyl, and -S-C₁₋₆alkyl.

2. A compound according to claim 1 wherein;
R₁ is selected from the group comprising: -C₂₋₆alkyl, -C₁₋₆alkyl-R₃, -C₂₋₆alkyl-Ar₁, -Ar₁, -Het₁ and -Cy₁;
R₂ is H;
R₃ is selected from the group comprising: -C₁₋₆alkyl, -C₁₋₆alkenyl, -Het₂ and -Cy₂,
X is C
Ar₁ is a 5- to 6-membered aromatic cycle; said Ar₁ optionally being substituted with one or more substituents independently selected from the list comprising: -halo, -C₁₋₆alkyl, and -O-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -CF₃, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 5- to 6-membered non-aromatic cycle; each of said Cy₁ and Cy₂ optionally being substituted with one or more substituents independently selected from the list comprising: -halo, -C₁₋₆alkyl, and -O-C₁₋₆alkyl.

3. A compound according to claim 1 wherein;
R₁ is selected from the group comprising: -C₂₋₆alkyl, -C₁₋₃alkyl-R₃, -C₂₋₃alkyl-Ar₁, -Ar₁, -Het₁ and -Cy₁;
R2 is H;
R₃ is selected from the group comprising: -C₁₋₆alkyl, -C₁₋₆alkenyl, - Het₂ and -Cy₂,
X is C;
Ar₁ is -phenyl; optionally being substituted with one or more substituents selected from the list comprising: -halo, -C₁₋₆alkyl, and -O-C₁₋₆alkyl;
Het₁ and Het₂ are each independently -pyridinyl;
Cy₁ and Cy₂ are each independently selected from - cyclopentane and -cycloheptane.

4. A compound according to claim 1 wherein;
R₁ is selected from the group comprising: -C₂₋₆alkyl, -C₁₋₃alkyl-R₃, -C₂₋₃alkyl-Ar₁, -Ar₁, -Het₁ and -Cy₁;
R2 is H;
R₃ is selected from the group comprising: -C₁₋₆alkyl, -C₁₋₆alkenyl, and -Cy₂,
X is C;
Ar₁ is -phenyl optionally being substituted with one or more substituents selected from the list comprising: -halo, -C₁₋₆alkyl, and -O-C₁₋₆alkyl;;
Het₁ is -pyridinyl;
Cy₁ and Cy₂ are each independently selected from -cyclopentane and -cycloheptane.

5. A compound according to claim 1 and being selected from the list comprising;

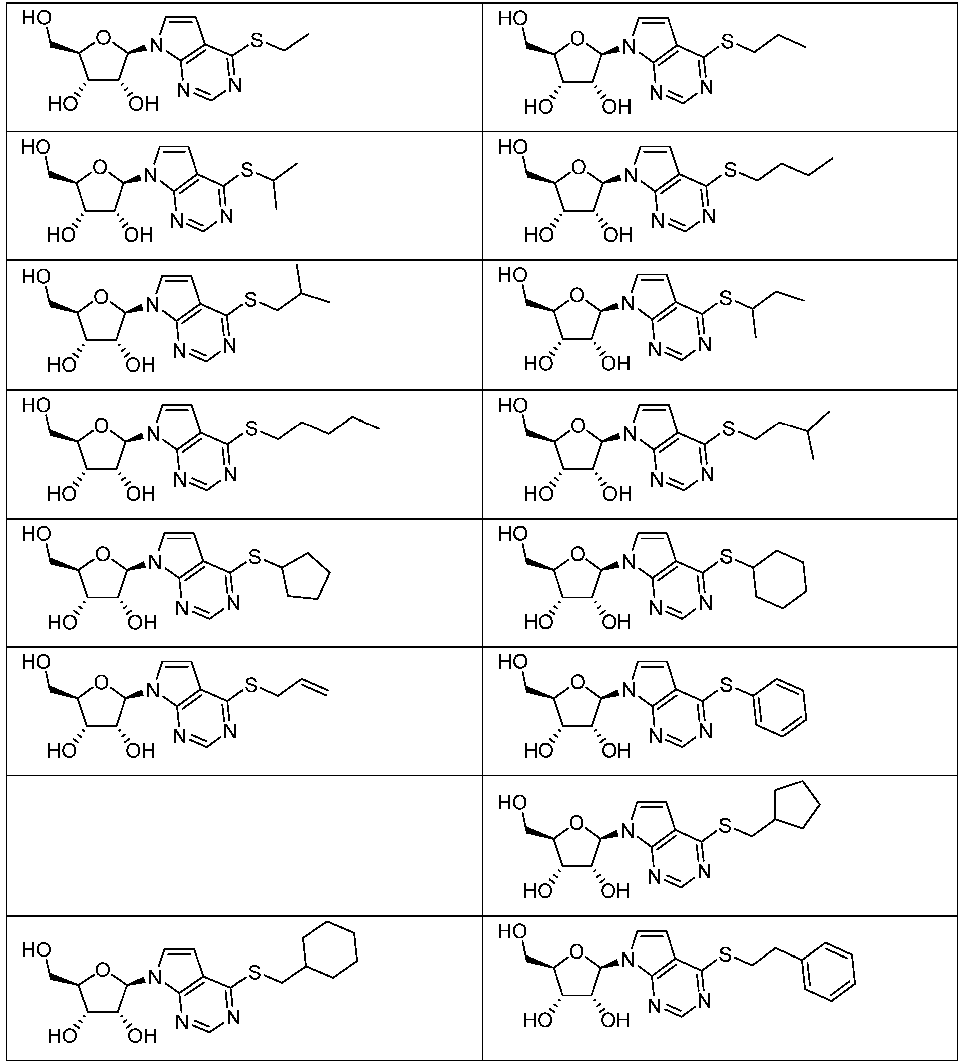

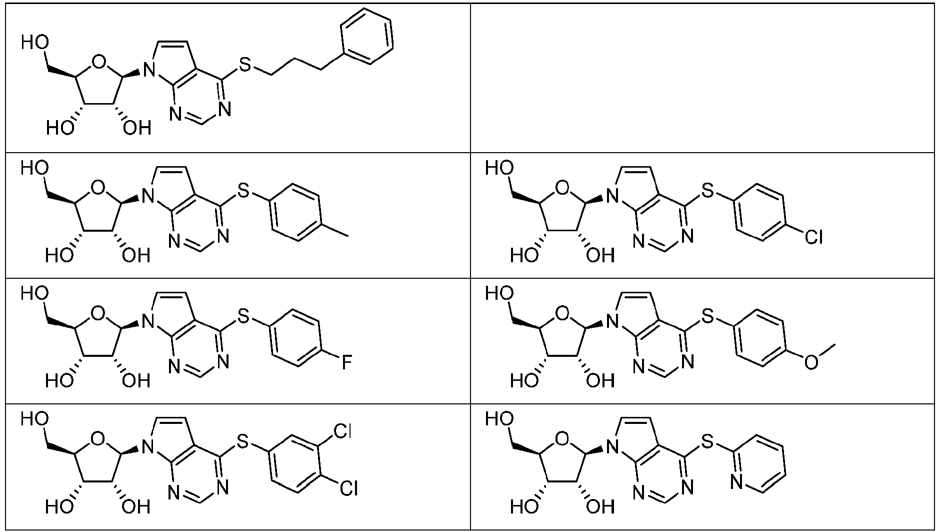

6. The compound as defined in any one of claims 1 to 5 having the stereochemistry as represented in formula II:

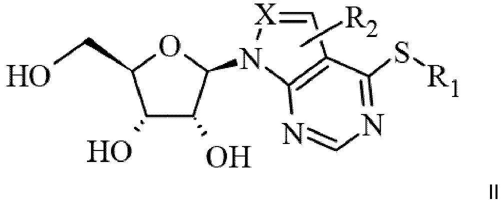

7. A pharmaceutical composition comprising a compound as defined in any one of anyone of claims 1 to 6; and at least one pharmaceutically acceptable carrier or diluent.

8. The compound as defined in any one of claims 1 to 6, or the pharmaceutical composition as defined in claim 7 for use as a human or veterinary medicine.

9. The compound as defined in any one of claims 1 to 6, or the pharmaceutical composition as defined in claim 7 for use in the diagnosis, prevention and/or treatment of a parasite infection, in particular a *Trypanosoma* infection.

10. The compound as defined in any one of claims 1 to 6, or the pharmaceutical composition as defined in claim 7 for use in the diagnosis, prevention and/or treatment of a *Trypanosoma* infection selected from the list comprising: *T. brucei, T.cruzi, T. congolense, T. vivax, T. evansi* and *T. equiperdum* infection.

11. A compound of Formula I or a stereoisomer, tautomer, racemic, salt, hydrate, or solvate thereof, for use in the diagnosis, prevention and/or treatment of a *Trypanosoma* infection 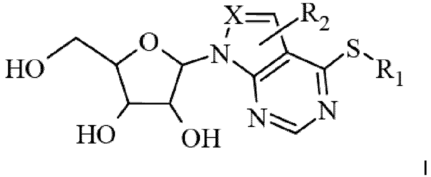 wherein
X is C;
R₁ is selected from the group comprising: -C₁₋₆alkyl, -C₁₋₆alkyl-R₃, -Ar₁, -Het₁ and -Cy₁;
R₂ is selected from the group comprising: -H or -halo;
R₃ is selected from the group comprising: -C₁₋₆alkyl, -C₁₋₆alkenyl, -C₁₋₆alkynyl, -Ar₂, -Het₂ and -Cy₂,
Ar₁ and Ar₂ are each independently selected from a 5- to 10-membered aromatic cycle optionally comprising 1 to 3 heteroatoms selected from O, N and S; each of said Ar₁ and Ar₂ optionally being substituted with one or more substituents independently selected from the list comprising: -halo, - C₁₋₆alkyl, -C₁₋₆alkenyl, -C₁₋₆alkynyl, -O-C₁₋₆alkyl, and -S-C₁₋₆alkyl;
Het₁ and Het₂ are each independently selected from a 3- to 10-membered non-aromatic heterocycle having from 1 to 3 heteroatoms selected from O, N and S; each of said Het₁ and Het₂ optionally being substituted with one or more substituents independently selected from the list comprising: - halo, -C₁₋₆alkyl, -C₁₋₆alkenyl, -C₁₋₆alkynyl, -O-C₁₋₆alkyl, and -S-C₁₋₆alkyl;
Cy₁ and Cy₂ are each independently selected from a 3- to 10-membered non-aromatic cycle; each of said Cy₁ and Cy₂ optionally being substituted with one or more substituents independently selected from the list comprising: -halo, -C₁₋₆alkyl, -C₁₋₆alkenyl, -C₁₋₆alkynyl, -O-C₁₋₆alkyl, and -S-C₁₋₆alkyl.

12. A pharmaceutical composition for use in the diagnosis, prevention and/or treatment of a *Trypanosoma* infection; said composition comprising a compound as defined in claim 11.

13. The compound for use as defined in claim 11, or the pharmaceutical composition for use as defined in claim 12, wherein said *Trypanosoma* infection is selected from the list comprising: *T. brucei, T.cruzi, T. congolense, T. vivax, T. evansi* and *T. equiperdum* infection.

## Patentansprüche

1. Verbindung der Formel (I) oder ein Stereoisomer, Tautomer, Racemat, Salz, Hydrat oder Solvat davon, 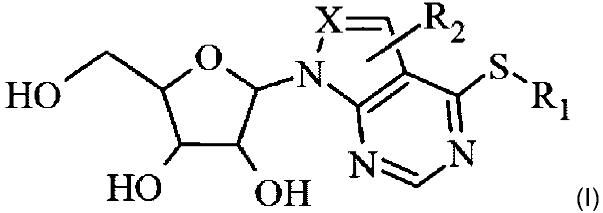 wobei
X C ist;
R₁ ausgewählt ist aus der Gruppe umfassend: -C₂₋₆-Alkyl, -C₂₋₆-Alkenyl, -CF₃, -C₁₋₆-Alkyl-R₃, -Ar₁, -C₂₋₆-Alkyl-Ar₁, -Het₁ und -Cy₁;
R₂ ausgewählt ist aus der Gruppe umfassend: -H oder -Halogen;
R₃ ausgewählt ist aus der Gruppe umfassend: -C₁₋₆-Alkyl, -C₁₋₆-Alkenyl, -C₁₋₆-Alkinyl, -Het₂ und -Cy₂,
Ar₁ ein 5- bis 10-gliedriger aromatischer Zyklus ist, der optional 1 bis 3 Heteroatome umfasst, die aus O, N und S ausgewählt sind; wobei Ar₁ optional mit einem oder mehreren Substituenten substituiert ist, die unabhängig voneinander aus der Liste ausgewählt sind, die umfasst: -Halogen, -CF₃, -C₁₋₆-Alkyl, -C₁₋₆-Alkenyl, -C₁₋₆-Alkinyl, -O-C₁-₆-Alkyl und -S-C₁-₆-Alkyl;
Het₁ und Het₂ jeweils unabhängig voneinander ausgewählt sind aus einem 3- bis 10-gliedrigen nicht-aromatischen Heterozyklus mit 1 bis 3 Heteroatomen, die aus O, N und S ausgewählt sind; jedes von Het₁ und Het₂ optional mit einem oder mehreren Substituenten substituiert ist, die unabhängig voneinander aus der Liste ausgewählt sind, die umfasst: -Halogen, -CF₃, -C₁₋₆-Alkyl, -C₁₋₆-Alkenyl, -C₁₋₆-Alkinyl, -O-C₁-₆-Alkyl und -S-C₁-₆-Alkyl;
Cy₁ und Cy₂ jeweils unabhängig voneinander aus einem 3- bis 10-gliedrigen nicht-aromatischen Zyklus ausgewählt sind; wobei jedes von Cy₁ und Cy₂ optional mit einem oder mehreren Substituenten substituiert ist, die unabhängig voneinander aus der Liste ausgewählt sind, die
umfasst: -Halogen, -CF₃, -C₁₋₆-Alkyl, -C₁₋₆-Alkenyl, -C₁₋₆-Alkinyl, -O-C₁-₆-Alkyl und -S-C₁₋₆-Alkyl.

2. Verbindung nach Anspruch 1, wobei
R₁ ausgewählt ist aus der Gruppe umfassend: -C₂₋₆-Alkyl, -C₁₋₆-Alkyl-R₃, -C₂₋₆-Alkyl-Ar₁, -Ar₁, -Het₁ und -Cy₁;
R₂ H ist;
R₃ ausgewählt ist aus der Gruppe umfassend: -C₁₋₆-Alkyl, -C₁₋₆-Alkenyl, -Het₂ und -Cy₂,
X ist C
Ar₁ ein 5- bis 6-gliedriger aromatischer Zyklus ist; wobei Ar₁ optional mit einem oder mehreren Substituenten substituiert ist, die unabhängig voneinander aus der Liste ausgewählt sind, die umfasst: -Halogen, -C₁₋₆-Alkyl und -O-C₁₋₆-Alkyl;
Het₁ und Het₂ jeweils unabhängig voneinander ausgewählt sind aus einem 3- bis 10-gliedrigen nicht-aromatischen Heterozyklus mit 1 bis 3 Heteroatomen, die aus O, N und S ausgewählt sind; jedes von Het₁ und Het₂ optional mit einem oder mehreren Substituenten substituiert ist, die unabhängig voneinander aus der Liste ausgewählt sind, die umfasst: -Halogen, -CF₃, -C₁₋₆-Alkyl, -C₁₋₆-Alkenyl, -C₁₋₆-Alkinyl, -O-C₁-₆-Alkyl und -S-C₁-₆-Alkyl;
Cy₁ und Cy₂ jeweils unabhängig voneinander aus einem 5- bis 6-gliedrigen nicht-aromatischen Zyklus ausgewählt sind; jedes von Cy₁ und Cy₂ optional mit einem oder mehreren Substituenten substituiert ist, die unabhängig voneinander aus der Liste ausgewählt sind, die umfasst: -Halogen, -C₁₋₆-Alkyl und -O-C₁₋₆-Alkyl.

3. Verbindung nach Anspruch 1, wobei
R₁ ausgewählt ist aus der Gruppe umfassend: - C₂₋₆-Alkyl, -C₁₋₃-Alkyl-R₃, -C₂₋₃-Alkyl-Ar₁, -Ar₁, -Het₁ und -Cy₁;
R₂ H ist;
R₃ ausgewählt ist aus der Gruppe umfassend: -C₁₋₆-Alkyl, -C₁₋₆-Alkenyl, -Het₂ und -Cy₂,
X C ist;
Ar₁ ist -Phenyl; optional mit einem oder mehreren Substituenten substituiert, die aus der Liste ausgewählt sind, die umfasst: -Halogen, -C₁₋₆-Alkyl und -O-C₁₋₆-Alkyl;
Het₁ und Het₂ jeweils unabhängig voneinander -Pyridinyl sind;
Cy₁ und Cy₂ jeweils unabhängig voneinander ausgewählt sind aus -Cyclopentan und -Cycloheptan.

4. Verbindung nach Anspruch 1, wobei
R₁ ausgewählt ist aus der Gruppe umfassend: -C₂₋₆-Alkyl, -C₁₋₃-Alkyl-R₃, -C₂₋₃-Alkyl-Ar₁, -Ar₁, -Het₁ und -Cy₁;
R₂ H ist;
R₃ ausgewählt ist aus der Gruppe umfassend: -C₁₋₆-Alkyl, -C₁₋₆-Alkenyl und -Cy₂,
X C ist;
Ar₁ ist -Phenyl, das optional mit einem oder mehreren Substituenten substituiert ist, die aus der Liste ausgewählt sind, die umfasst: -Halogen, -C₁₋₆-Alkyl und -O-C₁₋₆-Alkyl;;
Het₁ -Pyridinyl ist;
Cy₁ und Cy₂ jeweils unabhängig voneinander aus -Cyclopentan und -Cycloheptan ausgewählt sind.

5. Verbindung nach Anspruch 1 und ausgewählt aus der Liste umfassend;

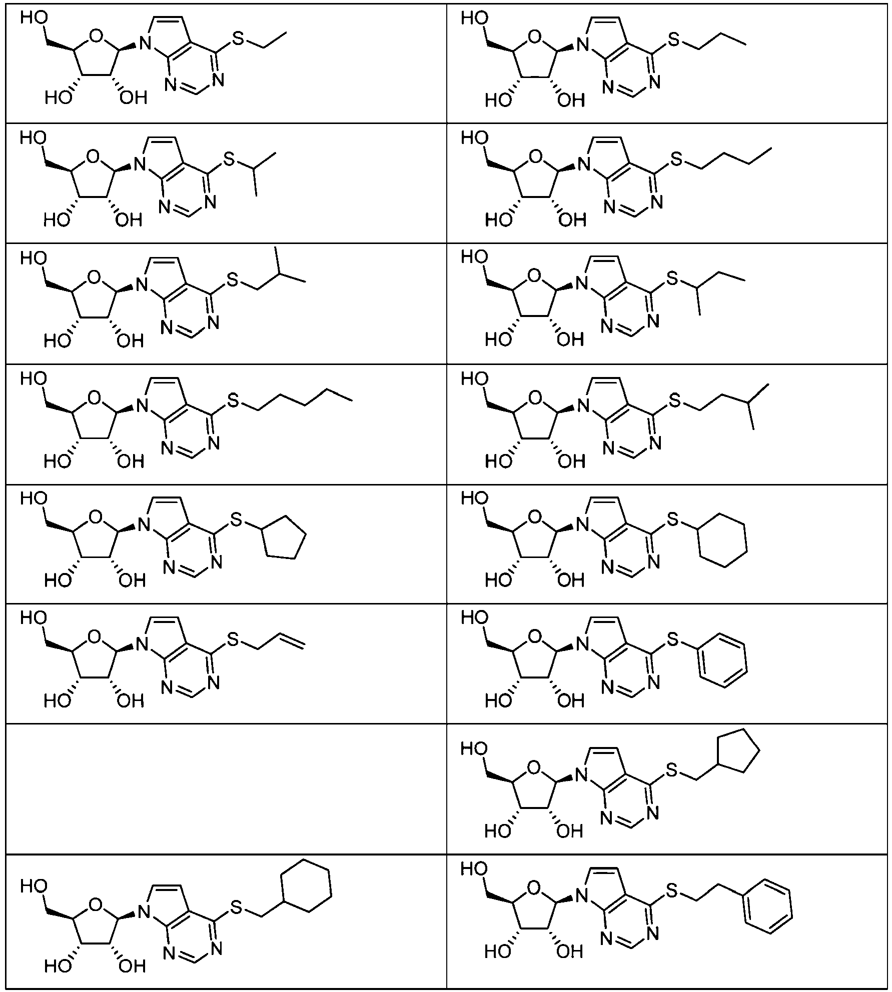

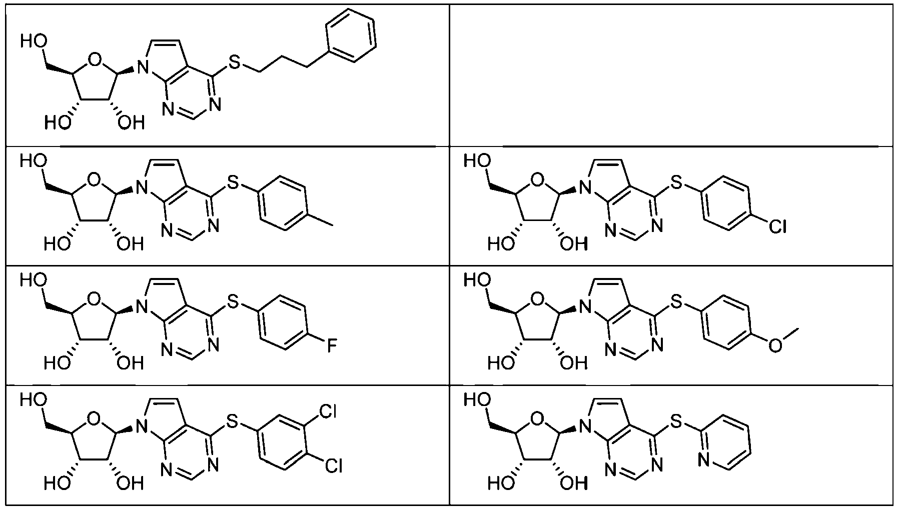

6. Verbindung wie in einem der Ansprüche 1 bis 5 definiert, mit der Stereochemie wie in Formel II dargestellt:

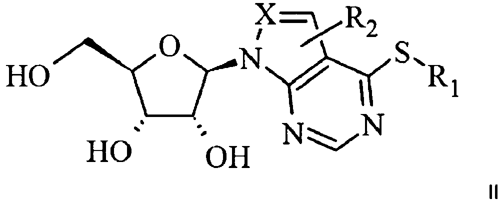

7. Pharmazeutische Zusammensetzung, umfassend eine Verbindung wie in einem der Ansprüche 1 bis 6 definiert, und mindestens einen pharmazeutisch geeigneten Träger oder Verdünnungsstoff.

8. Verbindung wie in einem der Ansprüche 1 bis 6 definiert, oder die pharmazeutische Zusammensetzung wie in Anspruch 7 definiert zur Verwendung in der Human- oder Veterinärmedizin.

9. Verbindung wie in einem der Ansprüche 1 bis 6 definiert oder die pharmazeutische Zusammensetzung wie in Anspruch 7 definiert zur Verwendung bei der Diagnose, Prävention und/oder Behandlung einer Parasiteninfektion, insbesondere einer *Trypanosoma-Infektion.*

10. Verbindung wie in einem der Ansprüche 1 bis 6 definiert oder die pharmazeutische Zusammensetzung wie in Anspruch 7 definiert zur Verwendung bei der Diagnose, Prävention und/oder Behandlung einer *Trypanosoma-Infektion,* ausgewählt aus der Liste umfassend: *T. brucei-, T. cruzi-, T. congolense-, T. vivax-, T. evansi-* und *T. equiperdum-Infektion.*

11. Verbindung der Formel I oder ein Stereoisomer, Tautomer, Racemat, Salz, Hydrat oder Solvat davon zur Verwendung bei der Diagnose, Prävention und/oder Behandlung einer *Trypanosoma-Infektion* 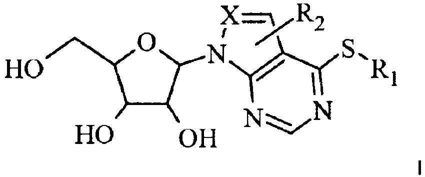 wobei
X C ist;
R₁ ausgewählt ist aus der Gruppe umfassend: -C₁₋₆-Alkyl, -C₁₋₆-Alkyl-R₃, -Ar₁, -Het₁ und -Cy₁;
R₂ ausgewählt ist aus der Gruppe umfassend: -H oder -Halogen;
R₃ ausgewählt ist aus der Gruppe umfassend: -C₁₋₆-Alkyl, -C₁₋₆-Alkenyl, -C₁₋₆-Alkinyl, -Ar₂, -Het₂ und -Cy₂,
Ar₁ und Ar₂ jeweils unabhängig voneinander ausgewählt sind aus einem 5-bis 10-gliedrigen aromatischen Zyklus, der optional 1 bis 3 Heteroatome umfasst, die aus O, N und S ausgewählt sind; wobei jedes Ar₁ and Ar₂ optional mit einem oder mehreren Substituenten substituiert ist, die unabhängig voneinander aus der Liste ausgewählt sind, die umfasst: -Halogen, -C₁₋₆-Alkyl, -C₁₋₆-Alkenyl, -C₁₋₆-Alkinyl, -O-C₁-₆-Alkyl und -S-C₁-₆-Alkyl;
Het₁ und Het₂ jeweils unabhängig voneinander ausgewählt sind aus einem 3- bis 10-gliedrigen nicht-aromatischen Heterozyklus mit 1 bis 3 Heteroatomen, die aus O, N und S ausgewählt sind; jedes von Het₁ und Het₂ optional mit einem oder mehreren Substituenten substituiert ist, die unabhängig voneinander aus der Liste ausgewählt sind, die umfasst: -Halogen, -C₁₋₆-Alkyl, -C₁₋₆-Alkenyl, -C₁₋₆-Alkinyl, -O-C₁₋₆-Alkyl und -S-C₁₋₆-Alkyl;
Cy₁ und Cy₂ jeweils unabhängig voneinander aus einem 3- bis 10-gliedrigen nicht-aromatischen Zyklus ausgewählt sind; jedes von Cy₁ und Cy₂ optional mit einem oder mehreren Substituenten substituiert ist, die unabhängig voneinander aus der Liste ausgewählt sind, die umfasst: -Halogen, -C₁₋₆-Alkyl, -C₁₋₆₋Alkenyl, -C₁₋₆-Alkinyl, -O-C₁₋₆-Alkyl und -S-C₁₋₆-Alkyl.

12. Pharmazeutische Zusammensetzung zur Verwendung bei der Diagnose, Prävention und/oder Behandlung einer Trypanosoma-Infektion; wobei die Zusammensetzung eine Verbindung wie in Anspruch 11 definiert umfasst.

13. Verbindung zur Verwendung wie in Anspruch 11 definiert oder die pharmazeutische Zusammensetzung zur Verwendung wie in Anspruch 12 definiert, wobei die *Trypanosoma-Infektion* aus der Liste ausgewählt ist, die umfasst: *T. brucei-, T. cruzi-, T. congolense-, T. vivax-, T. evansi-* und *T. equiperdum-Infektion.*

## Revendications

1. Composé de Formule I ou stéréo-isomère, tautomère, racémate, sel, hydrate, ou solvate de celui-ci, 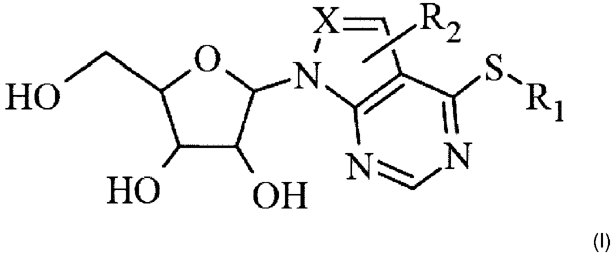 dans lequel
X est C ;
R₁ est choisi dans le groupe comprenant : -alkyle en C_{2 à 6}, -alcényle en C_{2 à 6}, -CF₃, -alkyl en C_{1 à 6}-R₃, -Ar₁, -alkyl en C_{2 à 6}-Ar₁, -Het₁ et -Cy₁ ;
R₂ est choisi dans le groupe comprenant : -H ou -halo ;
R₃ est choisi dans le groupe comprenant : -alkyle en C_{1 à 6}, -alcényle en C_{1 à 6}, -alcynyle en C_{1 à 6}, -Het₂ et -Cy₂,
Ar₁ est un cycle aromatique de 5 à 10 chaînons comprenant facultativement 1 à 3 hétéroatomes choisis parmi O, N et S ; ledit Ar₁ étant facultativement substitué par un ou plusieurs substituants indépendamment choisis parmi la liste comprenant : -halo, -CF₃, -alkyle en C_{1 à 6}, -alcényle en C_{1 à 6}, -alcynyle en C_{1 à 6}, -O-alkyle en C_{1 à 6}, et -S-alkyle en C_{1 à 6} ;
Het₁ et Het₂ sont chacun indépendamment choisis parmi un hétérocycle non aromatique de 3 à 10 chaînons ayant de 1 à 3 hétéroatomes choisis parmi O, N et S ; chacun desdits Het₁ et Het₂ étant facultativement substitué par un ou plusieurs substituants indépendamment choisis parmi la liste comprenant : -halo,-CF₃, -alkyle en C_{1 à 6}, -alcényle en C_{1 à 6}, -alcynyle en C_{1 à 6}, -O-alkyle en C_{1 à 6}, et-S-alkyle en C_{1 à 6} ;
Cy₁ et Cy₂ sont chacun indépendamment choisis parmi un cycle non aromatique de 3 à 10 chaînons ; chacun desdits Cy₁ et Cy₂ étant facultativement substitué par un ou plusieurs substituants indépendamment choisis parmi la liste comprenant : -halo, -CF₃, -alkyle en C_{1 à 6}, -alcényle en C_{1 à 6}, -alcynyle en C_{1 à 6},-O-alkyle en C_{1 à 6}, et -S-alkyle en C_{1 à 6}.

2. Composé selon la revendication 1, dans lequel ;
R₁ est choisi dans le groupe comprenant : -alkyle en C₂ à ₆, -alkyl en C_{1 à 6}-R₃, -alkyl en C_{2 à 6}-Ar₁, -Ar₁, -Het₁ et -Cy₁ ;
R₂ est H ;
R₃ est choisi dans le groupe comprenant : -alkyle en C_{1 à 6}, -alcényle en C_{1 à 6}, -Het₂ et -Cy₂,
X est C
Ar₁ est un cycle aromatique de 5 à 6 chaînons ; ledit Ar₁ étant facultativement substitué par un ou plusieurs substituants indépendamment choisis parmi la liste comprenant : -halo, -alkyle en C_{1 à 6}, et -O-alkyle en C_{1 à 6} ;
Het₁ et Het₂ sont chacun indépendamment choisis parmi un hétérocycle non aromatique de 3 à 10 chaînons ayant de 1 à 3 hétéroatomes choisis parmi O, N et S ; chacun desdits Het₁ et Het₂ étant facultativement substitué par un ou plusieurs substituants indépendamment choisis parmi la liste comprenant : -halo, - CF₃, -alkyle en C_{1 à 6}, -alcényle en C_{1 à 6}, -alcynyle en C_{1 à 6}, -O-alkyle en C_{1 à 6}, et-S-alkyle en C_{1 à 6} ;
Cy₁ et Cy₂ sont chacun indépendamment choisis parmi un cycle non aromatique de 5 à 6 chaînons ; chacun desdits Cy₁ et Cy₂ étant facultativement substitué par un ou plusieurs substituants indépendamment choisis parmi la liste comprenant : -halo, -alkyle en C_{1 à 6}, et -O-alkyle en C_{1 à 6}.

3. Composé selon la revendication 1, dans lequel ;
R₁ est choisi dans le groupe comprenant : -alkyle en C₂ à ₆, -alkyl en C_{1 à 3}-R₃, -alkyl en C_{2 à 3}-Ar₁, -Ar₁, -Het₁ et -Cy₁ ;
R₂ est H ;
R₃ est choisi dans le groupe comprenant : -alkyle en C_{1 à 6}, -alcényle en C_{1 à 6}, -Het₂ et -Cy₂,
X est C ;
Ar₁ est -phényle ; étant facultativement substitué par un ou plusieurs substituants choisis parmi la liste comprenant : -halo, -alkyle en C_{1 à 6}, et -O-alkyle en C_{1 à 6} ;
Het₁ et Het₂ sont chacun indépendamment -pyridinyle ;
Cy₁ et Cy₂ sont chacun indépendamment choisis parmi -cyclopentane et - cycloheptane.

4. Composé selon la revendication 1, dans lequel ;
R₁ est choisi dans le groupe comprenant : -alkyle en C_{2 à 6}, -alkyle en C_{1 à 3}-R₃, -alkyl en C_{2 à 3}-Ar₁, -Ar₁, -Het₁ et -Cy₁ ;
R₂ est H ;
R₃ est choisi dans le groupe comprenant : -alkyle en C_{1 à 6}, -alcényle en C_{1 à 6} et -Cy₂,
X est C ;
Ar₁ est -phényle étant facultativement substitué par un ou plusieurs substituants choisis parmi la liste comprenant : -halo, -alkyle en C_{1 à 6}, et -O-alkyle en C_{1 à 6} ;
Het₁ est -pyridinyle ;
Cy₁ et Cy₂ sont chacun indépendamment choisis parmi -cyclopentane et - cycloheptane.

5. Composé selon la revendication 1 et étant choisi dans la liste comprenant ;

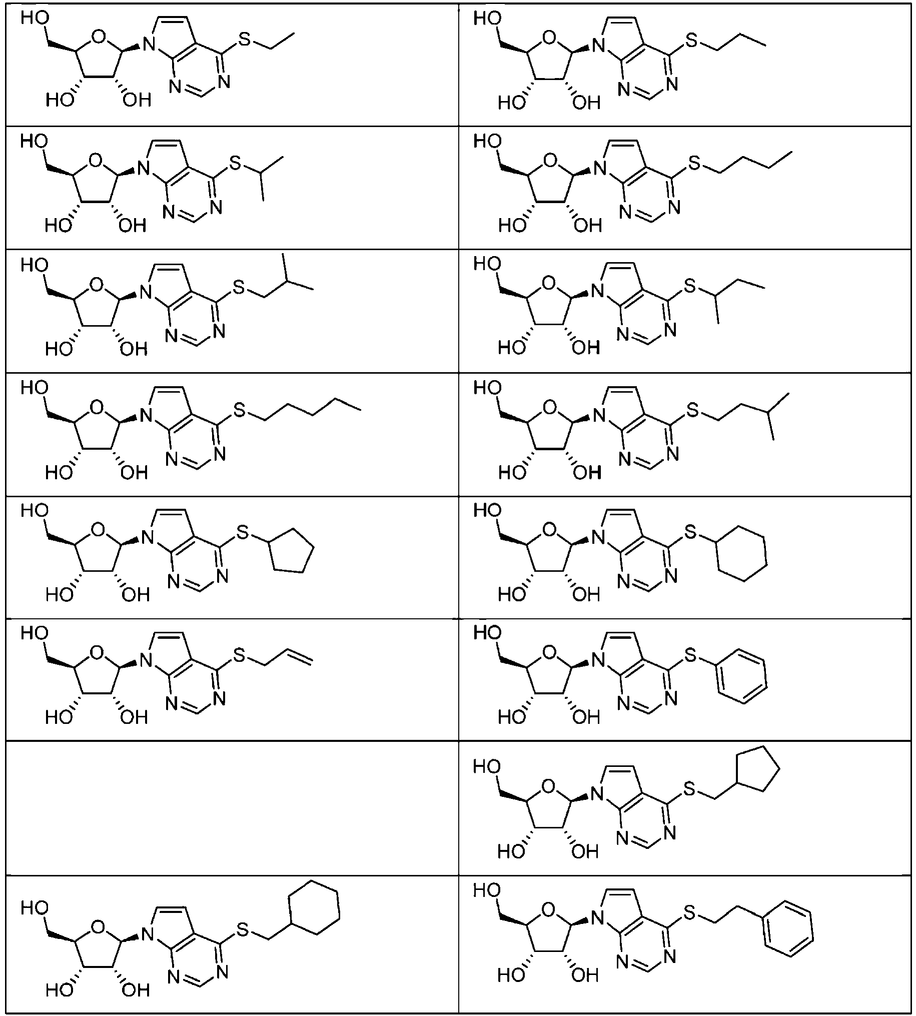

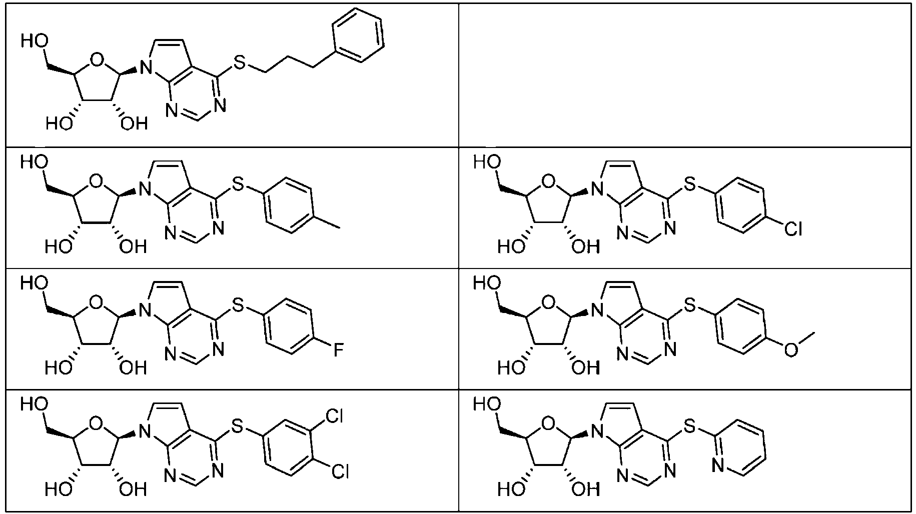

6. Composé selon l'une quelconque des revendications 1 à 5 ayant la stéréochimie telle que représentée dans la formule II :

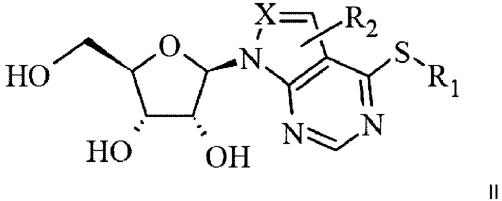

7. Composition pharmaceutique comprenant un composé tel que défini dans l'une quelconque des revendications 1 à 6 ; et au moins un support ou diluant pharmaceutiquement acceptable.

8. Composé selon l'une quelconque des revendications 1 à 6, ou composition pharmaceutique selon la revendication 7 pour une utilisation en tant que médicament humain ou vétérinaire.

9. Composé selon l'une quelconque des revendications 1 à 6, ou composition pharmaceutique selon la revendication 7 pour utilisation dans le diagnostic, la prévention et/ou le traitement d'une infection parasitaire, en particulier d'une infection par *Trypanosoma.*

10. Composé selon l'une quelconque des revendications 1 à 6, ou composition pharmaceutique selon la revendication 7 pour utilisation dans le diagnostic, la prévention et/ou le traitement d'une infection par *Trypanosoma* choisie dans la liste comprenant : une infection par *T. brucei, T.cruzi, T. congolense, T. vivax, T. evansi* et *T. equiperdum.*

11. Composé de Formule I ou stéréo-isomère, tautomère, racémate, sel, hydrate ou solvate de celui-ci, pour utilisation dans le diagnostic, la prévention et/ou le traitement d'une infection par *Trypanosoma* 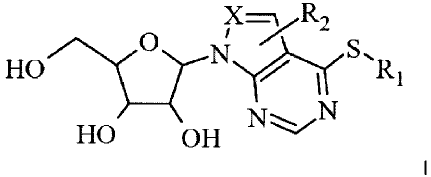 dans lequel
X est C ;
R₁ est choisi dans le groupe comprenant : -alkyle en C_{1 à 6}, -alkyl en C_{1 à 6}-R₃, -Ar₁, -Het₁ et -Cy₁ ;
R₂ est choisi dans le groupe comprenant : -H ou -halo ;
R₃ est choisi dans le groupe comprenant : -alkyle en C_{1 à 6}, -alcényle en C_{1 à 6}, -alcynyle en C_{1 à 6}, -Ar₂, -Het₂ et -Cy₂,
Ar₁ et Ar₂ sont chacun indépendamment choisis parmi un cycle aromatique de 5 à 10 chaînons comprenant facultativement 1 à 3 hétéroatomes choisis parmi O, N et S ; chacun desdits Ar₁ et Ar₂ étant facultativement substitué par un ou plusieurs substituants indépendamment choisis parmi la liste comprenant : -halo, -alkyle en C_{1 à 6}, -alcényle en C_{1 à 6}, -alcynyle en C_{1 à 6}, -O-alkyle en C_{1 à 6}, et -S-alkyle en C_{1 à 6} ;
Het₁ et Het₂ sont chacun indépendamment choisis parmi un hétérocycle non aromatique de 3 à 10 chaînons ayant de 1 à 3 hétéroatomes choisis parmi O, N et S ; chacun desdits Het₁ et Het₂ étant facultativement substitué par un ou plusieurs substituants indépendamment choisis parmi la liste comprenant : -halo, - alkyle en C_{1 à 6}, -alcényle en C_{1 à 6}, -alcynyle en C_{1 à 6}, -O-alkyle en C_{1 à 6}, et -S-alkyle en C_{1 à 6} ;
Cy₁ et Cy₂ sont chacun indépendamment choisis parmi un cycle non aromatique de 3 à 10 chaînons ; chacun desdits Cy₁ et Cy₂ étant facultativement substitué par un ou plusieurs substituants indépendamment choisis parmi la liste comprenant : -halo, -alkyle en C_{1 à 6}, -alcényle en C_{1 à 6}, -alcynyle en C_{1 à 6}, -O-alkyle en C_{1 à 6}, et -S-alkyle en C_{1 à 6}.

12. Composition pharmaceutique pour utilisation dans le diagnostic, la prévention et/ou le traitement d'une infection par *Trypanosoma ;* ladite composition comprenant un composé selon la revendication 11.

13. Composé pour utilisation selon la revendication 11, ou composition pharmaceutique pour utilisation selon la revendication 12, dans lequel ladite infection par *Trypanosoma* est choisie dans la liste comprenant : une infection par *T. brucei, T.cruzi, T. congolense, T. vivax, T. evansi* et *T. equiperdum.*
